# EUROPEAN PATENT APPLICATION

(11) **EP 1 939 201 A1**
(43) Date of publication of application: **02.07.2008**
(21) Application number: 06798243.9
(22) Date of filing: 22.09.2006
(51) Int. Cl.: C07D 473/18, A61K 31/522, A61P 11/02, A61P 11/06, A61P 17/00, A61P 27/02, A61P 31/12, A61P 31/18, A61P 35/00, A61P 37/02, A61P 37/08, A61P 43/00, C07D 473/16, C07D 473/24, C07D 473/34

(54) **NOVEL ADENINE COMPOUND**

(30) Priority: 22.09.2005 JP 2005275091
(71) Applicant: Dainippon Sumitomo Pharma Co., Ltd., Osaka 541-8524 (JP); AstraZeneca AB, 151 85 Södertälje (SE)
(72) Inventor: ISOBE, Yoshiaki, Osaka-shi, Osaka 554-0022 (JP); KURIMOTO, Ayumu, Osaka-shi, Osaka 554-0022 (JP); HASHIMOTO, Kazuki, Osaka-shi, Osaka 554-0022 (JP); NAKAMURA, Tomoaki, Osaka-shi, Osaka 554-0022 (JP); NAKAMURA, Kei, Osaka-shi, Osaka 554-0022 (JP)
(74) Representative: Vossius & Partner
(86) International application number: PCT/JP2006/318855
(87) International publication number: WO 2007/034917

(57) **Abstract**

A novel adenine compound represented by the formula (1): 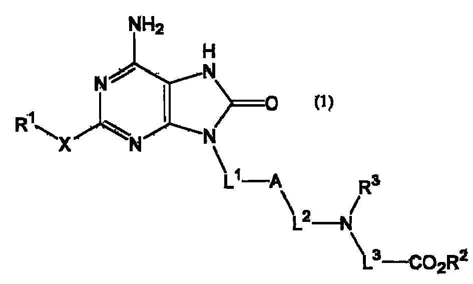 wherein A represents an (un)substituted aromatic carbocycle or (un)substituted aromatic heterocycle; L¹, L², and L³ each independently represents linear or branched alkylene, etc.; R¹ represents (un)substituted alkyl, (un)substituted aryl, etc.; R² represents hydrogen or (un)substituted alkyl; R³ represents (un)substituted alkyl, etc., provided that R³ may be bonded to L² or L³ to form a nitrogenous saturated heterocycle; and X represents oxygen, etc.; or a pharmaceutically acceptable salt of the compound. The compound and salt are useful as a medicine.

## Description

### TECHNICAL FIELD

The present invention relates to a novel adenine compound useful as a therapeutic and/or preventive agent for allergic disease, viral disease or cancer, etc.

### BACKGROUND ART

In case that foreign substances including bacteria, virus or parasite invade living organisms, immune systems exist in order to exclude said substances. In acquired immune systems, antigen processing by antigen presenting cells such as dendritic cells (DCs) is carried out when the foreign substances invade, and naive Th cells functionally differentiate via interactions of DCs/Th cells into Th1 cells or Th2 cells which play a central role of immune response in vivo. It is believed that immune diseases are developed by one-way deflection of immuno-balance of Th1 cells or Th2 cells in this process.

Specifically, cytokine such as interleukin-4 (IL-4) and interleukin-5 (IL-5) secreted by Th2 cells is secreted in an excess amount within the body of patients with allergic diseases, and the compound inhibiting immune response of Th2 cells may be expected to be a therapeutic agent for allergic disease. Also, the compound enhancing immune response of Th 1 cells may be expected to be a therapeutic or preventive agent for viral disease or cancer.

In the meantime, it was believed until recently that natural immune system was caused by nonspecific phagocytosis, but it was proved that Toll-like receptor (TLR) exists and principal parts of natural immunity activation are carried out via TLR. Moreover, a ligand of TLR may be expected to have a function as a Th1/Th2 differentiation controlling agent and to be useful for treatment or prevention of immune diseases in that TLR recognizes a ligand to induce inflammatory cytokine such as IL-12, TNF, and IL-12 differentiates and induces naive T cell to Th1 cell. Actually, it is known that Th2 cell predominates in patients with asthma, atopic dermatitis, etc., and asthma-targeted clinical trials are carried out for DNA (CpGDNA) derived from microorganism, TLR9 agonist. Additionally, it is known that TLR7/8 agonist imidazoquinoline derivative (see Patent Document 1) also shows producing inhibitory activity of Th2 cytokine interleukin-4 (IL-4) and interleukin-5 (IL-5), and is actually useful for allergic diseases in animal models.

Meanwhile, compounds described in, for example, Patent Documents 2 to 4 are known as compounds with adenine skeletons which are effective for immune diseases such as viral diseases and allergic diseases.
- Patent Document 1:: US Patent No. 4689338
- Patent Document 2:: WO98/01448
- Patent Document 3:: WO99/28321
- Patent Document 4:: WO04/029054

### DISCLOSURE OF INVENTION

### Problems to be Resolved by the Invention

Problems to be resolved by the invention are directed to provide a TLR activator, more particularly a novel adenine compound which activates as a TLR7 activator, and an immune-regulating agent comprising the same as an active ingredient, for example, a therapeutic or preventive agent for allergic disease such as asthma, COPD, allergic rhinitis, allergic conjunctivitis or atopic dermatitis, viral disease such as hepatitis B, hepatitis C, HIV or HPV, bacterial infectious disease, cancer or dermatitis, etc.

### Means of Solving the Problems

The present inventors found the novel adenine compounds of the present invention according to their intensive study in order to obtain a therapeutic or preventive agent for immune diseases such as allergic disease, viral disease or cancer with excellent TLR activating effect. In other words, the compounds of the present invention are effective as a therapeutic or preventive agent for allergic disease, viral disease, or cancer, etc.

The present invention has been achieved on the basis of the above knowledge. Specifically, the present invention relates to the following inventions.
[1] An adenine compound of the formula (1): 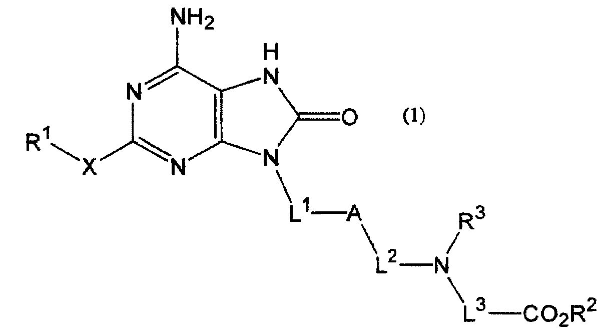 wherein A is substituted or unsubstituted aromatic carbocycle, or substituted or unsubsituted aromatic heterocycle;
L¹ is straight chain or branched chain alkylene, or a single bond;
L² and L³ are independently straight chain or branched chain alkylene, in which any 1 to 3 of methylene group(s) in the alkylene in L¹, L² and L³ may be replaced by oxygen, sulfur, SO, SO₂, carbonyl, NR⁴CO, CONR⁴, NR⁴SO₂, SO₂NR⁴, NR⁴CO₂, OCONR⁴, NR⁵CONR⁴, NR⁶C(=NR⁴)NR⁵, C(=NR⁴)NR⁵ wherein R⁴, R⁵ and R⁶ are independently hydrogen, or substituted or unsubstituted alkyl;
R¹ is halogen, substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl;
R² is hydrogen, or substituted or unsubstituted alkyl;
R³ is substituted or unsubstituted alkyl, substituted or unsubstituted alkenyl, substituted or unsubstituted alkynyl, substituted or unsubstituted cycloalkyl, or substituted or unsubstituted saturated heterocycle, or R³ may be combined together with L² or L³ to form nitrogen-containing saturated heterocycle;
X is oxygen, sulfur, SO, SO₂, NR⁷ wherein R⁷ is hydrogen or alkyl, or a single bond; provided that when R¹ is halogen, then X is a single bond; or a pharmaceutically acceptable salt thereof.

[2] The adenine compound according to [1], wherein A in the formula (1) is substituted or unsubstituted benzene ring, or substituted or unsubstituted 5- to 6-membered monocyclic nitrogen-containing aromatic heterocycle, in which the substituent on A is selected from halogen, hydroxyl, carboxy, alkyl with 1 to 6 carbon atom(s), alkoxy with 1 to 6 carbon atom(s), alkoxycarbonyl with 2 to 6 carbon atoms, alkylcarbonyl with 2 to 6 carbon atoms, alkylsulfonyl with 1 to 6 carbon atom(s), and alkylsulfinyl with 1 to 6 carbon atom(s), or a pharmaceutically acceptable salt thereof.

[3] The adenine compound according to [1] or [2], wherein in case that R², R⁴, R⁵ or R⁶ is substituted alkyl, or R³ is substituted alkyl, substituted alkenyl or substituted alkynyl, the substituents are selected from the following groups:
halogen, hydroxyl, carboxy, mercapto, alkoxy with 1 to 6 carbon atom(s), haloalkoxy with 1 to 6 carbon atom(s), alkoxycarbonyl with 2 to 6 carbon atoms, alkylsulfonyl with 1 to 6 carbon atom(s), alkylsulfinyl with 1 to 6 carbon atom(s), alkylcarbonyloxy with 2 to 6 carbon atoms, alkylthio with 1 to 6 carbon atom(s), substituted or unsubstituted amino, substituted or unsubstituted carbamoyl, substituted or unsubstituted sulfamoyl, and cycloalkyl in which the cycloalkyl may be substituted by halogen, hydroxyl, carboxy, alkyl with 1 to 4 carbon atom(s) or alkoxy with 1 to 4 carbon atom(s),
the substituents in the substituted amino, substituted carbamoyl and substituted sulfamoyl are selected from the following (a') or (b'):
(a') alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, 3- to 8-membered cycloalkoxycarbonyl, 3- to 8-membered cycloalkylsulfonyl, 3-to 8-membered cycloalkylsulfinyl,
   wherein each group may further be substituted by halogen, hydroxyl, alkyl with 1 to 6 carbon atom(s), alkoxy with 1 to 6 carbon atom(s), carboxy, or alkoxycarbonyl with 2 to 6 carbon atoms;
(b') 2 substituents are combined together with nitrogen atom to form 4- to 7-membered nitrogen-containing saturated heterocycle with 1 to 2 heteroatom(s) selected from 1 to 2 nitrogen(s), 0 to 1 oxygen and 0 to 1 sulfur,
   wherein the nitrogen-containing saturated heterocycle may be substituted on any carbon atoms or nitrogen atoms by halogen, hydroxyl, carboxy, alkyl with 1 to 6 carbon atom(s), alkoxy with 1 to 6 carbon atom(s), alkoxycarbonyl with 2 to 6 carbon atoms or alkylcarbonyl with 2 to 6 carbon atoms, where the substituent may be kept in chemically stable state,
in case that R³ is substituted cycloalkyl or substituted saturated heterocycle, the substituent is 1 or more members selected from halogen, hydroxyl, alkyl with 1 to 6 carbon atom(s), alkoxy with 1 to 6 carbon atom(s) and amino optionally substituted by 1 or 2 alkyl(s) with 1 to 6 carbon atom(s),
the substituted alkyl, substituted alkenyl and substituted alkynyl in R¹ is substituted by 1 or more members independently selected from the following (a) to (c):
(a) halogen, hydroxyl, carboxy, haloalkoxy with 1 to 6 carbon atom(s), mercapto;
(b) alkoxy with 1 to 6 carbon atom(s), alkylthio with 1 to 6 carbon atom(s), alkylcarbonyl with 2 to 6 carbon atoms, alkylcarbonyloxy with 2 to 6 carbon atoms, alkylsulfonyl with 1 to 6 carbon atom(s), alkylsulfinyl with 1 to 6 carbon atom(s), alkoxycarbonyl with 2 to 6 carbon atoms,
   wherein each group may further be substituted by 1 or more group(s) independently selected from halogen, hydroxyl, carboxy, alkoxy with 1 to 6 carbon atom(s), alkoxycarbonyl with 2 to 6 carbon atoms, amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), sulfamoyl optionally substituted by the same or different 1 or 2 alkyl(s) with 1 to 6 carbon atom(s), and alkylsulfonyl with 1 to 6 carbon atom(s);
(c) substituted or unsubstituted amino, substituted or unsubstituted carbamoyl and substituted or unsubstituted sulfamoyl, wherein each group may further be substituted by 1 or 2 substituent(s) selected from the following (j), (k) and (l), substituted or unsubstituted 3- to 8-membered cycloalkyl and substituted or unsubstituted 4- to 8-membered saturated heterocycle, wherein each group may further be substituted by 1 or more substituent(s) selected from the following (d), (e) and (f), and substituted or unsubstituted 6- to 10-membered aryl, substituted or unsubstituted 5- to 10-membered heteroaryl, substituted or unsubstituted 6- to 10-membered aryloxy and substituted or unsubstituted 5- to 10-membered heteroaryloxy, wherein each group may further be substituted by 1 or more substituent(s) selected from the following (g), (h) and (i);
   the substituted cycloalkyl in R¹ is substituted by 1 or more members independently selected from the following (d) to (f):
(d) halogen, hydroxyl, carboxy, mercapto, haloalkyl with 1 to 6 carbon atom(s), haloalkoxy with 1 to 6 carbon atom(s);
(e) alkyl with 1 to 6 carbon atom(s), alkenyl with 2 to 6 carbon atoms, alkynyl with 2 to 6 carbon atoms, alkoxy with 1 to 6 carbon atom(s), alkylcarbonyl with 2 to 6 carbon atoms, alkylcarbonyloxy with 2 to 6 carbon atoms, alkoxycarbonyl with 2 to 6 carbon atoms, alkylthio with 1 to 6 carbon atom(s), alkylsulfonyl with 1 to 6 carbon atom(s), alkylsulfinyl with 1 to 6 carbon atom(s);
   wherein each group may further be substituted by 1 or more group(s) independently selected from halogen, hydroxyl, carboxy, alkoxy with 1 to 6 carbon atom(s), alkoxycarbonyl with 2 to 6 carbon atoms, amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), sulfamoyl optionally substituted by the same or different 1 or 2 alkyl(s) with 1 to 6 carbon atom(s), and alkylsulfonyl with 1 to 6 carbon atom(s);
(f) substituted or unsubstituted amino, substituted or unsubstituted carbamoyl and substituted or unsubstituted sulfamoyl, wherein each group may further be substituted by 1 or 2 substituent(s) selected from the following (j), (k) and (l), and substituted or unsubstituted 6- to 10-membered aryl and substituted or unsubstituted 5- to 10-membered heteroaryl, wherein each group may further be substituted by 1 or more substituent(s) selected from the following (g), (h) and (i);
   the substituted aryl and substituted heteroaryl in R¹ is substituted by 1 or more members independently selected from the following (g) to (i):
(g) halogen, hydroxyl, mercapto, cyano, nitro, haloalkyl with 1 to 6 carbon atom(s), haloalkoxy with 1 to 6 carbon atom(s);
(h) alkyl with 1 to 6 carbon atom(s), alkenyl with 2 to 6 carbon atoms, alkynyl with 2 to 6 carbon atoms, alkoxy with 1 to 6 carbon atom(s), alkylcarbonyl with 2 to 6 carbon atoms, alkylcarbonyloxy with 2 to 6 carbon atoms, alkylthio with 1 to 6 carbon atom(s), alkylsulfonyl with 1 to 6 carbon atom(s), alkylsulfinyl with 1 to 6 carbon atom(s), 3- to 8-membered cycloalkyl and 4- to 8-membered saturated heterocycle,
   wherein each group may further be substituted by 1 or more group(s) independently selected from halogen, hydroxyl, carboxy, alkyl with 1 to 6 carbon atom(s), alkoxy with 1 to 6 carbon atom(s), alkoxycarbonyl with 2 to 6 carbon atoms, amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), sulfamoyl optionally substituted by the same or different 1 or 2 alkyl(s) with 1 to 6 carbon atom(s), and alkylsulfonyl with 1 to 6 carbon atom(s);
(i) substituted or unsubstituted amino, substituted or unsubstituted carbamoyl, substituted or unsubstituted sulfamoyl, wherein each group may further be substituted by 1 or 2 substituent(s) selected from the following (j), (k) and (l);
   the substituted amino, substituted carbamoyl and substituted sulfamoyl in the above (a) to (i) are substituted by 1 or 2 members independently selected from the following (j) to (l):
(j) alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, 3- to 8-membered cycloalkoxycarbonyl, 3- to 8-membered cycloalkylsulfonyl, 3-to 8-membered cycloalkylsulfinyl,
   wherein each group may further be substituted by 1 or more group(s) independently selected from halogen, hydroxyl, carboxy, alkyl with 1 to 6 carbon atom(s), alkoxy with 1 to 6 carbon atom(s), alkoxycarbonyl with 2 to 6 carbon atoms, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s), or alkylsulfonyl;
(k) 6- to 10-membered aryl, 6- to 10-membered arylcarbonyl, 6- to 10-membered aryloxycarbonyl, 6- to 10-membered arylsulfonyl, 6- to 10-membered arylsulfinyl, 5- to 10-membered heteroaryl, 5- to 10-membered heteroarylcarbonyl, 5- to 10-membered heteroaryloxycarbonyl, 5- to 10-membered heteroarylsulfonyl, 5- to 10-membered heteroarylsulfinyl,
   wherein each group may further be substituted by halogen, hydroxyl, carboxy, alkyl with 1 to 6 carbon atom(s), alkoxy with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), sulfamoyl optionally substituted by the same or different 1 or 2 alkyl(s) with 1 to 6 carbon atom(s), or alkylsulfonyl with 1 to 6 carbon atom(s);
(l) two substituents being combined together with nitrogen atom to form 4- to 7-membered nitrogen-containing saturated heterocycle with 1 to 4 heteroatom(s) selected from 1 to 2 nitrogen(s), 0 to 1 oxygen and 0 to 1 sulfur,
   wherein the nitrogen-containing saturated heterocycle may be substituted on any carbon atoms or nitrogen atoms by halogen, hydroxyl, carboxy, alkyl with 1 to 6 carbon atom(s), alkoxy with 1 to 6 carbon atom(s), alkoxycarbonyl with 2 to 6 carbon atoms, alkylcarbonyl with 2 to 6 carbon atoms, amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), sulfamoyl optionally substituted by the same or different 1 or 2 alkyl(s) with 1 to 6 carbon atom(s), or alkylsulfonyl with 1 to 6 carbon atom(s), where the substituent may be kept in chemically stable state; or a pharmaceutically acceptable salt thereof.

[4] The adenine compound according to any one of [1] to [3], wherein "-L²-NR³-L³-" in the formula (1) is a group of the formula (10): 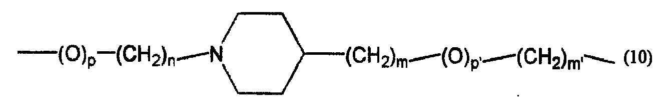 wherein p and p' are independently 0 or 1, m and m' are independently an integer of 0 to 6, n is an integer of 1 to 8 when p is 0 or an integer of 2 to 8 when p is 1,
the formula (11): 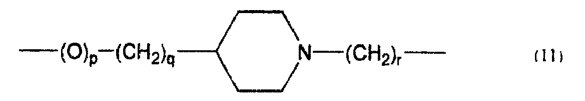 wherein p is 0 or 1, r is an integer of 1 to 6, q is an integer of 1 to 8 when p is 0 or an integer of 0 to 8 when p is 1,
the formula (12): 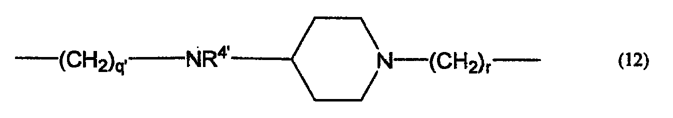 wherein r is the same as defined above, R^{4'} is hydrogen or alkyl with 1 to 3 carbon atom(s), q' is an integer of 0 to 4,
the formula (13): 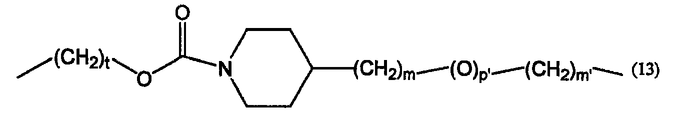 wherein t is an integer of 0 to 6, m, m' and p' are the same as defined above, or
the formula (14):

-(O)ₚ-(CH₂)ₙ-NR^{3'}-(CH₂)ₛ-(O)_{p'}-(CH₂)ₜ-

wherein p, n, t and p' are the same as defined above, s is an integer of 0 to 6 provided that when p' is 1, then s is 2 or more, R^{3'} is hydrogen, alkyl with 1 to 6 carbon atom(s), 3- to 8-membered cycloalkyl or 4- to 8-membered monocyclic saturated heterocycle, in which alkyl, cycloalkyl and saturated heterocycle may be substituted by substituents selected from halogen, hydroxyl, alkyl with 1 to 6 carbon atom(s), alkoxy with 1 to 6 carbon atom(s) and amino optionally substituted by 1 or 2 alkyl(s) with 1 to 6 carbon atom(s);
or a pharmaceutically acceptable salt thereof.

[5] The adenine compound according to any one of [1] to [4], wherein L¹ is alkylene with 1 to 6 carbon atom(s) in the formula (1), or a pharmaceutically acceptable salt thereof.

[6] The adenine compound according to any one of [1] to [5], wherein R² is alkyl with 1 to 4 carbon atom(s) in the formula (1), or a pharmaceutically acceptable salt thereof.

[7] The adenine compound according to [6], wherein R² is methyl in the formula (1), or a pharmaceutically acceptable salt thereof.

[8] The adenine compound according to any one of [1] to [5], wherein R² is alkyl with 2 to 8 carbon atoms substituted by substituted or unsubstituted amino in the formula (1), or a pharmaceutically acceptable salt thereof.

[9] The adenine compound according to [1], which is selected from the following compounds:
2-butoxy-7,8-dihydro-9-[4-{4-[4-(methoxycarbonyl)piperidin-1-yl]-butoxy}benzyl]-8-oxoadenine;
2-butoxy-7,8-dihydro-9-[4-{4-[4-(carboxy)piperidin-1-yl]butoxy}benzyl]-8-oxoadenine;
2-butoxy-7,8-dihydro-9-(4-[4-{4-[4-(N,N-dimethylamino)-butoxycarbonyl]-piperidin-1-yl}butoxy]benzyl)-8-oxoadenine;
2-butoxy-7,8-dihydro-9-[4-{3-[(N-methyl-N-methoxycarbonylmethyl)-amino]propoxy}benzyl]-8-oxoadenine;
2-butoxy-7,8-dihydro-9-{4-[1-(methoxycarbonylmethyl)piperidin-4-ylmethyloxy]benzyl}-8-oxoadenine;
2-butoxy-7,8-dihydro-9-{4-[1-(carboxymethyl)piperidin-4-ylmethyloxy]-benzyl}-8-oxoadenine;
2-butoxy-7,8-dihydro-9-[4-{1-[4-(N,N-dimethylamino)-butoxycarbonylmethyl]-piperidin-4-ylmethyloxy}benzyl]-8-oxoadenine;
2-butoxy-7,8-dihydro-9-[4-(N-ethoxycarbonylmethyl-N-methylaminomethyl)benzyl]-8-oxoadenine;
2-butoxy-7,8-dihydro-9-[4-(N-methoxycarbonylmethyl-N-methylaminomethyl)benzyl]-8-oxoadenine;
2-butoxy-7,8-dihydro-9-[4-(N-hydroxycarbonylmethyl-N-methylaminomethyl)benzyl]-8-oxoadenine;
7,8-dihydro-9-[4-(N-ethoxycarbonylmethyl-N-methylaminomethyl)-benzyl]-2-(2-methoxyethoxy)-8-oxoadenine;
7,8-dihydro-9-[4-(N-methoxycarbonylmethyl-N-methylaminomethyl)-benzyl]-2-(2-methoxyethoxy)-8-oxoadenine;
7,8-dihydro-9-[4-(N-hydroxycarbonylmethyl-N-methylaminomethyl)-benzyl]-2-(2-methoxyethoxy)-8-oxoadenine;
7,8-dihydro-9-[4-(4-methoxycarbonylpiperidin-1-ylmethyl)benzyl]-2-(2-methoxyethoxy)-8-oxoadenine;
7,8-dihydro-9-[4-(4-hydroxycarbonylpiperidin-1-ylmethyl)benzyl]-2-(2-methoxyethoxy)-8-oxoadenine;
2-butoxy-7,8-dihydro-9-[2-methoxy-4-(N-methoxycarbonylmethyl-N-methylaminomethyl)benzyl]-8-oxoadenine;
2-butoxy-7,8-dihydro-9-[2-methoxy-4-(N-hydroxycarbonylmethyl-N-methylaminomethyl)benzyl]-8-oxoadenine;
2-butoxy-7,8-dihydro-9-{4-[2-(4-methoxycarbonylpiperidin-1-yl)ethyl]-benzyl}-8-oxoadenine;
2-butoxy-7,8-dihydro-9-{4-[2-(4-hydroxycarbonylpiperidin-1-yl)ethyl]-benzyl}-8-oxoadenine;
2-butoxy-7,8-dihydro-9-{4-[2-(N-methoxycarbonylmethyl-N-methylamino)ethyl]benzyl}-8-oxoadenine;
2-butoxy-7,8-dihydro-9-{4-[3-(N-methoxycarbonylmethyl-N-methylamino)ethyl]propyl}-8-oxoadenine;
2-butoxy-7,8-dihydro-9-{4-[3-(N-hydroxycarbonylmethyl-N-methylamino)ethyl]propyl}-8-oxoadenine;
2-butoxy-7,8-dihydro-9-{4-[N-(4-dimethylaminobutoxycarbonylmethyl)-N-methylaminomethyl]-2-methoxybenzyl}-8-oxoadenine;
2-butoxy-7,8-dihydro-9-{6-[4-(4-methoxycarbonylpiperidin-1-yl)butoxy]-pyridin-3-ylmethyl}-8-oxoadenine;
2-butoxy-7,8-dihydro-9-{6-[4-(4-hydroxycarbonylpiperidin-1-yl)butoxy]-pyridin-3-ylmethyl}-8-oxoadenine;
2-butoxy-7,8-dihydro-9-(6-{4-[(N-methyl-N-methoxycarbonylmethyl)-amino]butoxy}pyridin-3-ylmethyl)-8-oxoadenine;
2-butoxy-7,8-dihydro-9-(6-{4-[(N-hydroxycarbonylinethyl-N-methyl)-amino]butoxy}pyridin-3-ylmethyl}-8-oxoadenine;
2-butoxy-7,8-dihydro-9-[6-(4-{N'-methyl-N'-[4-(N,N-dimethylamino)-butoxycarbonylmethyl]}aminobutoxy)pyridin-3-ylmethyl]-8-oxoadenine;
2-butoxy-7,8-dihydro-9-{6-[3-(4-methoxycarbonylpiperidin-1-yl)-propoxy]pyridin-3-ylmethyl}-8-oxoadenine;
2-butoxy-7,8-dihydro-9-{6-[3-(4-hydroxycarbonylpiperidin-1-yl)-butoxy]-pyridin-3-ylmethyl}-8-oxoadenine;
2-butoxy-7,8-dihydro-9-{6-[3-(methoxycarbonylmethyl)-aminopropoxy]-pyridin-3-ylmethyl}-8-oxoadenine;
2-butoxy-7,8-dihydro-9-{6-[2-(4-methoxycarbonylpiperidin-1-yl)ethoxy]-pyridin-3-ylmethyl}-8-oxoadenine;
2-butoxy-7,8-dihydro-9-{6-[2-(4-hydroxycarbonylpiperidin-1-yl)ethoxy]-pyridin-3-ylmethyl}-8-oxoadenine;
methyl N-(3-{4-[(6-amino-2-butoxy-8-oxo-7,8-dihydro-9H-purin-9-yl)-methyl]phenoxy}propyl)-N-[2-(dimethylamino)ethyl]glycinate;
7,8-dihydro-9-(4-{[N-methyl-N-(N'-methoxycarbonylmethylpiperidin-4-yl)]amino}methylbenzyl)-2-(2-methoxyethoxy)-8-oxoadenine;
7,8-dihydro-9-{4-[4-(methoxycarbonylmethoxymethyl)piperidin-1-ylmethyl]benzyl}-2-(2-methoxyethoxy)-8-oxoadenine;
7,8-dihydro-9-[4-(3-methoxycarbonylmethoxypropyl)(methyl)-aminomethylbenzyl]-2-(2-methoxyethoxy)-8-oxoadenine;
9-[4-(3-carboxymethoxypropyl)(methyl)aminomethylbenzyl]-7,8-dihydro-2-(2-methoxyethoxy)-8-oxoadenine;
7,8-dihydro-9-{4-[(3-methoxycarbonylmethoxypropyl)(1-methylaaetidin-3-yl)aminomethyl]benzyl}-2-(2-methoxyethoxy)-8-oxoadenine; or
9-{4-[(3-carboxymethoxypropyl)(1-methylazetidin-3-yl)aminomethyl]-benzyl}-7,8-dihydro-2-(2-methoxyethoxy)-8-oxoadenine;
or a pharmaceutically acceptable salt thereof.

[10] A pharmaceutical composition comprising as an active ingredient the adenine compound according to any one of [1] to [9], or a pharmaceutically acceptable salt thereof.

[11] An agent for increasing TLR7 activity comprising as an active ingredient the adenine compound according to any one of [1] to [9], or a pharmaceutically acceptable salt thereof.

[12] An immune-regulating agent comprising as an active ingredient the adenine compound according to any one of [1] to [9], or a pharmaceutically acceptable salt thereof.

[13] An agent for the treatment or prevention of allergic disease, viral disease or cancer, which comprises as an active ingredient the adenine compound according to any one of [1] to [9], or a pharmaceutically acceptable salt thereof.

[14] An agent for the treatment or prevention of asthma, COPD, allergic rhinitis, allergic conjunctivitis, atopic dermatitis, cancer, hepatitis B, hepatitis C, HIV, HPV, bacterial infectious disease or dermatitis, which comprises as an active ingredient the adenine compound according to any one of [1] to [9], or a pharmaceutically acceptable salt thereof.

[15] A pharmaceutical composition for local administration, which comprises as an active ingredient the adenine compound according to any one of [1] to [9], or a pharmaceutically acceptable salt thereof.

### BEST MODE FOR CARRYING OUT THE INVENTION

The embodiments of the present invention are explained in detail below.
The term "halogen" as used herein includes fluorine, chlorine, bromine or iodine, preferably fluorine or chlorine.

The term "alkyl" includes straight chain or branched chain alkyl with 1 to 12 carbon atom(s), particularly, methyl, ethyl, propyl, 1-methylethyl, butyl, 2-methylpropyl, 1-methylpropyl, 1,1-dimethylethyl, pentyl, 3-methylbutyl, 2-methylbutyl, 2,2-dimethylpropyl, 1-ethylpropyl, 1,1-dimethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, heptyl, 1-methylhexyl, 1-ethylpentyl, octyl, 1-methylheptyl, 2-ethylhexyl, nonyl, decyl, etc. Preferable one is alkyl with 1 to 6 carbon atom(s), more preferably, alkyl with 1 to 4 carbon atom(s).

The term "alkenyl" includes straight chain or branched chain alkenyl with 2 to 10 carbon atoms, particularly, ethenyl, propenyl, 1-methylethenyl, butenyl, 2-methylpropenyl, 1-methylpropenyl, pentenyl, 3-methylbutenyl, 2-methylbutenyl, 1-ethylpropenyl, hexenyl, 4-methylpentenyl, 3-methylpentenyl, 2-methylpentenyl, 1-methylpentenyl, 3,3-dimethylbutenyl, 1,2-dimethylbutenyl, heptenyl, 1-methylhexenyl, 1-ethylpentenyl, octenyl, 1-methylheptenyl, 2-ethylhexenyl, nonenyl, decenyl, etc. Preferable one is alkenyl with 2 to 6 carbon atoms, more preferably, alkenyl with 2 to 4 carbon atoms.

The term "alkynyl" includes straight chain or branched chain alkynyl with 1 to 10 carbon atoms, particularly, ethynyl, propynyl, butynyl, pentynyl, 3-methylbutynyl, hexynyl, 4-methylpentynyl, 3-methylpentynyl, 3,3-dimethylbutynyl, heptynyl, octynyl, 3-methylheptynyl, 3-ethylhexynyl, nonyl, or decynyl, etc. Preferable one is alkynyl with 2 to 6 carbon atoms, more preferably, alkynyl with 2 to 4 carbon atoms.

The term "cycloalkyl" includes 3- to 8-membered monocyclic cycloalkyl, particularly, cyclopropyl, cyclobutyl, cyclopentyl, cyclohexyl, cycloheptyl or cyclooctyl.

The term "cycloalkoxy" includes 3- to 8-membered monocyclic cycloalkoxy, particularly, cyclopropyloxy, cyclobutyloxy, cyclopentyloxy, cyclohexyloxy, cycloheptyloxy or cyclooctyloxy.

The term "aryl" includes 6- to 10-membered aryl, particularly, phenyl, 1-naphthyl or 2-naphthyl.

The term "heteroaryl" includes 5- to 10-membered mono- or bi-cyclic heteroaryl containing 1 to 4 heteroatom(s) selected from 0 to 2 nitrogen(s), 0 to 1 oxygen and 0 to 1 sulfur, particularly, furyl, thienyl, pyrrolyl, pyridyl, indolyl, isoindolyl, quinolyl, isoquinolyl, pyrazolyl, imidazolyl, pyrimidinyl, pyrazinyl, pyridazinyl, thiazolyl, oxazolyl, etc.

The term "saturated heterocycle" includes 4- to 10-membered mono- or bi-cyclic saturated heterocycle containing 1 to 3 heteroatom(s) selected from 0 to 3 nitrogen(s), 0 to 1 oxygen and 0 to 1 sulfur, wherein sulfur may be substituted by 1 or 2 oxygen(s), particularly, pyrrolidinyl, piperidinyl, piperazinyl, morpholinyl, thiomorpholinyl, 1-oxothiomorpholinyl, 1,1-dioxothiomorpholinyl, tetrahydrofuranyl, etc. Substituents may bind on any carbon atoms or nitrogen atoms where it may be kept in chemically stable state without any limitation for binding positions. Preferable one is 4- to 8-membered monocyclic saturated heterocycle.

The term "alkylene" includes straight chain or branched chain alkylene with 1 to 12 carbon atom(s), particularly, methylene, ethylene, trimethylene, tetramethylene, pentamethylene, hexamethylene, heptamethylene, octamethylene, nonamethylene, decamethylene, 1-methylmethylene, 1-ethylmethylene, 1-propylmethylene, 1-methylethylene, 2-methylethylene, 1-methyltrimethylene, 2-methyltrimethylene, 2-methyltetramethylene, or 3-methylpentamethylene, etc.

The term "haloalkyl" includes alkyl substituted by 1 to 5 of the same or different halogen(s), particularly, trifluoromethyl, 2,2,2-trifluoroethyl, 2,2-difluoroethyl, pentafluoroethyl, etc.

The term "alkoxy" includes straight chain or branched chain alkoxy with 1 to 10 carbon atom(s), particularly, methoxy, ethoxy, propoxy, 1-methylethoxy, butoxy, 2-methylpropoxy, 1-methylpropoxy, 1,1-dimethylethoxy, pentoxy, 3-methylbutoxy, 2-methylbutoxy, 2,2-dimethylpropoxy, 1-ethylpropoxy, 1,1-dimethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, heptyloxy, 1-methylhexyloxy, 1-ethylpentyloxy, octyloxy, 1-methylheptyloxy, 2-ethylhexyloxy, nonyloxy, decyloxy, etc. Preferable one is alkoxy with 1 to 6 carbon atom(s), more preferably, alkoxy with 1 to 4 carbon atom(s).

The term "haloalkoxy" includes alkoxy substituted by 1 to 5 of the same or different halogen(s), particularly, trifluoromethoxy, 2,2,2-trifluoroethoxy, 2,2-difluoroethoxy, 2-fluoroethoxy, pentafluoroethoxy, etc.

The term "alkylthio" includes straight chain or branched chain alkylthio with 1 to 10 carbon atom(s), particularly, methylthio, ethylthio, propylthio, 1-methylethylthio, butylthio, 2-methylpropylthio, 1-methylpropylthio, 1,1-dimethylethylthio, pentylthio, 3-methylbutylthio, 2-methylbutylthio, 2,2-dimethylpropylthio, 1-ethylpropylthio, 1,1-dimethylpropylthio, hexylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, heptylthio, 1-methylhexylthio, 1-ethylpentylthio, octylthio, 1-methylheptylthio, 2-ethylhexylthio, nonylthio, decylthio, etc. Preferable one is alkylthio with 1 to 6 carbon atom(s), more preferably, alkylthio with 1 to 4 carbon atom(s).

The term "alkyl" in "alkylcarbonyl", "alkylcarbonyloxy", "alkylsulfonyl" or "alkylsulfinyl" includes the same as the alkyl group as defined hereinbefore.

The term "alkylcarbonyl" particularly includes acetyl, propanoyl, butanoyl, 2-methylpropanoyl, pentanoyl, 3-methylbutanoyl, 2-methylbutanoyl, 2,2-dimethylpropanoyl (pivaloyl), hexanoyl, 4-methylpentanoyl, 3-methylpentanoyl, 2-methylpentanoyl, 3,3-dimethylbutanoyl, 2,2-dimethylbutanoyl, heptanoyl, octanoyl, 2-ethylhexanoyl, nonanoyl, decanoyl, etc. Preferable one is alkylcarbonyl with 2 to 6 carbon atoms, more preferably, straight chain or branched chain alkylcarbonyl with 2 to 5 carbon atoms.

The term "alkylcarbonyloxy" particularly includes acetoxy, propanoyloxy, butanoyloxy, 2-methylpropanoyloxy, pentanoyloxy, 3-methylbutanoyloxy, 2-methylbutanoyloxy, 2,2-dimethylpropanoyloxy (pivaloyloxy), hexanoyloxy, 4-methylpentanoyloxy, 3-methylpentanoyloxy, 2-methylpentanoyloxy, 3,3-dimethylbutanoyloxy, 2,2-dimethylbutanoyloxy, heptanoyloxy, octanoyloxy, 2-ethylhexanoyloxy, nonanoyloxy, decanoyloxy, etc. Preferable one is alkylcarbonyloxy with 2 to 6 carbon atoms, more preferably, straight chain or branched chain alkylcarbonyloxy with 2 to 5 carbon atoms.

The term "alkylsulfonyl" particularly includes methanesulfonyl, ethanesulfonyl, propylsulfonyl, 1-methylethylsulfonyl, butylsulfonyl, 2-methylpropylsulfonyl, 1-methylpropylsulfonyl, 1,1-dimethylethylsulfonyl, pentylsulfonyl, 3-methylbutylsulfonyl, 2-methylbutylsulfonyl, 2,2-dimethylpropylsulfonyl, 1-ethylpropylsulfonyl, 1,1-dimethylpropylsulfonyl, hexylsulfonyl, 4-methylpentylsulfonyl, 3-methylpentylsulfonyl, 2-methylpentylsulfonyl, 1-methylpentylsulfonyl, 3,3-dimethylbutylsulfonyl, 2,2-dimethylbutylsulfonyl, 1,1-dimethylbutylsulfonyl, 1,2-dimethylbutylsulfonyl, heptylsulfonyl, 1-methylhexylsulfonyl, 1-ethylpentylsulfonyl, octylsulfonyl, 1-methylheptylsulfonyl, 2-ethylhexylsulfonyl, nonylsulfonyl, decylsulfonyl, etc. Preferable one is alkylsulfonyl with 1 to 6 carbon atom(s), more preferably, straight chain or branched chain alkylsulfonyl with 1 to 4 carbon atom(s).

The term "alkylsulfinyl" particularly includes methylsulfinyl, ethylsulfinyl, propylsulfinyl, 1-methylethylsulfinyl, butylsulfinyl, 2-methylpropylsulfinyl, 1-methylpropylsulfinyl, 1,1-dimethylethylsulfinyl, pentylsulfinyl, 3-methylbutylsulfinyl, 2-methylbutylsulfinyl, 2,2-dimethylpropylsulfinyl, 1-ethylpropylsulfinyl, 1,1-dimethylpropylsulfinyl, hexylsulfinyl, 4-methylpentylsulfinyl, 3-methylpentylsulfinyl, 2-methylpentylsulfinyl, 1-methylpentylsulfinyl, 3,3-dimethylbutylsulfinyl, 2,2-dimethylbutylsulfinyl, 1,1-dimethylbutylsulfinyl, 1,2-dimethylbutylsulfinyl, heptylsulfinyl, 1-methylhexylsulfinyl, 1-ethylpentylsulfinyl, octylsulfinyl, 1-methylheptylsulfinyl, 2-ethylhexylsulfinyl, nonylsulfinyl, decylsulfinyl, etc. Preferable one is alkylsulfinyl with 1 to 6 carbon atom(s), more preferably, straight chain or branched chain alkylsulfinyl with 1 to 4 carbon atom(s).

The term "alkoxy" in "alkoxycarbonyl" includes the same as the alkoxy group as defined hereinbefore. Suitable examples of the alkoxycarbonyl are methoxycarbonyl, ethoxycarbonyl, propoxycarbonyl, 1-methylethoxycarbonyl, butoxycarbonyl, 2-methylpropoxycarbonyl, 1-methylpropoxycarbonyl, 1,1-dimethylethoxycarbonyl, pentoxycarbonyl, 3-methylbutoxycarbonyl, 2-methylbutoxycarbonyl, 2,2-dimethylpropoxycarbonyl, 1-ethylpropoxycarbonyl, 1,1-dimethylpropoxycarbonyl, hexyloxycarbonyl, 4-methylpentyloxycarbonyl, 3-methylpentyloxycarbonyl, 2-methylpentyloxycarbonyl, 1-methylpentyloxycarbonyl, 3,3-dimethylbutoxycarbonyl, 2,2-dimethylbutoxycarbonyl, 1,1-dimethylbutoxycarbonyl, 1,2-dimethylbutoxycarbonyl, heptyloxycarbonyl, 1-methylhexyloxycarbonyl, 1-ethylpentyloxycarbonyl, octyloxycarbonyl, 1-methylheptyloxycarbonyl, 2-ethylhexyloxycarbonyl, nonyloxycarbonyl, decyloxycarbonyl, etc. Preferable one is alkoxycarbonyl with 2 to 6 carbon atoms, more preferably, straight chain or branched chain alkoxycarbonyl with 2 to 4 carbon atoms.

The term "alkenyl" in "alkenyloxy", "alkenylcarbonyl", "alkenylcarbonyloxy", "alkenylsulfonyl", "alkenylsulfinyl" and "alkenyloxycarbonyl" includes the same as the alkenyl group as defined hereinbefore.

The term "alkenyloxy" particularly includes ethenyloxy, propenyloxy, 1-methylethenyloxy, butenyloxy, 2-methylpropenyloxy, 1-methylpropenyloxy, pentenyloxy, 3-methylbutenyloxy, 2-methylbutenyloxy, 1-ethylpropenyloxy, hexenyloxy, 4-methylpentenyloxy, 3-methylpentenyloxy, 2-methylpentenyloxy, 1-methylpentenyloxy, 3,3-dimethylbutenyloxy, 1,2-dimethylbutenyloxy, heptenyloxy, 1-methylhexenyloxy, 1-ethylpentenyloxy, octenyloxy, 1-methylheptenyloxy, 2-ethylhexenyloxy, nonenyloxy, or decenyloxy, etc. Preferable one is alkenyloxy with 2 to 5 carbon atoms.

The term "alkenylcarbonyl" particularly includes ethenylcarbonyl, propenylcarbonyl, 1-methylethenylcarbonyl, butenylcarbonyl, 2-methylpropenylcarbonyl, 1-methylpropenylcarbonyl, pentenylcarbonyl, 3-methylbutenylcarbonyl, 2-methylbutenylcarbonyl, 1-ethylpropenylcarbonyl, hexenylcarbonyl, 4-methylpentenylcarbonyl, 3-methylpentenylcarbonyl, 2-methylpentenylcarbonyl, 1-methylpentenylcarbonyl, 3,3-dimethylbutenylcarbonyl, 1,2-dimethylbutenylcarbonyl, heptenylcarbonyl, 1-methylhexenylcarbonyl, 1-ethylpentenylcarbonyl, octenylcarbonyl, 1-methylheptenylcarbonyl, 2-ethylhexenylcarbonyl, nonenylcarbonyl, decenylcarbonyl, etc. Preferable one is alkenylcarbonyl with 3 to 6 carbon atoms, more preferably, alkenylcarbonyl with 3 to 5 carbon atoms.

The term "alkenylcarbonyloxy" particularly includes those in which an oxygen atom binds to carbonyl of the above "alkenylcarbonyl". Preferable one is alkenylcarbonyloxy with 3 to 6 carbon atoms, more preferably, alkenylcarbonyloxy with 3 to 5 carbon atoms.

The term "alkenylsulfonyl" particularly includes ethenylsulfonyl, propenylsulfonyl, 1-methylethenylsulfonyl, butenylsulfonyl, 2-methylpropenylsulfonyl, 1-methylpropenylsulfonyl, pentenylsulfonyl, 3-methylbutenylsulfonyl, 2-methylbutenylsulfonyl, 1-ethylpropenylsulfonyl, hexenylsulfonyl, 4-methylpentenylsulfonyl, 3-methylpentenylsulfonyl, 2-methylpentenylsulfonyl, 1-methylpentenylsulfonyl, 3,3-dimethylbutenylsulfonyl, 1,2-dimethylbutenylsulfonyl, heptenylsulfonyl, 1-methylhexenylsulfonyl, 1-ethylpentenylsulfonyl, octenylsulfonyl, 1-methylheptenylsulfonyl, 2-ethylhexenylsulfonyl, nonenylsulfonyl, or decenylsulfonyl, etc. Preferable one is alkenylsulfonyl with 2 to 5 carbon atoms.

The term "alkenylsulfinyl" particularly includes ethenylsulfinyl, propenylsulfinyl, 1-methylethenylsulfinyl, butenylsulfinyl, 2-methylpropenylsulfinyl, 1-methylpropenylsulfinyl, pentenylsulfinyl, 3-methylbutenylsulfinyl, 2-methylbutenylsulfinyl, 1-ethylpropenylsulfinyl, hexenylsulfinyl, 4-methylpentenylsulfinyl, 3-methylpentenylsulfinyl, 2-methylpentenylsulfinyl, 1-methylpentenylsulfinyl, 3,3-dimethylbutenylsulfinyl, 1,2-dimethylbutenylsulfinyl, heptenylsulfinyl, 1-methylhexenylsulfinyl, 1-ethylpentenylsulfinyl, octenylsulfinyl, 1-methylheptenylsulfinyl, 2-ethylhexenylsulfinyl, nonenylsulfinyl, decenylsulfinyl, etc. Preferable one is alkenylsulfinyl with 2 to 6 carbon atoms, more preferably, alkenylsulfinyl with 2 to 5 carbon atoms.

The term "alkenyloxycarbonyl" particularly includes ethenyloxycarbonyl, propenyloxycarbonyl, 1-methylethenyloxycarbonyl, butenyloxycarbonyl, 2-methylpropenyloxycarbonyl, 1-methylpropenyloxycarbonyl, pentenyloxycarbonyl, 3-methylbutenyloxycarbonyl, 2-methylbutenyloxycarbonyl, 1-ethylpropenyloxycarbonyl, hexenyloxycarbonyl, 4-methylpentenyloxycarbonyl, 3-methylpentenyloxycarbonyl, 2-methylpentenyloxycarbonyl, 1-methylpentenyloxycarbonyl, 3,3-dimethylbutenyloxycarbonyl, 1,2-dimethylbutenyloxycarbonyl, heptenyloxycarbonyl, 1-methylhexenyloxycarbonyl, 1-ethylpentenyloxycarbonyl, octenyloxycarbonyl, 1-methylheptenyloxycarbonyl, 2-ethylhexenyloxycarbonyl, nonenyloxycarbonyl, decenyloxycarbonyl, etc. Preferable one is alkenyloxycarbonyl with 3 to 6 carbon atoms, more preferably, alkenyloxycarbonyl with 3 to 5 carbon atoms.

The term "alkynyl" in "alkynyloxy", "alkynylcarbonyl", "alkynylcarbonyloxy", "alkynylsulfonyl", "alkynylsulfinyl" and "alkynyloxycarbonyl" includes the same as the alkynyl group as defined hereinbefore.

The term "alkynyloxy" particularly includes ethynyloxy, propynyloxy, butynyloxy, pentynyloxy, 3-methylbutynyloxy, hexynyloxy, 4-methylpentynyloxy, 3-methylpentynyloxy, 3,3-dimethylbutynyloxy, heptynyloxy, octynyloxy, 3-methylheptynyloxy, 3-ethylhexynyloxy, nonyloxy, or decynyloxy, etc. Preferable one is alkynyloxy with 2 to 5 carbon atoms.

The term "alkynylcarbonyl" particularly includes ethynylcarbonyl, propynylcarbonyl, butynylcarbonyl, pentynylcarbonyl, 3-methylbutynylcarbonyl, hexynylcarbonyl, 4-methylpentynylcarbonyl, 3-methylpentynylcarbonyl, 3,3-dimethylbutynylcarbonyl, heptynylcarbonyl, octynylcarbonyl, 3-methylheptynylcarbonyl, 3-ethylhexynylcarbonyl, nonylcarbonyl, decynylcarbonyl, etc. Preferable one is alkynylcarbonyl with 3 to 6 carbon atoms, more preferably, alkynylcarbonyl with 3 to 5 carbon atoms.

The term "alkynylcarbonyloxy" particularly includes those in which an oxygen atom binds to carbonyl in the above "alkynylcarbonyl". Preferable one is alkynylcarbonyloxy with 3 to 5 carbon atoms.

The term "alkynylsulfonyl" particularly includes ethynylsulfonyl, propynylsulfonyl, butynylsulfonyl, pentynylsulfonyl, 3-methylbutynylsulfonyl, hexynylsulfonyl, 4-methylpentynylsulfonyl, 3-methylpentynylsulfonyl, 3,3-dimethylbutynylsulfonyl, heptynylsulfonyl, octynylsulfonyl, 3-methylheptynylsulfonyl, 3-ethylhexynylsulfonyl, nonylsulfonyl, decynylsulfonyl, etc. Preferable one is alkynylsulfonyl with 2 to 6 carbon atoms, more preferably, alkynylsulfonyl with 2 to 5 carbon atoms.

The term "alkynylsulfinyl" particularly includes ethynylsulfinyl, propynylsulfinyl, butynylsulfinyl, pentynylsulfinyl, 3-methylbutynylsulfinyl, hexynylsulfinyl, 4-methylpentynylsulfinyl, 3-methylpentynylsulfinyl, 3,3-dimethylbutynylsulfinyl, heptynylsulfinyl, octynylsulfinyl, 3-methylheptynylsulfinyl, 3-ethylhexynylsulfinyl, nonylsulfinyl, decynylsulfinyl, etc. Preferable one is alkynylsulfinyl with 2 to 6 carbon atoms, more preferably, alkynylsulfinyl with 2 to 5 carbon atoms.

The term "alkynyloxycarbonyl" particularly includes ethynyloxycarbonyl, propynyloxycarbonyl, butynyloxycarbonyl, pentynyloxycarbonyl, 3-methylbutynyloxycarbonyl, hexynyloxycarbonyl, 4-methylpentynyloxycarbonyl, 3-methylpentynyloxycarbonyl, 3,3-dimethylbutynyloxycarbonyl, heptynyloxycarbonyl, octynyloxycarbonyl, 3-methylheptynyloxycarbonyl, 3-ethylhexynyloxycarbonyl, nonyloxycarbonyl, decynyloxycarbonyl, etc. Preferable one is alkynyloxycarbonyl with 3 to 6 carbon atoms, more preferably, alkynyloxycarbonyl with 3 to 5 carbon atoms.

The term "cycloalkyl" in "cycloalkylcarbonyl", "cycloalkylcarbonyloxy", "cycloalkylsulfonyl" and "cycloalkylsulfinyl" includes the same as the cycloalkyl as defined hereinbefore.

The term "cycloalkylcarbonyl" particularly includes cyclopropylcarbonyl, cyclobutylcarbonyl, cyclopentylcarbonyl, cyclohexylcarbonyl, cycloheptylcarbonyl or cyclooctylcarbonyl.

The term "cycloalkylcarbonyloxy" particularly includes those in which an oxygen atom binds to carbonyl of the above "cycloalkylcarbonyl". Suitable examples are cyclopropylcarbonyloxy, cyclobutylcarbonyloxy, cyclopentylcarbonyloxy, cyclohexylcarbonyloxy, cycloheptylcarbonyloxy or cyclooctylcarbonyloxy.

The term "cycloalkylsulfonyl" particularly includes cyclopropylsulfonyl, cyclobutylsulfonyl, cyclopentylsulfonyl, cyclohexylsulfonyl, cycloheptylsulfonyl or cyclooctylsulfonyl.

The term "cycloalkylsulfinyl" particularly includes cyclopropylsulfinyl, cyclobutylsulfinyl, cyclopentylsulfinyl, cyclohexylsulfinyl, cycloheptylsulfinyl or cyclooctylsulfinyl.

The term "cycloalkoxy" in "cycloalkoxycarbonyl" includes the same as the cycloalkoxy group as defined hereinbefore. Suitable examples are cyclopropyloxycarbonyl, cyclobutyloxycarbonyl, cyclopentyloxycarbonyl, cyclohexyloxycarbonyl, cycloheptyloxycarbonyl or cyclooctyloxycarbonyl.

The term "aryl" in "aryloxy", "arylcarbonyl", "aryloxycarbonyl", "arylcarbonyloxy", "arylsulfonyl" and "arylsulfinyl" includes the same as the aryl group as defined hereinbefore. Suitable examples of "aryloxy" are phenoxy, 1-naphthoxy or 2-naphthoxy. Suitable examples of "arylcarbonyl" are benzoyl, 1-naphthaloyl or 2-naphthaloyl. Particularly, "aryloxycarbonyl" includes phenoxycarbonyl, 1-naphthoxycarbonyl or 2-naphthoxycarbonyl. Suitable examples of "arylcarbonyloxy" are benzoyloxy, 1-naphthoyloxy or 2-naphthoyloxy. Particularly, "arylsulfonyl" includes phenylsulfonyl, 1-naphthylsulfonyl, 2-naphthylsulfonyl. Particularly, "arylsulfinyl" includes phenylsulfinyl, 1-naphthylsulfinyl, 2-naphthylsulfinyl.

The term "heteroaryl" in "heteroaryloxy", "heteroarylcarbonyl", "heteroaryloxycarbonyl", "heteroarylcarbonyloxy", "heteroarylsulfonyl" and "heteroarylsulfinyl" includes the same as the heteroaryl group as defined hereinbefore. Suitable examples of "heteroaryloxy" are pyrrolyloxy, pyridyloxy, pyrazinyloxy, pyrimidinyloxy, pyridazinyloxy, furyloxy, thienyloxy. Suitable examples of "heteroarylcarbonyl" are pyrrolylcarbonyl, pyridylcarbonyl, pyrazinylcarbonyl, pyrimidinylcarbonyl, pyridazinylcarbonyl, furylcarbonyl, thienylcarbonyl, etc. Suitable examples of "heteroaryloxycarbonyl" are pyrrolyloxycarbonyl, pyridyloxycarbonyl, pyrazinyloxycarbonyl, pyrimidinyloxycarbonyl, pyridazinyloxycarbonyl, furyloxycarbonyl, thienyloxycarbonyl. Suitable examples of "heteroarylcarbonyloxy" are pyrrolylcarbonyloxy, pyridylcarbonyloxy, pyrazinylcarbonyloxy, pyrimidinylcarbonyloxy, pyridazinylcarbonyloxy, furylcarbonyloxy, thienylcarbonyloxy. Suitable examples of "heteroarylsulfonyl" are pyrrolylsulfonyl, pyridylsulfonyl, pyrazinylsulfonyl, pyrimidinylsulfonyl, pyridazinylsulfonyl, furylsulfonyl, thienylsulfonyl. Suitable examples of "heteroarylsulfinyl" are pyrrolylsulfinyl, pyridylsulfinyl, pyrazinylsulfinyl, pyrimidinylsulfinyl, pyridazinylsulfinyl, furylsulfinyl, thienylsulfinyl.

The term "nitrogen-containing saturated heterocycle" used herein includes, preferably, 4- to 7-membered nitrogen-containing saturated heterocycle containing 1 to 2 heteroatom(s) selected from 1 to 2 nitrogen(s), 0 to 1 oxygen and 0 to 1 sulfur, and the sulfur atom may be substituted by 1 or 2 oxygen atom(s).

The "nitrogen-containing saturated heterocycle" formed by combining R³ with L² may preferably include nitrogen-containing saturated heterocycle of the formulae (2) to (5): 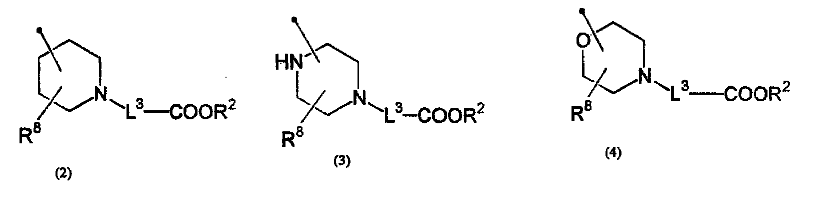 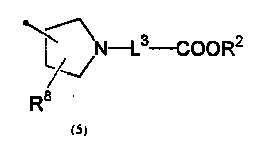 wherein R² and L³ are the same as defined above, R⁸ is halogen, hydroxyl, carboxy, alkyl with 1 to 6 carbon atom(s), alkoxy with 1 to 6 carbon atom(s), alkoxycarbonyl with 2 to 6 carbon atoms, alkylcarbonyl with 2 to 6 carbon atoms, alkylsulfonyl with 1 to 6 carbon atom(s) or alkylsulfinyl with 1 to 6 carbon atom(s) and may bind to any carbon atoms and imino.

The "nitrogen-containing saturated heterocycle" formed by combining R³ with L³ may preferably include nitrogen-containing saturated heterocycle of the formulae (6) to (9): 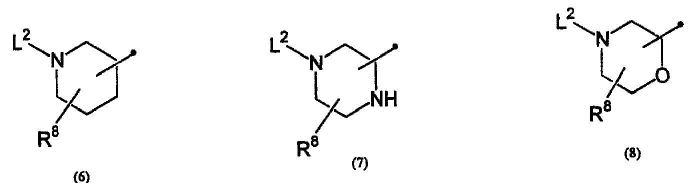 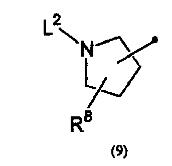 wherein L² and R⁸ are the same as defined above.

The "-L²-NR³-L³-" in the formula (1) is preferably a group of the formula (10): 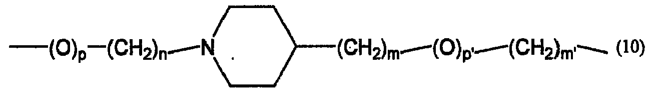 wherein p and p' are independently 0 or 1, m and m' are independently an integer of 0 to 6, n is an integer of 1 to 8 when p is 0 or an integer of 2 to 8 when p is 1, the formula (11): 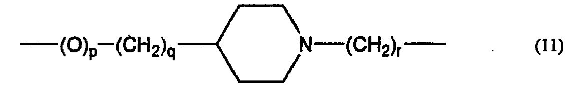 wherein p is 0 or 1, r is an integer of 1 to 6, q is an integer of 1 to 8 when p is 0 or an integer of 0 to 8 when p is 1, the formula (12): 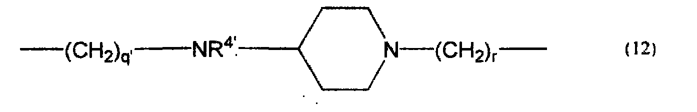 wherein r is the same as defined above, R^{4'} is hydrogen or alkyl with 1 to 3 carbon atom(s), q' is an integer of 0 to 4, the formula (13): 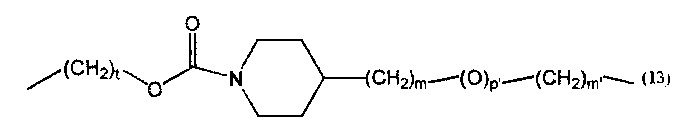 wherein t is an integer of 0 to 6, m, m' and p' are the same as defined above, or the formula (14):

-(O)ₚ-(CH₂)ₙ-NR^{3'}-(CH₂)ₛ-(O)_{p'}-(CH₂)ₜ-

wherein p, n, t and p' are the same as defined above, s is an integer of 0 to 6 provided that when p' is 1, then s is 2 or more, R^{3'} is hydrogen, alkyl with 1 to 6 carbon atom(s), 3- to 8-membered cycloalkyl or 4- to 8-membered monocyclic saturated heterocycle, in which the alkyl, cycloalkyl and saturated heterocycle may be substituted by substituents selected from halogen, hydroxyl, alkyl with 1 to 6 carbon atom(s), alkoxy with 1 to 6 carbon atom(s) and amino optionally substituted by 1 or 2 alkyl(s) with 1 to 6 carbon atom(s).

The aromatic carbocycle in A includes benzene ring or naphthalene ring without any limitation for binding positions.

The aromatic heterocycle in A includes 5- to 10-membered mono- or bi-cyclic aromatic heterocycle containing 1 to 4 heteroatom(s) selected from 0 to 3 nitrogen(s), 0 to 1 oxygen and 0 to 1 sulfur without any limitation for binding positions, where it may be kept in chemically stable state. The aromatic heterocycle particularly includes furan, thiophen, pyrrole, pyridine, indole, isoindole, quinoline, isoquinoline, pyrazole, imidazole, pyrimidine, pyrazine, pyridazine, thiazole or oxazole, etc.

The aromatic carbocycle and aromatic heterocycle in A may be substituted by the same or different 1 to 3 substituent(s), wherein the substituent includes halogen, hydroxyl, carboxy, alkyl, alkoxy, alkoxycarbonyl with 2 to 6 carbon atoms, alkylcarbonyl with 2 to 6 carbon atoms, alkylsulfonyl with 1 to 6 carbon atom(s) and alkylsulfinyl with 1 to 6 carbon atom(s), etc.

The term "4- to 7-membered saturated nitrogen-containing heterocycle" as used herein includes 4- to 7-membered saturated nitrogen-containing heterocycle containing 1 to 3 heteroatom(s) selected from 1 to 3 nitrogen(s), 0 to 1 oxygen and 0 to 1 sulfur, and the substituent may bind on any binding positions without any limitation where it may be kept in chemically stable state. The sulfur atom may be substituted by 1 or 2 oxygen atom(s). Suitable examples are azetidine, pyrrolidine, piperidine, piperazine, morpholine, thiomorpholine, thiomorpholin-1-oxide, thiomorpholine-1,1-dioxide, perhydroazepine, imidazolidine, oxazolidine, etc.

The substituents of the substituted alkyl, alkenyl and alkynyl herein include the following (a) to (c):
(a) halogen, hydroxyl, carboxy, haloalkoxy, mercapto;
(b) alkoxy, alkylthio, alkylcarbonyl, alkylcarbonyloxy, alkylsulfonyl, alkylsulfinyl, alkoxycarbonyl, alkenyloxy, alkenylcarbonyl, alkenylcarbonyloxy, alkenylsulfonyl, alkenylsulfinyl, alkenyloxycarbonyl, alkynyloxy, alkynylcarbonyl, alkynylcarbonyloxy, alkynyloxycarbonyl, alkynylsulfonyl, alkynylsulfinyl,
   wherein each group may further be substituted by halogen, hydroxyl, alkoxy, carboxy, alkoxycarbonyl, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s) or alkylsulfonyl;
(c) substituted or unsubstituted amino, substituted or unsubstituted carbamoyl and substituted or unsubstituted sulfamoyl, wherein each group may further be substituted by 1 or 2 substituent(s) selected from the following (j), (k) and (l), substituted or unsubstituted aryl, substituted or unsubstituted aryloxy, substituted or unsubstituted arylcarbonyl, substituted or unsubstituted arylcarbonyloxy, substituted or unsubstituted arylsulfonyl, substituted or unsubstituted arylsulfinyl, substituted or unsubstituted aryloxycarbonyl, substituted or unsubstituted heteroaryl, substituted or unsubstituted heteroaryloxy, substituted or unsubstituted heteroarylcarbonyl, substituted or unsubstituted heteroarylcarbonyloxy, substituted or unsubstituted heteroarylsulfonyl, substituted or unsubstituted heteroarylsulfinyl and substituted or unsubstituted heteroaryloxycarbonyl, wherein each group may further be substituted by 1 or more substituent(s) selected from the following (g), (h) and (i), or substituted or unsubstituted cycloalkyl, substituted or unsubstituted cycloalkoxy, substituted or unsubstituted cycloalkylcarbonyl, substituted or unsubstituted cycloalkylcarbonyloxy, substituted or unsubstituted cycloalkylsulfonyl, substituted or unsubstituted cycloalkylsulfinyl, substituted or unsubstituted cycloalkoxycarbonyl and substituted or unsubstituted saturated heterocycle, wherein each group may further be substituted by 1 or more group(s) selected from the following (d), (e) and (f), etc.; said substituents being the same or different and being substituted on each group by 1 or more, preferably 1 to 5, more preferably 1 to 3 thereof.

The substituents of the substituted cycloalkyl, cycloalkoxy, cycloalkylcarbonyl, cycloalkylsulfonyl, cycloalkylsulfinyl, cycloalkylcarbonyloxy, cycloalkoxycarbonyl or saturated heterocycle herein include the following (d) to (f):
(d) halogen, hydroxyl, carboxy, mercapto, haloalkyl, haloalkoxy;
(e) alkyl, alkenyl, alkynyl, alkoxy, alkylthio, alkylcarbonyl, alkylcarbonyloxy, alkylsulfonyl, alkylsulfinyl, alkoxycarbonyl,
   wherein each group may further be substituted by halogen, hydroxyl, alkoxy, carboxy, alkoxycarbonyl, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s) or alkylsulfonyl;
(f) substituted or unsubstituted aryl and substituted or unsubstituted heteroaryl, wherein each group may further be substituted by 1 or more substituent(s) selected from the following (g), (h) and (i), or substituted or unsubstituted amino, substituted or unsubstituted carbamoyl and substituted or unsubstituted sulfamoyl,
   wherein each group may further be substituted by 1 or 2 substituent(s) selected from the following (j), (k) and (l), etc.;
   said substituents being the same or different and being substituted on each group by 1 or more, preferably 1 to 5, more preferably 1 to 3 thereof.

The substituents of the substituted aryl, heteroaryl, aryloxy, arylcarbonyl, arylcarbonyloxy, aryloxycarbonyl, arylsulfonyl, arylsulfinyl, heteroaryloxy, heteroarylcarbonyl, heteroarylcarbonyloxy, heteroaryloxycarbonyl, heteroarylsulfonyl, heteroarylsulfinyl, aromatic carbocycle or aromatic heterocycle herein include the following (g) to (i):
(g) halogen, hydroxyl, mercapto, cyano, nitro, haloalkyl, haloalkoxy;
(h) alkyl, alkoxy, alkylthio, alkylcarbonyl, alkylcarbonyloxy, alkylsulfonyl, alkylsulfinyl, alkenyl, alkynyl, cycloalkyl, saturated heterocycle,
   wherein each group may further be substituted by halogen, hydroxyl, alkyl, alkoxy, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s), or alkylsulfonyl;
(i) substituted or unsubstituted amino, substituted or unsubstituted carbamoyl, or substituted or unsubstituted sulfamoyl wherein each group may further be substituted by 1 or 2 substituent(s) selected from the following (j), (k) and (l), etc.;
   said substituents being the same or different and being substituted on each group by 1 or more, preferably 1 to 5, more preferably 1 to 3 thereof.

The substituents in the substituted or unsubstituted "amino", substituted or unsubstituted "carbamoyl" and substituted or unsubstituted "sulfamoyl" include the following (j), (k) and (l):
(j) alkyl, alkenyl, alkynyl, alkylcarbonyl, alkylcarbonyloxy, alkoxycarbonyl, alkylsulfonyl, alkylsulfinyl, alkenylcarbonyl, alkenylcarbonyloxy, alkenyloxycarbonyl, alkenylsulfonyl, alkenylsulfinyl, alkynylcarbonyl, alkynylcarbonyloxy, alkynyloxycarbonyl, alkynylsulfonyl, alkynylsulfinyl, cycloalkyl, cycloalkylcarbonyl, cycloalkylcarbonyloxy, cycloalkoxycarbonyl, cycloalkylsulfonyl, cycloalkylsulfinyl, saturated heterocycle,
   wherein each group may further be substituted by halogen, hydroxyl, alkoxy, carboxyl, alkoxycarbonyl, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s), or alkylsulfonyl;
(k) aryl, arylcarbonyl, arylcarbonyloxy, aryloxycarbonyl, arylsulfonyl, arylsulfinyl, heteroaryl, heteroarylcarbonyl, heteroarylcarbonyloxy, heteroaryloxycarbonyl, heteroarylsulfonyl, heteroarylsulfinyl,
   wherein each group may further be substituted by halogen, hydroxyl, alkyl, alkoxy, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s), or alkylsulfonyl;
(l) when 2 substituents of amino, carbamoyl and sulfamoyl are combined together with nitrogen atom to form 4- to 7-membered saturated nitrogen-containing heterocycle with 1 to 4 heteroatom(s) selected from 1 to 2 nitrogen(s), 0 to 1 oxygen and 0 to 1 sulfur,
wherein the saturated nitrogen-containing heterocycle may further be substituted on any carbon atoms or nitrogen atoms by halogen, hydroxyl, alkoxy, carboxyl, alkoxycarbonyl, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s), or alkylsulfonyl, where the substituent may be kept in chemically stable state, etc.;
said substituents being substituted by 1 or 2 substituent(s) where those may be kept in chemically stable state.

The "nitrogen-containing saturated heterocycle" particularly includes azetidine, pyrrolidine, piperidine, piperazine, morpholine, thiomorpholine, thiopmorpholin-1-oxide, thiomorpholin-1,1-dioxide, perhydroazepine, etc.

In the formula (1), A is preferably 4- to 7-membered nitrogen-containing saturated heterocycle containing 1 to 2 heteroatom(s) selected from 1 to 2 nitrogen(s) and 0 to 1 oxygen. Particular one is piperidinyl, piperazinyl, morpholino, etc.

In formula (1), R² is preferably alkyl with 1 to 4 carbon atom(s), alkylcarbonyloxyalkyl with 3 to 8 carbon atoms, 6- to 10-membered arylcarbonyloxyalkyl, or alkyl substituted by substituted or unsubstituted amino. The alkylcarbonyloxyalkyl particularly includes acetoxymethyl, 1-acetoxyethyl. The arylcarbonyloxyalkyl particularly includes benzoyloxymethyl, etc. The substituted or unsubstituted aminoalkyl particularly includes dimethylaminobutyl, 4-morpholinobutyl, etc. More preferably, R² is methyl.

In formula (1), X is preferably oxygen or a single bond. In case that X is NR⁷, R⁷ is preferably hydrogen or alkyl with 1 to 3 carbon atom(s), more preferably, hydrogen or methyl.

In formula (1), R¹ is preferably substituted or unsubstituted straight chain or branched chain alkyl with 1 to 6 carbon atom(s), particularly, substituted or unsubstituted methyl, ethyl, propyl, butyl, pentyl, 1-methylethyl, 1-methylpropyl, 2-methylbutyl, etc. More preferable one is straight-chain alkyl with 1 to 4 carbon atom(s).

In case that R¹ is substituted alkyl, the substituent of the alkyl preferably includes fluorine, hydroxyl, straight chain or branched chain alkoxy with 1 to 4 carbon atom(s), or straight chain or branched chain alkylthio with 1 to 4 carbon atom(s), etc. More preferably, the substituent includes hydroxyl, or straight chain or branched chain alkoxy with 1 to 3 carbon atom(s).

The adenine compounds of the present invention are intended to include all tautomers, geometric isomers or stereoisomers, and optionally, a mixture thereof depending on the kinds of substituents.

In other words, in case that one or more asymmetric carbon atom(s) may exist in the compound of the formula (1), diastereomers and enantiomers may also exist, and the present invention includes the diastereomers, the enantiomers, and mixtures and isolated forms thereof.

Additionally, the adenine compound of the formula (1) and a tautomer thereof are chemically equivalent, and the adenine compound of the present invention also includes the tautomer thereof. Particularly, the tautomer is in the form of hydroxy of the formula (1'): 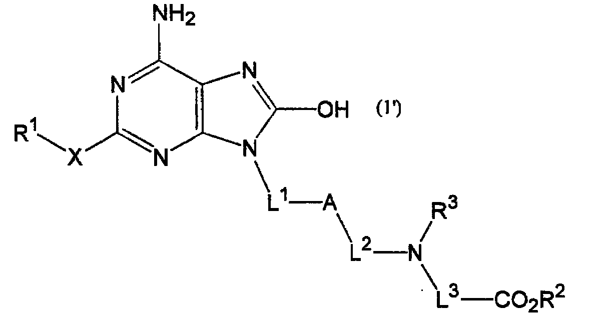 wherein R¹, R², R³, A, X, L¹, L² and L³ are the same as defined above.

A pharmaceutically acceptable salt includes acid addition salt and base addition salt. For example, the acid addition salt includes an inorganic acid salt such as hydrochloride, hydrobromide, sulfate, hydroiodide, nitrate, phosphate, etc., and an organic acid salt such as citrate, oxalate, acetate, formate, propionate, benzoate, trifluoroacetate, fumarate, maleate, succinate, tartrate, lactate, pyruvate, methanesulfonate, benzenesulfonate, para-toluenesulfonate, etc., and the base addition salt includes an inorganic base salt such as sodium salt, potassium salt, calcium salt, magnesium salt, ammonium salt, etc., and an organic base salt such as triethyl ammonium salt, triethanol ammonium salt, pyridinium salt, diisopropyl ammonium salt, etc., and additionally, amino acid salt such as basic or acidic amino acid including arginine, aspartic acid and glutamic acid. The compound of the formula (1) may be a hydrate, or a solvate such as ethanolate.

The compound of the general formula (1) may be prepared by the following methods. Starting compounds which are not described below may be prepared according to the following methods or known methods or those similar thereto.

### Preparation Method 1

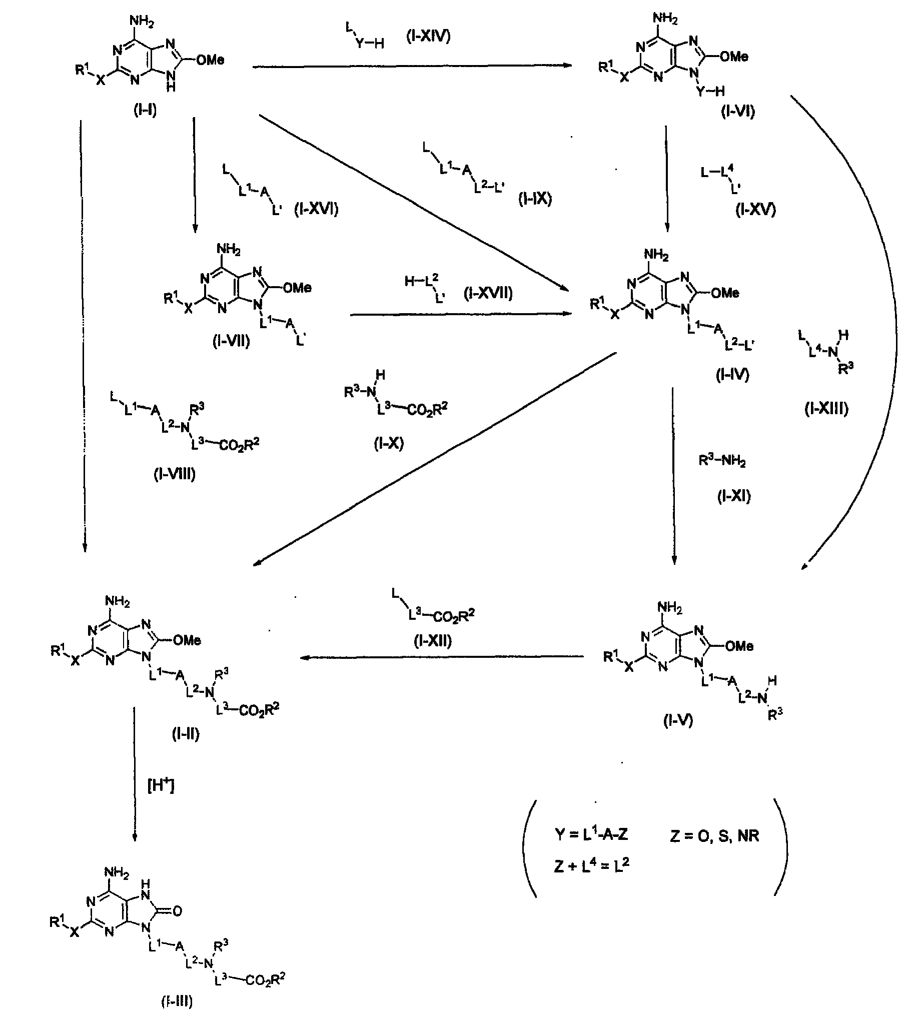

In the above Scheme, L and L' are leaving groups which may be the same or different each other; A, R¹, R², R³, X, L¹, L² and L³ are the same as defined above; "-Y-" is a group of the following formula: 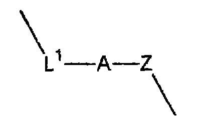 wherein Z is oxygen, sulfur or NR⁴ in which R⁴ is the same as defined above, and Z is combined together with L⁴ to represent L². The leaving groups may include halogen, sulfonyl such as p-toluenesulfonyl or methanesulfonyl in alkylation or acylation reaction, etc.

In case that the compound or the intermediate thereof of the present invention has a functional group such as amino, carboxy, hydroxyl or oxo, a protection or deprotection technique may be applied, if necessary. A preferable protective group, a method for protection and deprotection are particularly described in "Protective Groups in Organic Synthesis 2nd Edition (John Wiley & Sons, Inc.; 1990)", etc.

Compound (I-I) may be reacted with compound (I-VIII) in the presence of a base to give compound (I-II). For example, the base which may be used therein includes alkali metal carbonate such as sodium carbonate or potassium carbonate, alkaline earth metal carbonate such as calcium carbonate, metal hydroxide such as sodium hydroxide or potassium hydroxide, metal hydride such as sodium hydride, or metal alkoxide such as potassium t-butoxide, etc. For example, the solvent which may be used therein includes aprotic solvent such as dimethylformamide, dimethylsulfoxide or acetonitrile, halogenated hydrocarbon solvent such as carbon tetrachloride, chloroform or methylene chloride, ether solvent such as diethyl ether, tetrahydrofuran or 1,4-dioxane, etc. For example, the reaction temperature is selected from the range of about 0°C to around a boiling point of the solvent.

Compound (I-II) may be treated under an acidic condition to give compound (I-III). For example, the acid used in the acid-treatment includes an inorganic acid such as hydrochloric acid, hydrobromic acid or sulfuric acid, or an organic acid such as trifluoroacetic acid, etc. For example, the solvent which may be used therein includes water, or a mixture of water and an organic solvent. The organic solvent includes ether solvent such as diethyl ether or tetrahydrofuran, aprotic solvent such as dimethylformamide or acetonitrile, or alcoholic solvent such as methanol or ethanol, etc. The reaction temperature is, for example, selected from the range of room temperature to around a boiling point of the solvent. The conversion of methoxy on 8-position of the adenine ring into oxo by acid treatment may be carried out not in the final step but in any steps.

Compound (I-VIII) may be prepared by the following methods.

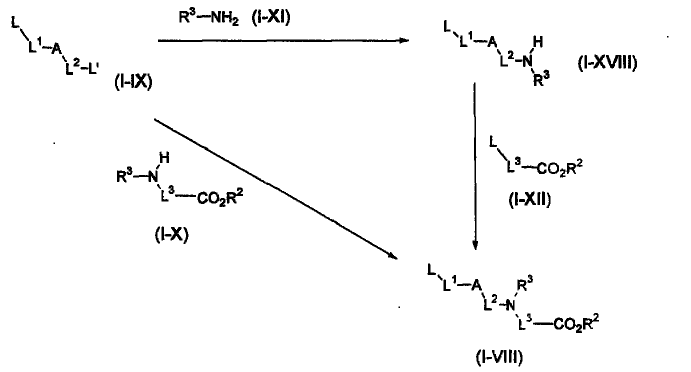

In the above Scheme, L and L' are leaving groups which may be the same or different each other; A, R², R³, L¹, L² and L³ are the same as defined above.
Compound (I-IX) may be reacted with compound (I-X) in the similar manner to the above to give compound (I-VIII). Alternatively, compound (I-IX) may be reacted with compound (I-XI) in the similar manner to the above to give compound (I-XVIII), followed by reacting with compound (I-XII) in the similar manner to the above to give compound (I-VIII).

In the preparation step from compound (I-I) to compound (I-II), compound (I-I) may be also reacted with compound (I-IX) in the similar manner to the above to give compound (I-IV), followed by reacting with compound (I-X) in the similar manner to the above to give compound (I-II).

In the preparation step from compound (I-IV) to compound (I-II), compound (I-IV) may be also reacted with compound (I-XI) in the similar manner to the above to give compound (I-V), followed by reacting with compound (I-XII) in the similar manner to the above to give compound (I-II).

In the preparation step from compound (I-I) to compound (I-IV), compound (I-I) may be also reacted with compound (I-XIV) in the similar manner to the above to give compound (I-VI), followed by reacting with compound (I-XV) in the similar manner to the above to give compound (I-IV). Compound (I-VI) may be also reacted with compound (I-XIII) to give compound (I-V). Alternatively, compound (I-I) may be also reacted with compound (I-XVI) in the similar manner to the above to give compound (I-VII), followed by reacting with compound (I-XVII) in the similar manner to the above to give compound (I-IV).

In each step, each compound may be also prepared according to a synthetic method known to those skilled in the art (e.g., alkylation reaction, dehydrative condensing reaction of carboxylic acid and amine compound, or reductive alkylation reaction of amine compound, etc.) optionally selected depending on a structure of each preparation intermediate.

Compound (I-I) may be prepared according to the following methods.

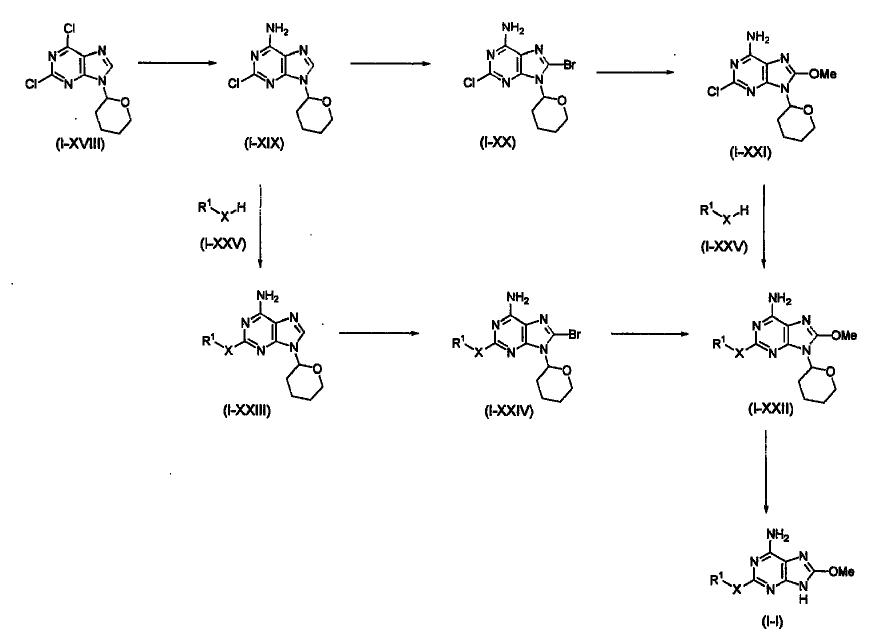

In the above Scheme, R¹ and X are the same as defined above.

Compound (I-XVIII) may be reacted with ammonia in aqueous solution, organic solvent or a mixture of water and organic solvent to give compound (I-XIX).

For example, the organic solvent includes alcoholic solvent such as methanol, ethanol, propanol or butanol, ether solvent such as tetrahydrofuran, 1,4-dioxane or diglyme, aprotic solvent such as acetonitrile, etc. For example, the reaction temperature is selected from the range of room temperature to 200°C. A reaction container such as autoclave may be used in the reaction.

Compound (I-XIX) may be brominated to give compound (I-XX). For example, a brominating agent which may be used therein includes bromine, hydrobromic acid perbromide or N-bromosuccinimide, etc., and for example, a reaction auxiliary such as sodium acetate may be added to the reaction. For example, the solvent which may be used therein includes halogenated hydrocarbon solvent such as carbon tetrachloride, methylene chloride or dichloroethane, ether solvent such as diethyl ether, acetic acid, or carbon disulfide, etc. For example, the reaction temperature is selected from the range of about 0°C to around a boiling point of the solvent.

Compound (I-XX) may be reacted with sodium methoxide to give compound (I-XXI).

For example, the organic solvent which may be used therein includes ether solvent such as diethyl ether, tetrahydrofuran or 1,4-dioxane, aprotic solvent such as dimethylformamide, or alcoholic solvent such as methanol, etc. For example, the reaction temperature is selected from the range of room temperature to around a boiling point of the solvent.

Compound (I-XX) may be also treated in an aqueous alkaline solution containing methanol to give compound (I-XXI).

The aqueous alkaline solution which may be used therein includes an aqueous solution of alkali metal hydroxide such as sodium hydroxide or potassium hydroxide. For example, the reaction temperature is selected from the range of room temperature to around a boiling point of the solvent.

Compound (I-XXI) may be reacted with compound (I-XXV) to give compound (I-XXII).

The reaction is carried out in the presence or absence of a base in case that X is NR⁴ wherein R⁴ is the same as defined above. For example, the base which may be used therein includes alkali metal carbonate such as sodium carbonate or potassium carbonate, alkaline earth metal carbonate such as calcium carbonate, metal hydroxide such as sodium hydroxide or potassium hydroxide, or an organic base such as triethylamine, diisopropylethylamine or 4-dimethylaminopyridine, etc. For example, the solvent which may be used therein includes ether solvent such as tetrahydrofuran, 1,4-dioxane or diglyme, alcoholic solvent such as propanol or butanol, or aprotic solvent such as dimethylformamide, or the reaction may be carried out in the absence of solvent. For example, the reaction temperature is selected from the range of about 50°C to 200°C.

The reaction is carried out in the presence of a base in case that X is oxygen or sulfur. For example, the base which may be used therein includes alkali metal such as sodium or potassium, or alkali metal hydride such as sodium hydride. For example, the solvent which may be used therein includes ether solvent such as tetrahydrofuran, 1,4-dioxane or diglyme, or aprotic solvent such as dimethylformamide or dimethylsulfoxide, or the reaction may be carried out in the absence of solvent. For example, the reaction temperature is selected from the range of about 50°C to 200°C.

The reaction may be carried out by oxidizing the corresponding intermediate for preparation wherein X is sulfur with oxone^{™} or m-chloroperbenzoic acid (mCPBA) in case that X is SO₂.

Alternatively, in the preparation step from compound (I-XIX) to compound (I-XXII), compound (I-XXIII) may be synthesized in the similar manner to the above to give compound (I-XXIV), followed by obtaining compound (I-XXII).

Compound (I-XXII) may be treated with trifluoroacetic acid in an organic solvent such as methanol to give compound (I-I).

For example, the acid which may be used therein includes an inorganic acid such as hydrochloric acid, hydrobromic acid or sulfuric acid, or an organic acid such as trifluoroacetic acid. For example, the solvent which may be used therein includes water, or a mixture of water and an organic solvent. The organic solvent includes ether solvent such as diethyl ether or tetrahydrofuran, aprotic solvent such as dimethylformamide or acetonitrile, or alcoholic solvent such as methanol or ethanol. For example, the reaction temperature is selected from the range of room temperature to around a boiling point of the solvent.

The compound of the general formula (1) may be also prepared by the following methods using compound (II-I) as a starting compound. The starting compound (II-I) is disclosed in WO 2002/085905 and WO 2004/029054 in detail. Starting compounds which are not described below may be prepared according to the following methods or known methods or those similar thereto.

### Preparation Method 2

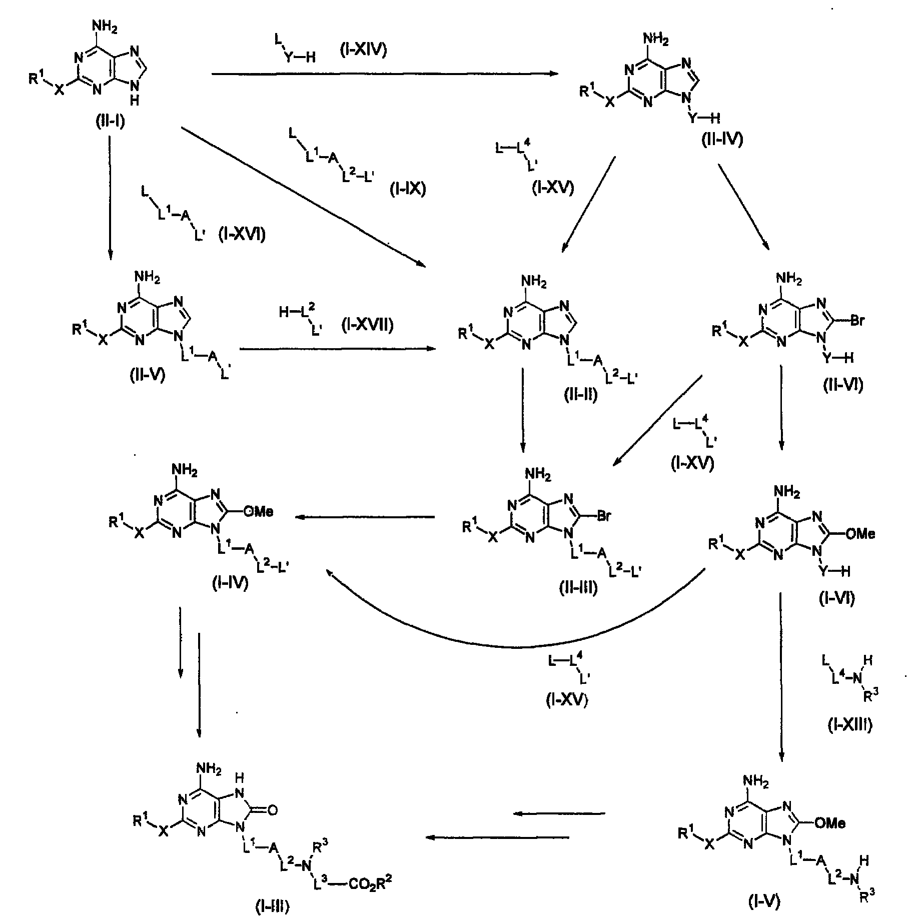

Compound (II-I) may be reacted with compound (I-IX) in the presence of a base to give compound (II-II). For example, the base which may be used therein includes alkali metal carbonate such as sodium carbonate or potassium carbonate, alkaline earth metal carbonate such as calcium carbonate, metal hydroxide such as sodium hydroxide or potassium hydroxide, metal hydride such as sodium hydride, or metal alkoxide such as potassium t-butoxide. For example, the solvent which may be used therein includes aprotic solvent such as dimethylformamide, dimethylsulfoxide or acetonitrile, halogenated hydrocarbon solvent such as carbon tetrachloride, chloroform or methylene chloride, ether solvent such as diethyl ether, tetrahydrofuran or 1,4-dioxane. For example, the reaction temperature is selected from the range of about 0°C to around a boiling point of the solvent.

Alternatively, in the preparation step from compound (II-I) to compound (II-II) in the similar manner to the above, compound (II-II) may also be obtained via synthesis of compound (II-IV) or compound (II-V).

Compound (II-II) may be brominated to give compound (II-III). For example, a brominating agent which may be used therein includes bromine, hydrobromic acid perbromide or N-bromosuccinimide, etc., and for example, a reaction auxiliary such as sodium acetate may be added to the reaction. For example, the solvent which may be used therein includes halogenated hydrocarbon solvent such as carbon tetrachloride, methylene chloride or dichloroethane, ether solvent such as diethyl ether, acetic acid, or carbon disulfide, etc. For example, the reaction temperature is selected from the range of about 0°C to around a boiling point of the solvent.

Compound (II-III) may be reacted with metal alkoxide such as sodium methoxide to give compound (I-IV).

For example, the solvent which may be used in the reaction with metal alkoxide includes ether solvent such as diethyl ether, tetrahydrofuran or 1,4-dioxane, aprotic solvent such as dimethylformamide, or alcoholic solvent corresponding to metal alkoxide used therein such as methanol, etc. For example, the reaction temperature is selected from the range of room temperature to around a boiling point of the solvent.

Alternatively, compound (II-IV) may be also brominated in the similar manner to the above to give compound (II-VI), followed by converting into compound (II-III) or compound (I-VI) in the similar manner to the above to give compound (I-IV).

Compound (I-III) may be obtained using compound (I-IV) in the method described above. Alternatively, compound (I-III) may be also obtained via compound (I-VI) and compound (I-V).

The adenine compounds, intermediates or starting compounds thereof with any functional groups in the present invention may be optionally subjected to homologation reaction, substituent introduction reaction or functional group transformation reaction, etc. in an appropriate step, or more specifically, in any halfway step of each preparation method described in the above Preparation Method 1 or 2 according to a conventional method known to those skilled in the art. For these reactions, a method described in "Jikken-Kagaku-Koza (edited by the Chemical Society of Japan, Maruzen)", or "Comprehensive Organic Transformation, Author: R. C. Larock, (VCH Publishers, Inc, 1989)", etc. may be used. The homologation reaction includes, for example, a method wherein ester is converted into hydroxymethyl using a reducing agent such as lithium aluminum hydride, followed by introducing a leaving group to introduce cyano, etc. The functional group transformation reaction includes, for example, acylation or sulfonylation reaction using acid halide, sulfonyl halide, etc., a reaction using alkylating agent such as halogenated alkyl, hydrolysis reaction, carbon-carbon bond formation reaction such as Friedel-Crafts reaction or Wittig reaction, oxidation or reduction reaction, etc.

When the compound of the present invention or an intermediate thereof contains a functional group such as amino, carboxy, hydroxyl or oxo in the present invention, a protection or deprotection technique may optionally be applied. A preferable protective group, a method for protection and deprotection are specifically described in "Protective Groups in Organic Synthesis 2nd Edition (John Wiley & Sons, Inc.; 1990)", etc.

The compound of the formula (1) or an intermediate for preparing the same of the present invention may be purified by a method known to those skilled in the art. For example, it may be purified by column chromatography (e.g., silica gel column chromatography, or ionexchange column chromatography), or recrystallization, etc. The solvent which may be used in the recrystallization includes, for example, alcoholic solvents such as methanol, ethanol or 2-propanol, ether solvents such as diethyl ether, ester solvents such as ethyl acetate, aromatic hydrocarbon solvents such as benzene or toluene, ketone solvents such as acetone, hydrocarbon solvents such as hexane, aprotic solvents such as dimethylformamide or acetonitrile, water, or a mixture thereof, etc. Other purification methods include a method described in Jikken-Kagaku-Koza (edited by the Chemical Society of Japan, Maruzen), vol. 1, etc.

The compound of the formula (1) with 1 or more asymmetric center(s) of the present invention may be prepared by using a starting material with asymmetric centers or introducing asymmetric centers in any half way steps according to a conventional method. For example, enantiomers may be obtained by using optically active starting materials or carrying out optical resolution in an appropriate step of the preparation. For example, the optical resolution may be carried out by a diastereomeric method wherein the compound of the formula (1) or an intermediate thereof is reacted with an optically active acid (e.g., monocarboxylic acid such as mandelic acid, N-benzyloxyalanine or lactic acid, dicarboxylic acid such as tartaric acid, o-diisopropylidene tartaric acid or malic acid, or sulfonic acid such as camphorsulfonic acid or bromocamphorsulfonic acid) to form a salt thereof in an inactive solvent (e.g., alcoholic solvent such as methanol, ethanol or 2-propanol, ether solvent such as diethyl ether, ester solvent such as ethyl acetate, hydrocarbon solvent such as toluene, or aprotic solvent such as acetonitrile, and a mixture thereof).

The optical resolution may be also carried out by reacting the compound of the formula (1) or an intermediate thereof having an acidic functional group such as carboxy with an optically active amine (e.g., organic amine such as α-phenethylamine, quinine, quinidine, cinchonidine, cinchonine, strychnine) to form a salt thereof.

A temperature for forming the salt is selected from the range of room temperature to a boiling point of the solvent. In order to improve an optical purity, it is desirable to raise the temperature up to around a boiling point of the solvent. The precipitated salt may be cooled in filtration to improve its yield as necessary. The usage of an optically active acid or amine is properly in the range of about 0.5 to about 2.0 equivalents, preferably around 1 equivalent, to the substrate. The crystal may be also, as necessary, recrystallized in an inactive solvent (e.g., alcoholic solvent such as methanol, ethanol, 2-propanol, ether solvent such as diethyl ether, ester solvent such as ethyl acetate, hydrocarbon solvent such as toluene, aprotic solvent such as acetonitrile, and a mixture thereof) to give an optically active salt in high purity. The optically resolved salt may be also, as necessary, treated with acid or base in a conventional manner to give in a free form.

The adenine compound, or a pharmaceutically acceptable salt thereof of the present invention activates toll-like receptor (TLR), specifically TLR7, and is useful as an immune-regulating agent and a therapeutic or preventive agent for diseases such as diseases associated with abnormality of immune response (e.g., autoimmune diseases and allergic diseases), various infectious diseases wherein an immune response is desired to be activated or cancer. For example, the adenine compound or a pharmaceutically acceptable salt thereof of the present invention is useful as a therapeutic or preventive agent for diseases including the following (1) to (8).

(1) Respiratory affections, including intermittent or persistent asthma of every severity (e.g., bronchial asthma, allergic asthma, intrinsic asthma, extrinsic asthma, exercise-induced asthma, asthma induced by drug (e.g., NSAID such as aspirin and indometacin), dust-induced asthma, and reactive airway diseases caused by other factors); chronic obstructive lung disease (COPD); bronchitis (e.g., infectious bronchitis, eosinophilic bronchitis); emphysema; bronchiectasis; cystic fibrosis; sarcoidosis; farmer's lung and related diseases thereof; hypersensitivity pneumonitis; lung fibrosis (e.g., cryptogenic fibrosing alveolitis, idiopathic interstitial pneumonias, and fibrosis caused by antineoplastic therapy, chronic infectious diseases including tuberculosis bacterial, aspergillus or other fungal infectious diseases, etc.); complication by lung transplantation; vascular and thrombotic pulmonary disease and pulmonary hypertension; antitussive including treatment of chronic cough and iatrogenic cough associated with inflammation or secretion of airway; acute or chronic rhinitis including rhinitis medicamentosa or vasomotor rhinitis; perennial or seasonal allergic rhinitis including rhinitis nervosa (hay fever); nasal polyposis; acute virus infection including common cold disease and respiratory infectious diseases by respiratory syncytium virus, influenza, coronavirus (including SARS) and adenovirus;

(2) Cutaneous diseases, including psoriasis, atopic dermatitis, contact dermatitis and other eczematous dermatoses, and delayed-type hypersensitivity reaction; phyto- and photodermatitis; seborrheic dermatitis, herpetiformis dermatitis; lichen planus, lichen sclerosis, lichen sclerosus et atrophicus, pyoderma gangrenosum, dermal sarcoidosis, discoid lupus erythematosus, pemphigus, pemphigoid, epidermolysis bullosa, urticaria, angioedema, vasculitides, toxic erythema, cutaneous eosinophilia, alopecia areata, male pattern baldness, Sweet's syndrome, Weber-Christian syndrome, erythema multiforme; infectious or noninfectious cellulitis; panniculitis; cutaneous lymphoma, nonmelanoma skin cancer or other dysplasia lesions; drug-induced disease including fixed drug eruption;

(3) Eye diseases, including blepharitis; conjunctivitis including perennial and vernal allergic conjunctivitis; iritis; anterior and posterior uveitis; choroiditis; retinal disease associated with autoimmune, denaturation or inflammation; ophthalmia including sympathetic ophthalmia; sarcoidosis; viral, fungal or bacterial infectious diseases;

(4) Genitourinary diseases, including nephritis including interstitial and glomerulonephritis; nephrotic syndrome; cystitis including acute or chronic (interstitial) cystitis and Hunner's ulcer; acute or chronic urethritis, prostatitis, epididymitis, oophoritis and salpingitis; vulvovaginitis; Peyronie's disease; erectile dysfunction (male and female);

(5) Allograft rejections, including acute and chronic rejection after transplantation of kidney, heart, liver, lung, bone marrow, skin or cornea, or after blood transfusion, etc.; or chronic graft-versus-host disease;

(6) Autoimmune diseases, including chronic rheumatoid arthritis, ulcerative colitis, systemic lupus erythematosus, multiple sclerosis, Hashimoto's thyroiditis, Grave's disease, Addison's disease, diabetes, idiopathic thrombocytopenic purpura, eosinophilic fasciitis, hyper IgE syndrome, or other autoimmune diseases and allergic diseases such as autoimmune disease syndrome including antiphospholipid antibody syndrome;

(7) Cancer diseases, including prostate cancer, breast cancer, lung cancer, uterus cancer, pancreas cancer, liver cancer, colon cancer, stomach cancer, skin cancer or brain tumor, and malignant bone marrow neoplasm (e.g., leukemia) and lymphoproliferative tumor such as Hodgkin's lymphoma or non-Hodgkin's lymphoma. It is useful for usual treatment of these cancer diseases and also for prevention or treatment of metastasis, tumor recurrence and paraneoplastic syndrome;

(8) Infectious diseases, including viral infectious diseases such as genital wart, common wart, plantar wart, hepatitis B, hepatitis C, herpes simplex viral disease, molluscum contagiosum, variola, acquired immune deficiency syndrome (HIV), or infectious diseases caused by human papillomavirus (HPV), cytomegalovirus (CMV), varicella-zoster virus (VZV), rhinovirus, adenovirus, coronavirus, influenza virus or parainfluenza virus; bacterial diseases such as tuberculosis, mycobacterium avium complex, or leprosy; other infectious diseases such as infectious diseases caused by various fungi, candida, chlamydia or aspergillus, cryptococcus meningitis, carinii pneumonia, cryptosporidiosis, histoplasmosis, toxoplasmosis, trypanosome infectious diseases, or leishmaniasis.

The adenine compound or a pharmaceutically acceptable salt thereof of the present invention is also useful as a vaccine adjuvant.

The adenine compound or a pharmaceutically acceptable salt thereof of the present invention has a TLR activating effect, more specifically a TLR7 activating effect. The adenine compound or a pharmaceutically acceptable salt thereof of the present invention also shows interferon-α- and interferon-γ-inducing activity, and IL-4/IL-5 producing inhibition activity, and acts as an agent with helper T cell type 1 (Th1 cell) / helper T cell type 2 (Th2 cell) selective immunoregulatory activity. In other words, it is preferably useful as a therapeutic or preventive agent for allergic diseases caused by Th2 cell such as asthma, COPD, allergic rhinitis, allergic conjunctivitis or atopic dermatitis due to its Th2 cell selective immunosuppressive action. On the other hand, owing to its immunostimulatory action, they are also useful as a therapeutic or preventive agent for various diseases, such as viral infectious diseases (e.g., cancer, hepatitis B, hepatitis C, acquired immune deficiency syndrome (HIV), human papillomavirus disease (HPV)), bacterial infectious diseases, skin diseases (e.g., psoriasis), etc.

The adenine compound or a pharmaceutically acceptable salt thereof of the present invention is useful for treatment of airway obstruction diseases/conditions such as asthma or COPD, or for reducing the risk of these diseases.

The adenine compound or a pharmaceutically acceptable salt thereof of the present invention may be orally or parenterally administered without any limitation to the dosage forms. For example, an oral preparation may include capsules, powders, tablets, granules, subtle granules, syrups, liquids, suspensions, etc., and a parenteral preparation may include injections, drips, eye-drops, preparations for intrarectal administration, inhalations, air sprays (e.g., liquid/suspensions for sprays, aerosols, dry powders or for cartridge sprays for inhalators or insufflators, etc.), lotions, gels, ointments, creams, transdermal absorbents, transmucosal absorbents, nasal preprations, eardrops, tapes, transdermal patches, cataplasms, external powders, etc. These preparations may be prepared according to a conventional technique, and may contain conventional carriers, excipients, binders, lubricants, stabilizers, disintegrants, buffers, solubilizing agents, isotonic agents, surfactants, antiseptic agents, perfumes, and further optionally contains two or more kinds of additives for preparations.

The adenine compound or a pharmaceutically acceptable salt thereof of the present invention may be incorporated with a pharmaceutically acceptable carrier in a manner known to those skilled in the art to prepare a pharmaceutical composition suitable for each dosage form. For example, the adenine compound or a pharmaceutically acceptable salt thereof may be formed into a pharmaceutical composition comprising as an active ingredient 0.05 to 99% by weight, preferably 0.05 to 80% by weight, more preferably 0.1 to 70% by weight, more preferably 0.1 to 50% by weight of the compound.

Among the oral preparations, liquid preparations such as emulsions and syrups may be prepared by optionally using additives for preparations including water; sugars such as sucrose, sorbit, fructose; ethanol; glycols such as polyethyleneglycol, propyleneglycol, glycerol; oils such as sesame oil, olive oil, soybean oil; preservative such as p-hydroxybenzoate; sweetener such as saccharin; thickener such as carboxymethylcellulose; flavors such as strawberry flavor, peppermint flavor, or colorants etc.

Solid preparations such as capsules, tablets, powders, granules, etc. may be prepared by optionally compounding the following carriers. Specifically, they may be prepared by using excipient such as lactose, glucose, sucrose, sorbitol, mannitol (mannite), cellulose derivatives; disintegrant such as starch (e.g., potato starch, cornstarch, amylopectin), sodium alginate; lubricant such as magnesium stearate, calcium stearate, polyethyleneglycol, wax, paraffin, talc; binder such as polyvinyl alcohol, polyvinylpyrrolidone, hydroxypropylcellulose, gelatin; surfactant such as fatty acid ester; plasticizer such as glycerin, etc. Sugar coated tablets may be coated by concentrated carbohydrate solutions, optionally containing gum arabic, gelatin, talc, titanium oxide, etc., on tablet cores prepared by using the above carriers. Alternatively, tablets may be film-coated by appropriate polymers dissolved in organic solvents which may be easily distilled away.

Soft gelatin capsules may be prepared by, for example, compounding the present compound with vegetable oil or polyethyleneglycol. Hard gelatin capsules may be prepared by using granules of the present compound which may be prepared by optionally compounding any one of the above carriers.

Among the parenteral preparations, liquid preparations in the form of injections, drips, eye-drops, eardrops, etc. may be preferably prepared as sterile isotonic liquid preparations. For example, the injections may be prepared by using aqueous media comprising saline solution, glucose solution, or a mixture of saline solution and glucose solution. The preparations for intrarectal administration may be prepared by using carriers such as cacao butter, and usually prepared in the form of suppositories.

The ointments, creams and gels usually contain 0.01 to 10% by weight of the present compound, and to aqueous or oily base may be optionally added preferable thickener and/or gelatinizing agent and/or solvent. For example, the base includes water and/or oil such as liquid paraffin, vegetable oil such as peanut oil or castor oil, or solvent such as polyethyleneglycol. The thickener and gelatinizing agent include soft paraffin, aluminum stearate, cetostearyl alcohol, polyethyleneglycol, lanolin, bee wax, carboxypolymethylene and cellulose derivative and/or glyceryl monostearate and/or nonionic emulsifier.

The lotions usually contain 0.01 to 10% by weight of the present compound, and may be formulated by aqueous or oily base and may typically comprise emulsifier, stabilizer, dispersing agent, precipitation inhibitor or thickener.

The external powders usually contain 0.01 to 10% by weight of the present compound, and may be formed by preferable powder base such as talc, lactose or starch.

The drips may be formulated by aqueous or nonaqueous base and may contain dispersing agent, solubilizer, precipitation inhibitor or preservative.

The air spray (e.g., spray, aerosol, dry powder preparation, etc.) may be optionally formulated as aqueous solution or suspension, or aerosol delivered from pressurized pack such as quantitative dose inhalators by using, for example, a preferable liquefied propellant. Dry powder preparation may be also used.

The aerosol appropriate for inhalation may be either suspension or solution, and typically contains the present compound and any appropriate propellants such as fluorocarbon or hydrogen-containing chlorofluorocarbon or a mixture thereof. Specifically, it contains hydrofluoroalkane, particularly 1,1, 1,2-tetrafluoroethane, heptafluoroalkane (HFA) such as 1,1,1,2,3,3,3-heptafluoro-n-propane, or a mixture thereof. The aerosol may optionally contain additional preparation excipient well-known to those skilled in the art such as surfactant (e.g., oleic acid or lecithin) and cosolvent (e.g., ethanol), etc. Specifically, it may include inhalator known as "Turbuhaler^{™}".

For example, capsule or cartridge of gelatin used in inhalators or insufflators may be formulated containing a powder mixture and preferable powder base such as lactose or starch for inhalation of the compound used in the present invention. Each capsule or cartridge usually contains 20 µg to 10 mg of the present compound. Alternatively, the compound used in the present invention may be provided without an excipient such as lactose.

In case of oral or nasal inhalation as pressurized HFA aerosol and dry powder preparation, etc., the adenine compound or a pharmaceutically acceptable salt thereof of the present invention may be finely ground into 10 µm or less to suspend in fatty acid with 8 to 20 carbon atoms or a salt thereof (e.g., oleic acid), bile acid salt, phospholipid, alkyl saccharide, fully-fluorinated or polyethoxylated surfactant, or other pharmaceutically acceptable dispersing agent.

It is preferable that the adenine compound in the present invention is parenterally administered as a preparation for local administration. Specifically, the preferable preparation includes ointment, lotion (solution or suspension), cream, gel, tape, transdermal patch, cataplasm, spray, aerosol, dry powder preparation, water/suspensions for cartridge sprays for inhalator or insufflator, eye-drops, eardrops, nasal drops, transdermal patches, lung absorbents, airway absorbents or external powders, etc.

In the preparation for local administration in the present invention, the ratio of the active compound used in the present invention is generally 0.001 to 10% by weight, preferably 0.005 to 1% by weight depending on the forms of preparations. The ratio used in powders for inhalation or ventilation is in the range of 0.1 to 5% by weight.

Each quantitative dose or "one-sprayed amount" in the aerosol preferably contains 20 µg to 2000 µg, preferably about 20 µg to 500 µg of the compound used in the present invention. The administration may be once or several times a day, for example 2, 3, 4 or 8 times a day, for example 1, 2 or 3 units each.

The pharmacological activity may be measured in any assessments well-known to those skilled in the art, preferably in vitro assessments. Specific measuring method includes the one discribed in Examples in the present specification.

The present invention also encompasses a combination therapy for treating diseases described in the present specification wherein the compound of the formula (1) or a pharmaceutically acceptable salt thereof, or a pharmaceutical composition comprising the compound of the formula (1) or a pharmaceutically acceptable salt thereof is sequentially or simultaneously administered in combination with 1 or more of other following medicaments.

Particularly, the medicaments for treating inflammatory disease, COPD, asthma and allergic rhinitis include TNF-α inhibitor such as anti TNF monoclonal antibody (e.g., Remicade, CDP-870 and adalimumab) or TNF receptor immunoglobulin molecule (e.g., enbrel); locally- or systemically-administered nonselective cyclooxygenase: COX-1/COX-2 inhibitor (e.g., piroxicam, diclofenac, propionic acids such as naproxen, flurbiprofen, fenoprofen, ketoprofen and ibuprofen, fenamate such as mefenamic acid, indomethacin, sulindac, azapropazone, pyrazolone such as phenylbutazone, salicylate salt such as aspirin), COX-2 inhibitor (e.g., meloxicam, celecoxib, rofecoxib, valdecoxib, lumarocoxib, parecoxib and etoricoxib); glucocorticoid which is administered locally, orally, intramuscularly, intravenously or intraarticularly; methotrexate, leflunomide; hydroxychloroquine, d-penicillamine, auranofin, or other parenteral or oral gold preparation, etc.

The present invention also encompasses a combination of the present compounds with leukotriene biosynthetic inhibitor, 5-lipoxygenase (5-LO) inhibitor or 5-lipoxygenase activated protein (FLAP) antagonist, for example zileutone; ABT-761; fenleutone; tepoxalin; Abbott-79175; Abbott-85761; N-(5-substituted)-thiophen-2-alkylsulfonamide; 2,6-di-tert-butylphenolhydrazone; methoxytetrahydropyrane such as Zeneca ZD-2138; SB-210661; pyridinyl-substituted-2-cyanonaphthalene compound such as L-739010; 2-cyanoquinoline compound such as L-746530; MK-591, MK-886 and BAY-X-1005, etc.

The present invention also encompasses a combination therapy of the present compound with leukotriene (LT) B4, LTC4, LTD4, LTE4 receptor antagonist selected from the following group:
phenothiazine compound such as L-651392; amidino compound such as CGS-25019c; benzoxalamine such as ontazolast; benzenecarboximidamide such as BIIL284/260; and compounds such as zafirlukast, ablukast, montelukast, pranlukast, Verlukast (MK-679), RG-12525, Ro-245913, iralukast (CGP45715A) and BAY-X-7195, etc.

The present invention also encompasses a combination therapy of the present compound with phosphodiesterase (PDE) inhibitor such as methylxanthanin including theophylline and aminophylline; selective PDE isoenzyme including PDE4 inhibitor, isoform PDE4D inhibitor or PDE5 inhibitor.

The present invention also encompasses a combination therapy of the present compound which is orally or topically administered with, for example, histamine H1 receptor antagonist such as cetirizine, loratadine, desloratadine, fexofenadine, acrivastine, terfenadine, astemizole, azelastine, levocabastine, chlorpheniramine, promethazine, cyclizine or mizolastine, etc.

The present invention also encompasses a combination therapy of the present compound with histamine type 2 receptor antagonists which protect gastrointestinal.

The present invention also encompasses a combination therapy of the present compound with histamine type 4 receptor antagonists.

The present invention also encompasses a combination therapy of the present compound with α1/α2 adrenaline receptor agonist and vasoconstrictive sympathetic stimulant such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, or ethyl norepinephrine hydrochloride.

The present invention also encompasses a combination therapy of the present compound with anticholinergic agent including muscarinic receptor (M1, M2 and M3) antagonist such as atropine, hyoscine, glycopyrrolate, ipratropium bromide; tiotropium bromide; oxytropium bromide; pirenzepine; or telenzepine.

The present invention also encompasses a combination therapy of the present compound with β-adrenaline receptor agonist including β receptor subtypes 1 to 4 such as isoprenaline, salbutamol, formoterol, salmeterol, terbutaline, orciprenaline, bitolterol mesylate or pirbuterol.

The present invention also encompasses a combination therapy of the present compound with chromone such as sodium cromoglycate or nedocromil sodium.

The present invention also encompasses a combination therapy of the present compound with insulin-like growth factor type 1 (IGF-1) mimic.

The present invention also encompasses a combination therapy of the present compound with inhaled glucocorticoid such as flunisolide, triamcinolone acetonide, beclomethasone dipropionate, budesonide, fluticasone propionate, ciclesonide or mometasone furoate.

The present invention also encompasses a combination therapy of the present compound with matrix metalloprotease inhibitor, specifically inhibitor of stromelysin, collagenase, gelatinase, aggrecanase, particularly collagenase-1 (MMP-1), collagenase-2 (MMP-8), collagenase-3 (MMP-13), stromelysin-1 (MMP-3), stromelysin-2 (MMP-10) and stromelysin-3 (MMP-11), MMP-9 or MMP-12.

The present invention also encompasses a combination therapy of the present compound with chemokine receptor regulators of antagonists of CCR1, CCR2, CCR2A, CCR2B, CCR3, CCR4, CCR5, CCR6, CCR7, CCR8, CCR9, CCR10 and CCR11 (CC family); CXCR1, CXCR2, CXCR3, CXCR4 and CXCR5 (C-X-C family); C-X3-C family such as CX3CR1.

The present invention also encompasses a combination therapy of the present compound with cytokine function regulator including cytokine or medicaments acting on cytokine signaling pathway, for example α-, β- and γ-interferon, interleukin (IL) including IL1 to 15, and interleukin antagonist or inhibitor.

The present invention also encompasses a combination therapy of the present compound with immunoglobulin (Ig), immunoglobulin preparations, or antibodies and antagonists regulating Ig functions such as anti IgE antibody (omalizumab).

The present invention also encompasses a combination therapy of the present compound with systemically- or locally-administered antiinflammatory drugs such as thalidomide or derivatives thereof, retinoid, dithranol or calcipotriol.

The present invention also encompasses a combination therapy of the present compound with antibacterial agents such as penicillin derivative, tetracycline, macrolide, β-lactam, fluoroquinolone, metronidazole and inhaled aminoglycoside; and antiviral agents including acyclovir, famciclovir, valaciclovir, ganciclovir, cidofovir, amantadine, rimantadine, ribavirin; zanamivir, oseltamavir; enzyme inhibitor such as indinavir, nelfinavir, ritonavir and saquinavir; nucleoside reverse transcriptase inhibitor such as didanosine, lamivudine, stavudine, zalcitabine, zidovudine; or nonnucleoside reverse transcriptase inhibitor such as nevirapine or efavirenz.

The present invention also encompasses a combination therapy of the present compound with medicaments known as therapeutic agents for cancer. Preferable agents include the following (i) to (ix).
(i) Antiproliferative agents/antitumor agents and a combination thereof used as a therapeutic agent for tumors, for example alkylating agents (e.g., cisplatin, carboplatin, cyclophosphamide, nitrogen mustard, melphalan, chlorambucil, busulfan, or nitrosourea); antimetabolite (e.g., fluoropyrimidine such as 5-fluorouracil and tegafur, antifolate such as raltitrexed, methotrexate, cytosine arabinoside, hydroxyurea, gemcitabine or paclitaxel); antineoplastic antibiotics (e.g., anthracycline such as adriamycin, bleomycin, doxorubicin, daunomycin, epirubicin, idarubicin, mitomycin-C, dactinomycin, or mithramycin); antimitotic agents (e.g., vinca alkaloid such as vincristine, vinblastine, vindesine or vinorelbine, taxoid such as taxol and taxotere); or topoisomerase inhibitors (e.g., epipodophyllotoxine such as etoposide, teniposide, amsacrine, topotecan or camptothecin).

(ii) Cytostatic agents including antiestrogens (e.g., tamoxifen, toremifene, raloxifene, droloxifene or iodoxifene, etc.), estrogen receptor down regulators (e.g., fulvestrant), antiandrogenic agents (e.g., bicalutamide, flutamide, nilutamide, and cyproterone acetate), LHRH antagonists or LHRH agonists (e.g., goserelin, leuprorelin or buserelin), progestogen (e.g., megestrol acetate), aromatase inhibitors (e.g., anastrozole, letrozole, vorazole or exemestane) and 5α-reductase inhibitors (e.g., finasteride).

(iii) Inhibiting agents of invasion of cancer cells (e.g., metalloprotease inhibitors such as marimastat or inhibitors of urokinase plasminogen activating receptor functions).

(iv) Growth factor function inhibitors, e.g., growth factor antibody, growth factor receptor antibody (e.g., anti-erbb2 antibody trastuzumab or anti-erbbl antibody cetuximab [C225]), farnesyl transferase inhibitors, tyrosine kinase inhibitors, or serine/threonine kinase function inhibitors; e.g., epidermal growth factor inhibitors (e.g., EGFR family tyrosine kinase inhibitors such as N-(3-chloro-4-fluorophenyl)-7-methoxy-6-(3-morpholinopropoxy)quinazolin-4-amine (Gefitinib, AZD 1839), N-(3-ethynylphenyl)-6,7-bis(2-methoxyethoxy)quinazolin-4-amine (erlotinib, OSI-774) or 6-acrylamide-N-(3-chloro-4-fluorophenyl)-7-(3-morpholinopropoxy)quinazolin-4-amine (CI1033)); e.g., platelet-derived growth factor family inhibitors; or hepatocellular growth factor family inhibitors.

(v) Antiangiogenic agents, for example inhibiting agents of activity of vascular endothelial cell growth factor (e.g., anti-vascular endothelial cell growth factor antibody bevacizumab, compounds disclosed in international publications: WO97/22596, WO97/30035, WO97/32856 or WO98/13354), or compounds acting in other mechanisms (e.g., linomid, integrin αvβ3 function inhibitors or angiostatin).

(vi) Vascular damaging agents such as combretastatin A4 or compounds disclosed in international publications: WO99/02166, WO00/40529, WO00/41669, WO01/92224, WO02/04434 and WO02/08213.

(vii) Antisense therapeutics, for example antisense to the above targets such as ISIS2503, anti-ras antisense.

(viii) Gene therapy, for example aberrant gene exchanging approach such as aberrant p53 and aberrant BRCA1 or BRCA2, GDEPT (Gene-directed enzyme pro-drug therapy) approach using cytosine deaminase, thymidine kinase or bacterial nitroreductase enzyme, approach enhancing patients' tolerance for chemotherapy or radiotherapy such as multi drug resistance gene therapy.

(ix) Immunotherapy approach, for example approach for enhancing immunity to cancer cells of patients by exposuring cytokine such as interleukin 2, interleukin 4 or Granulocyte-Macrophage Colony Stimulating Factor (GM-CSF) ex-vivo or in-vivo, T cell anergy reducing approach, approach transplanting immune cells such as cytokine exposed dendritic cells, approach using cytokine exposed tumor cell line, and approach using anti-idiotypic antibody, etc.

The present invention is specifically described in the following Examples, but it is not limited thereto.

### EXAMPLES

### Example 1: 2-Butoxy-7,8-dihydro-9-[4-{4-[4-(methoxycarbonyl)-piperidin-1-yl]butoxy}benzyl]-8-oxoadenine

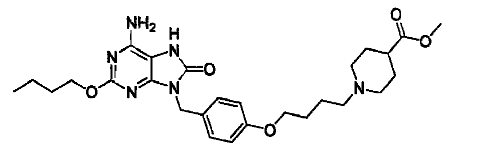

### Step (i): 9-(4-Benzyloxybenzyl)-2-butoxyadenine

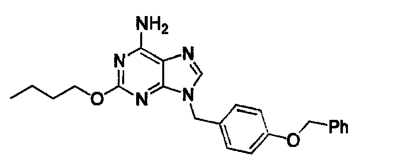

2-Butoxyadenine (7.2 g, 34.8 mmol) was suspended in DMF (150 ml), and thereto was added potassium carbonate (4.8 g, 34.8 mmol) at 60°C for 2 hours, and then the mixture was cooled to room temperature. Then, thereto was added 4-benzyloxybenzyl chloride (9.7 g, 41.8 mmol), and the mixture was stirred for 20 hours and evaporated. Then, thereto was added water, and the mixture was extracted with chloroform. The organic layer was dried over anhydrous magnesium sulfate, and then concentrated in vacuo. The residue was purified by silica gel column chromatography to give the subtitled compound (10.9 g) as a pale yellow solid. Yield 78%.
¹H NMR (CDCl₃) δ 7.58 (1H, s), 7.43-7.32 (5H, m), 7.25 (2H, d, J = 8.6 Hz), 6.94 (2H, d, J = 8.6 Hz), 5.93 (2H, brs), 5.20 (2H, s), 5.05 (2H, s), 4.35 (2H, t, J = 6.6 Hz), 1.79 (2H, tt, J = 7.6 Hz, 6.6 Hz), 1.50 (2H, tq, J = 7.6 Hz, 7.4 Hz), 0.97 (3H, t, J = 7.4 Hz).

### Step (ii): 9-(4-Benzyloxybenzyl)-8-bromo-2-butoxyadenine

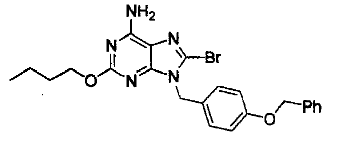

The compound obtained in Step (i) (10.9 g, 27.0 mmol) was dissolved in chloroform (250 ml), and thereto was added sodium acetate (4.0 g, 48.6 mmol) and the mixture was cooled to 0°C. Thereto was added dropwise bromine (6.47 g, 40.5 mmol), and the mixture was stirred at room temperature for 1 hour. The mixture was cooled to 0°C, and then thereto were added saturated sodium bicarbonate water and aqueous saturated sodium bisulfite solution, and the mixture was extracted with chloroform. The organic layer was dried over anhydrous magnesium sulfate, and then concentrated in vacuo to give the subtitled compound (13.0 g) as a pale yellow solid. Yield 100%.
¹H NMR (CDCl₃) δ 7.42-7.31 (7H, m), 6.91 (2H, d, J = 8.7 Hz), 6.08 (2H, brs), 5.23 (2H, s), 5.03 (2H, s), 4.35 (2H, t, J = 6.7 Hz), 1.79 (2H, tt, J = 7.5 Hz, 6.7 Hz), 1.51 (2H, tq, J = 7.5 Hz, 7.3 Hz), 0.97 (3H, t, J = 7.3 Hz).

### Step (iii): 9-(4-Benzyloxybenzyl)-2-butoxy-8-methoxyadenine

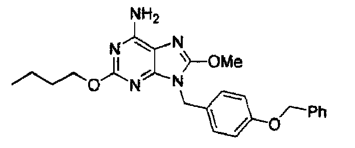

The compound obtained in Step (ii) (13.0 g, 27.0 mmol) was suspended in methanol (400 ml), and thereto was added 28% sodium methoxide/methanol solution (100 ml), and the mixture was heated to stir under refluxing for 3 hours. The mixture was cooled to 0°C, and then neutralized by acetic acid and evaporated, and thereto was added water and the precipitated solid was filtered. The solid was dried, and then purified by silica gel column chromatography to give the subtitled compound (9.05 g) as a white solid. Yield 77%.
¹H NMR (CDCl₃) δ 7.42-7.28 (7H, m), 6.91 (2H, d, J = 8.6 Hz), 5.29 (2H, brs), 5.03 (2H, s), 4.32 (2H, t, J = 6.7 Hz), 4.10 (3H, s), 1.78 (2H, tt, J = 7.5 Hz, 6.7 Hz), 1.50 (2H, tq, J = 7.5 Hz, 7.4 Hz), 0.97 (3H, t, J = 7.4 Hz).

### Step (iv): 2-Butoxy-9-(4-hydroxybenzyl)-8-methoxyadenine

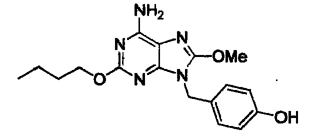

The compound obtained in Step (iii) (9.04 g, 20.9 mmol) was dissolved in THF (150 ml), and thereto was added 20% Pd(OH)₂/C (2.0 g), and the mixture was stirred for 9 hours under hydrogen. The mixture was filtered through Celite, and then the filtrate was concentrated and the precipitated solid was washed with hexane to give the subtitled compound (7.18 g) as a white solid. Yield 100%.
¹H NMR (DMSO-d₆) δ 9.44 (1H, s), 7.09 (2H, d, J = 8.5 Hz), 6.83 (2H, brs), 6.69 (2H, d, J = 8.5 Hz), 4.89 (2H, s), 4.17 (2H, t, J = 6.6 Hz), 4.03 (3H, s), 1.65 (2H, tt, J = 7.5 Hz, 6.6 Hz), 1.40 (2H, tq, J = 7.5 Hz, 7.3 Hz), 0.92 (3H, t, J = 7.3 Hz).

### Step (v): 2-Butoxy-9-[4-(4-chlorobutoxy)benzyl]-8-methoxyadenine

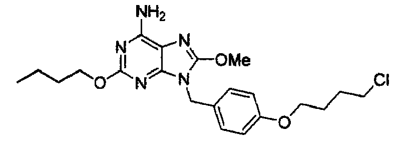

The compound obtained in Step (iv) (0.35 g, 1.02 mmol) was dissolved in DMF (15 ml), and thereto were added 1-bromo-4-chlorobutane (0.59 ml, 5.1 mmol) and potassium carbonate (0.28 g, 2.04 mmol), and the mixture was stirred at 70°C for 4 hours and evaporated. Then, thereto was added water, and the mixture was extracted with chloroform. The organic layer was dried over anhydrous magnesium sulfate, and then concentrated in vacuo. The residue was purified by silica gel column chromatography to give the subtitled compound (0.35 g) as a white solid. Yield 79%.
¹H NMR (CDCl₃) δ 7.30 (2H, d, J = 8.5 Hz), 6.80 (2H, d, J = 8.5 Hz), 5.09 (2H, brs), 5.02 (2H, s), 4.31 (2H, t, J = 6.6 Hz), 4.09 (3H, s), 3.96 (2H, t, J = 5.5 Hz), 3.61 (2H, t, J = 6.0 Hz), 1.96-1.90 (4H, m), 1.82-1.74 (2H, m), 1.52-1.45 (2H, m), 0.97 (3H, t, J = 7.4 Hz).

### Step (vi): 2-Butoxy-9-[4-{4-[4-(ethoxycarbonyl)piperidin-1-yl]butoxy}-benzyl]-8-methoxyadenine

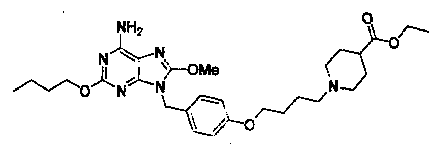

The compound obtained in Step (v) (0.35 g, 0.81 mmol) was dissolved in DMF (10 ml), and thereto were added ethyl isonipecotate (0.60 ml, 3.9 mmol), ethyl diisopropylamine (0.27 ml, 1.56 mmol) and sodium iodide (0.15 g, 1.0 mmol), and the mixture was stirred at 70°C for 30 hours and evaporated. Then, thereto was added water, and the mixture was extracted with chloroform. The organic layer was dried over anhydrous magnesium sulfate, and then concentrated in vacuo. The residue was purified by silica gel column chromatography to give the subtitled compound (0.30 g) as a colorless oil. Yield 71%.
¹H NMR (CDCl₃) δ 7.27 (2H, d, J = 8.6 Hz), 6.79 (2H, d, J = 8.6 Hz), 5.11 (2H, brs), 5.02 (2H, s), 4.31 (2H, t, J = 6.6 Hz), 4.13 (2H, q, J = 7.1 Hz), 4.09 (3H, s), 3.93 (2H, t, J = 5.7 Hz), 2.98-2.85 (2H, m), 2.60-1.60 (15H, m), 1.54-1.45 (2H, m), 1.25 (3H, t, J = 7.1 Hz), 0.97 (3H, t, J = 7.4 Hz).

### Step (vii): 2-Butoxy-7,8-dihydro-9-[4-{4-[4-(methoxycarbonyl)piperidin-1-yl]butoxy}benzyl]-8-oxoadenine

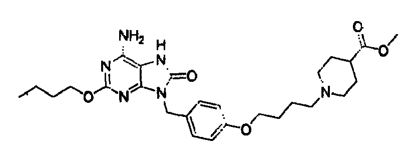

To the compound obtained in Step (vi) (0.30 g, 0.55 mmol) was added 3M hydrochloric acid/methanol solution (20 ml), and the mixture was stirred at room temperature for 12 hours and concentrated. Then, thereto was added saturated sodium bicarbonate water, and the mixture was neutralized. The precipitated solid was filtered and washed with water to give the titled compound (0.20 g) as a white solid. Yield 72%.
¹H NMR (DMSO-d₆) δ 7.21 (2H, d, J = 8.7 Hz), 6.86 (2H, brs), 6.84 (2H, d, J = 8.7 Hz), 4.76 (2H, s), 4.12 (2H, t, J = 6.6 Hz), 3.91 (2H, t, J = 6.4 Hz), 3.59 (3H, s), 2.78-2.73 (2H, m), 2.28-2.22 (3H, m), 1.95-1.83 (2H, m), 1.80-1.70 (2H, m), 1.69-1.42 (8H, m), 1.41-1.33 (2H, m), 0.91 (3H, t, J = 7.3 Hz).

### Example 2: Synthesis of 2-butoxy-7,8-dihydro-9-[4-{4-[4-(carboxy)-piperidin-1-yl]butoxy}benzyl]-8-oxoadenine

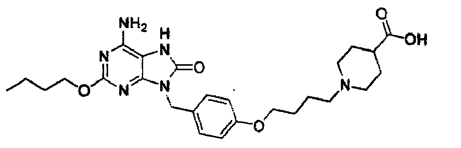

To the compound obtained in Example 1 (155 mg, 0.29 mmol) was added 2M aqueous sodium hydroxide solution (10 ml), and the mixture was heated to stir for 10 minutes under refluxing. The mixture was cooled to 0°C, and then neutralized by concentrated hydrochloric acid. The precipitated solid was filtered and washed with water to give the titled compound (104 mg) as a white solid. Yield 69%.
¹H NMR (DMSO-d₆) δ 10.31 (1H, brs), 7.21 (2H, d, J = 8.6 Hz), 6.85 (2H, d, J = 8.6 Hz), 6.49 (2H, brs), 4.76 (2H, s), 4.14 (2H, t, J = 6.6 Hz), 3.92 (2H, t, J = 6.4 Hz), 2.78-2.73 (2H, m), 2.26 (2H, t, J = 7.2 Hz), 2.16-2.06 (1H, m), 1.92-1.83 (2H, m), 1.80-1.42 (10H, m), 1.41-1.33 (2H, m), 0.91 (3H, t, J = 7.4 Hz).

### Example 3: Synthesis of 2-butoxy-7,8-dihydro-9-(4-[4-{4-[4-(N,N-dimethylamino)butoxycarbonyl]piperidin-1-yl}butoxy]benzyl)-8-oxoadenine

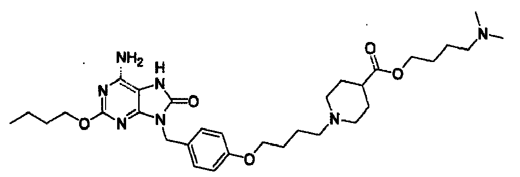

The compound obtained in Example 2 (65 mg, 0.13 mmol) was dissolved in DMF (5 ml), and thereto were added 4-dimethylaminobutanol (0.084 ml, 0.63 mmol), 1-hydroxybenzotriazole (85 mg, 0.63 mmol) and 1-ethyl-3-(3-dimethylaminopropyl)carbodiimide hydrochloride (122 mg, 0.63 mmol), and the mixture was stirred at room temperature for 6 hours and evaporated. Then, thereto was added saturated sodium bicarbonate water, and the precipitated solid was filtered and washed with water to give the titled compound (28 mg) as a white solid. Yield 35%.
¹H NMR (DMSO-d₆) δ9.96 (1H, brs), 7.22 (2H, d, J = 8.7 Hz), 6.85 (2H, d, J = 8.7 Hz), 6.45 (2H, brs), 4.76 (2H, s), 4.14 (2H, t, J = 6.6 Hz), 4.01 (2H, t, J = 6.5 Hz), 3.92 (2H, t, J = 6.4 Hz), 2.78-2.68 (2H, m), 2.28-2.23 (1H, m), 2.26 (2H, t, J = 7.1 Hz), 2.17 (2H, t, J = 7.1 Hz), 2.08 (6H, s), 1.95-1.83 (2H, m), 1.80-1.71 (2H, m), 1.68-1.31 (14H, m), 0.91 (3H, t, J = 7.4 Hz).

### Example 4: Synthesis of 2-butoxy-7,8-dihydro-9-[4-{3-[(N-methyl-N-methoxycarbonylmethyl)amino]propoxy}benzyl]-8-oxoadenine

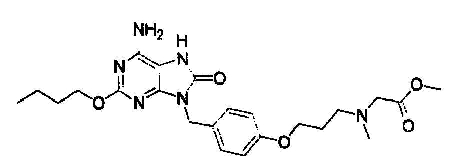

### Step (i): 9-[4-(3-Bromopropoxy)benzyl]-2-butoxy-8-methoxyadenine

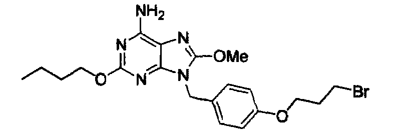

The compound obtained in Example 1 Step (iv) (1.50 g, 4.37 mmol) was dissolved in DMF (50 ml), and thereto were added 1,3-dibromopropane (4.4 ml, 43.7 mmol) and potassium carbonate (0.60 g, 4.37 mmol), and the mixture was stirred at 70°C for 6 hours and evaporated. Then, thereto was added water, and the mixture was extracted with chloroform. The organic layer was dried over anhydrous magnesium sulfate, and then concentrated in vacuo. The residue was purified by silica gel column chromatography to give the subtitled compound (0.48 g) as a white solid. Yield 24%.
¹H NMR (CDCl₃) δ 7.29 (2H, d, J = 8.6 Hz), 6.81 (2H, d, J = 8.6 Hz), 5.26 (2H, brs), 5.03 (2H, s), 4.31 (2H, t, J = 6.6 Hz), 4.09 (3H, s), 4.07 (2H, t, J = 5.8 Hz), 3.58 (2H, t, J = 6.4 Hz), 2.29 (2H, tt, J = 6.4 Hz, 5.8 Hz), 1.78 (2H, tt, J = 7.5 Hz, 6.6 Hz), 1.50 (2H, tq, J = 7.5 Hz, 7.4 Hz), 0.97 (3H, t, J = 7.4 Hz).

### Step (ii): 2-Butoxy-9-[4-{3-[(N-ethoxycarbonylmethyl-N-methyl)amino]-propoxy}benzyl]-8-methoxyadenine

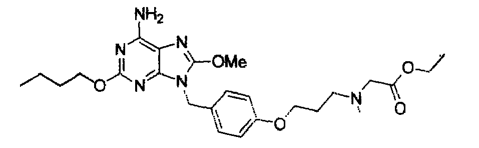

The compound obtained in Step (i) (0.17 g, 0.37 mmol) was treated using N-methylglycine ethyl ester hydrochloride (0.57 g, 3.7 mmol) in the similar manner to Example 1 Step (vi) to give the subtitled compound (71 mg) as a white solid. Yield 39%.
¹H NMR (CDCl₃) δ 7.28 (2H, d, J = 8.7 Hz), 6.80 (2H, d, J = 8.7 Hz), 5.16 (2H, brs), 5.02 (2H, s), 4.31 (2H, t, J = 6.7 Hz), 4.16 (2H, q, J = 7.2 Hz), 4.09 (3H, s), 3.98 (2H, t, J = 6.3 Hz), 3.25 (2H, s), 2.65 (2H, t, J = 7.2 Hz), 2.38 (3H, s), 1.98-1.90 (2H, m), 1.82-1.74 (2H, m), 1.54-1.47 (2H, m), 1.26 (3H, t, J = 7.2 Hz), 0.97 (3H, t, J = 7.4 Hz).

### Step (iii): 2-Butoxy-7,8-dihydro-9-[4-{3-[(N-methyl-N-methoxycarbonylmethyl)amino]propoxy}benzyl]-8-oxoadenine

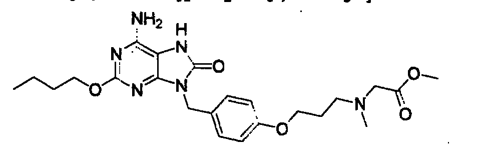

To the compound obtained in Step (ii) (70 mg, 0.14 mmol) were added methanol (2 ml) and 5M aqueous sodium hydroxide solution (2 ml), and the mixture was heated to stir for 30 minutes under refluxing. The mixture was cooled to 0°C, and then neutralized by concentrated hydrochloric acid and evaporated to dryness. Thereto were added methanol (7 ml) and concentrated sulfuric acid (0.3 ml), and the mixture was heated to stir for 6 hours under refluxing. The mixture was cooled to 0°C, and then thereto wad added saturated sodium bicarbonate water, and the mixture was extracted with chloroform/ethanol (3/1). The organic layer was dried over anhydrous magnesium sulfate, and then concentrated in vacuo to give the titled compound (45 mg) as a white solid. Yield 68%.
¹H NMR (DMSO-d₆) δ9.95 (1H, brs), 7.22 (2H, d, J = 8.6 Hz), 6.85 (2H, d, J = 8.6 Hz), 6.45 (2H, brs), 4.76 (2H, s), 4.14 (2H, t, J = 6.6 Hz), 3.94 (2H, t, J = 6.4 Hz), 3.58 (3H, s), 3.26 (2H, s), 2.56 (2H, t, J = 7.0 Hz), 2.26 (3H, s), 1.83-1.76 (2H, m), 1.66-1.57 (2H, m), 1.42-1.32 (2H, m), 0.91 (3H, t, J = 7.4 Hz).

### Example 5: Synthesis of 2-butoxy-7,8-dihydro-9-{4-[1-(methoxycarbonylmethyl)piperidin-4-ylmethyloxy]benzyl}-8-oxoadenine

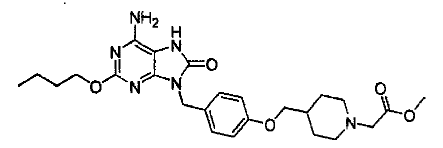

### Step (i): 2-Butoxy-9-{4-[1-(tert-butoxycarbonyl)piperidin-4-ylmethyloxy]benzyl}-8-methoxyadenine

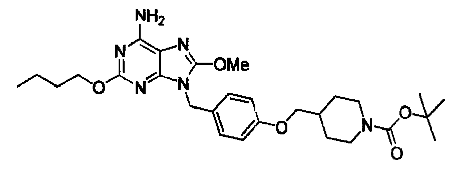

The compound obtained in Example 1 Step (iv) (0.50 g, 1.46 mmol) was dissolved in DMF (20 ml), and thereto were added 4-[2-(methanesulfonyloxy)methyl]-piperidine-1-carboxylic acid tert-butyl ester (0.85 g, 2.91 mmol) and potassium carbonate (0.20 g, 1.46 mmol), and the mixture was stirred at 100°C for 14 hours and evaporated. Then, thereto was added water, and the mixture was extracted with chloroform. The organic layer was dried over anhydrous magnesium sulfate, and then concentrated in vacuo. The residue was purified by silica gel column chromatography to give the subtitled compound (0.53 g) as a pale yellow solid. Yield 68%.
¹H NMR (CDCl₃) δ 7.29 (2H, d, J = 8.7 Hz), 6.80 (2H, d, J = 8.7 Hz), 5.20 (2H, brs), 5.02 (2H, s), 4.31 (2H, t, J = 6.7 Hz), 4.11 (3H, s), 3.75 (2H, d, J = 6.4 Hz), 2.80-2.60 (2H, m), 1.98-1.89 (1H, m), 1.81-1.71 (4H, m), 1.70-1.45 (3H, m), 1.46 (9H, s), 1.30-1.22 (3H, m), 0.97 (3H, t, J = 7.4 Hz).

### Step (ii): 2-Butoxy-8-methoxy-9-{4-[1-(methoxycarbonylmethyl)-piperidin-4-ylmethyloxy]benzyl}adenine)

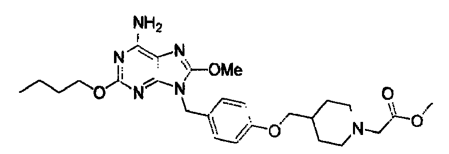

To the compound obtained in Step (i) (0.53 g, 0.99 mmol) was added trifluoroacetic acid (20 ml), and the mixture was stirred for 30 minutes. Trifluoroacetic acid was distilled away, and then to the mixture was added DMF (20 ml), potassium carbonate (0.66 g, 4.8 mmol) and methyl bromoacetate (0.11 ml, 1.2 mmol), and the mixture was stirred at room temperature for 12 hours and evaporated. Then, thereto was added water, and the mixture was extracted with chloroform/ethanol (3/ 1). The organic layer was dried over anhydrous magnesium sulfate, and then concentrated in vacuo. The residue was purified by silica gel column chromatography to give the subtitled compound (0.39 g) as a pale yellow solid. Yield 77%.
¹H NMR (CDCl₃) δ 7.28 (2H, d, J = 8.6 Hz), 6.79 (2H, d, J = 8.6 Hz), 5.27 (2H, brs), 5.02 (2H, s), 4.30 (2H, t, J = 6.6 Hz), 4.08 (3H, s), 3.75 (2H, d, J = 5.9 Hz), 3.72 (3H, s), 3.23 (2H, s), 3.00-2.94 (2H, m), 2.21-2.14 (2H, m), 1.83-1.71 (5H, m), 1.53-1.44 (4H, m), 0.97 (3H, t, J = 7.4 Hz).

### Step (iii): 2-Butoxy-7,8-dihydro-9-{4-[1-(methoxycarbonylmethyl)-piperidin-4-ylmethyloxy]benzyl}-8-oxoadenine

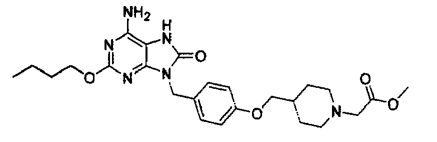

To the compound obtained in Step (ii) (0.39 g, 0.76 mmol) was added 3M hydrochloric acid/methanol solution (15 ml), and the mixture was stirred at room temperature for 12 hours and evaporated. Then, the residue was purified by HPLC and concentrated, and then thereto was added saturated sodium bicarbonate water, and the mixture was extracted with chloroform/ethanol (3/1). The organic layer was dried over anhydrous magnesium sulfate, and then concentrated in vacuo to give the titled compound (0.17 g) as a white solid. Yield 46%.
¹H NMR (DMSO-d₆) δ9.93 (1H, s), 7.22 (2H, d, J = 8.6 Hz), 6.85 (2H, d, J = 8.6 Hz), 6.44 (2H, brs), 4.76 (2H, s), 4.14 (2H, t, J = 6.6 Hz), 3.76 (2H, d, J = 5.8 Hz), 3.60 (3H, s), 3.20 (2H, s), 2.86-2.80 (2H, m), 2.16-2.12 (2H, m), 1.72-1.56 (5H, m), 1.41-1.23 (4H, m), 0.91 (3H, t, J = 7.4 Hz).

### Example 6: 2-Butoxy-7,8-dihydro-9-{4-(1-(carboxymethyl)piperidin-4-ylmethyloxy]benzyl}-8-oxoadenine

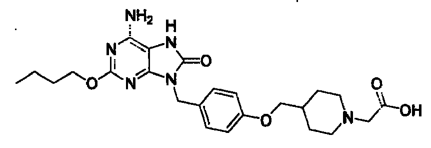

The compound obtained in Example 5 (0.15g, 0.29 mmol) was treated in the similar manner to Example 2 to give the titled compound (91 mg) as a white solid. Yield 64%.
¹H NMR (DMSO-d₆) δ 10.41 (1H, brs), 7.22 (2H, d, J = 8.6 Hz), 6.86 (2H, d, J = 8.6 Hz), 6.60 (2H, brs), 4.76 (2H, s), 4.14 (2H, t, J = 6.6 Hz), 3.78 (2H, d, J = 6.0 Hz), 3.18-3.14 (2H, m), 3.15 (2H, s), 2.56-2.45 (2H, m), 1.80-1.74 (3H, m), 1.66-1.57 (2H, m), 1.44-1.33 (4H, m), 0.91 (3H, t, J = 7.4 Hz).

### Example 7: 2-Butoxy-7,8-dihydro-9-[4-{1-[4-(N,N-dimethylamino)-butoxycarbonylmethyl]piperidin-4-ylmethyloxy}benzyl]-8-oxoadenine

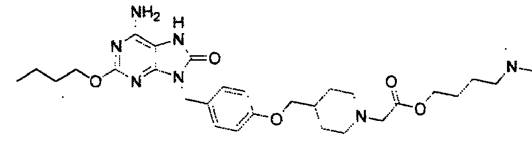

The compound obtained in Example 6 (69 mg, 0.14 mmol) was treated in the similar manner to Example 3 to give the titled compound.
¹H NMR (DMSO-d₆) δ 9.92 (1H, s), 7.22 (2H, d, J = 8.6 Hz), 6.85 (2H, d, J = 8.6 Hz), 6.43 (2H, brs), 4.76 (2H, s), 4.14 (2H, t, J = 6.6 Hz), 4.03 (2H, t, J = 6.6 Hz), 3.76 (2H, d, J = 5.9 Hz), 3.18 (2H, s), 2.86-2.80 (2H, m), 2.19-2.08 (4H, m), 2.08 (6H, s), 1.68-1.54 (6H, m), 1.42-1.37 (4H, m), 1.35-1.20 (3H, m), 0.91 (3H, t, J = 7.4 Hz).

### Example 8: 2-Butoxy-7,8-dihydro-9-[4-(N-ethoxycarbonylmethyl-N-methylaminomethyl)benzyl]-8-oxoadenine

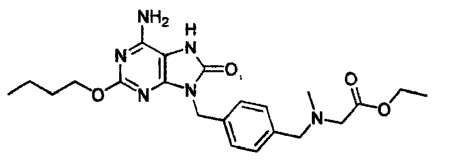

### Step (i): 2-Butoxy-9-[4-(hydroxymethyl)benzyl]-8-methoxyadenine

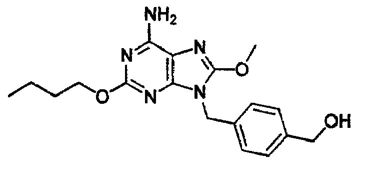

To a solution of 2-butoxy-8-methoxyadenine trifluoroacetic acid salt (10 g, 42.1 mmol) in DMF (90 ml) were added potassium carbonate (17.5 g, 126.4 mmol) and (4-hydroxymethyl)benzyl chloride (7.3 g, 46.4 mmol), and the mixture was stirred at room temperature for 18 hours. The carbonate salt in the mixture was filtered off and the filtrate was concentrated. Thereto was added water, and the mixture was extracted with 5% methanol-chloroform (800 ml). The organic layer was sequentially washed with water and saturated saline, and then dried over sodium sulfate. To the residue were added chloroform (125 ml), methanol (25 ml) and diethyl ether (125 ml), and the insoluble solid was filtered off. The filtrate was concentrated in vacuo, and then thereto was added diethyl ether (150 ml), and the white solid was filtered and dried to give the subtitled compound (7.2 g, 20.1 mmol) as a white solid.
Yield 71%.
¹H NMR (DMSO-d₆) δ 7.26 (2H, d, J = 8.2 Hz), 7.19 (2H, d, J = 8.2 Hz), 6.47 (2H, brs), 5.15 (1H, t, J = 5.6 Hz), 5.01 (2H, s), 4.44 (2H, d, J = 5.6 Hz), 4.17 (2H, t, J = 6.6 Hz), 4.03 (3H, s), 1.68-1.59 (2H, m), 1.44-1.34 (2H, m), 0.91 (3H, t, J = 7.4 Hz).

### Step (ii): 2-Butoxy-7,8-dihydro-9-[4-(chloromethyl)benzyl]-8-oxoadenine

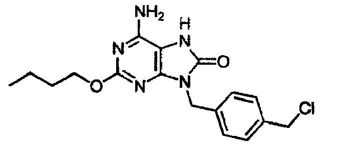

To the compound obtained in Step (i) (7.1 g, 19.6 mmol) was added dichloromethane (140 ml), and to the suspension added thionyl chloride (4.3 ml), and the mixture was stirred at 50°C for 2 hours. Thereto was added toluene (30 ml), and the mixture was evaporated, and then thereto was added additional toluene (100 ml). The mixture was evaporated, and sequentially dried in vacuo to give the subtitled compound (7.2 g, 19.6 mmol) as a pale yellow solid. Yield 99%.
¹H NMR (DMSO-d₆) δ 7.39 (2H, d, J = 8.2 Hz), 7.30 (2H, d, J = 8.2 Hz), 4.88 (2H, s), 4.73 (2H, s), 4.21 (2H, t, J = 6.6 Hz), 1.68-1.59 (2H, m), 1.43-1.32 (2H, m), 0.90 (3H, t, J = 7.4 Hz).

### Step (iii): 2-Butoxy-7,8-dihydro-9-[4-(N-ethoxycarbonylmethyl-N-methylaminomethyl)benzyl]-8-oxoadenine

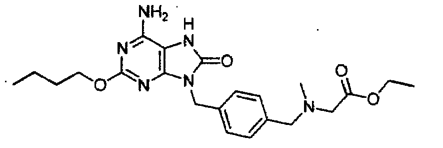

To the compound obtained in Step (ii) (200 mg, 0.55 mmol) was added DMF (5 ml), and to the solution were added diisopropylethylamine (286 µl, 1.66 mmol) and N-ethoxycarbonylmethyl-N-methylamine (130 mg, 1.11 mmol), and the mixture was stirred at 60°C for 3 hours. The filtrate was concentrated, and thereto was added water, and the precipitated solid was filtered and dried in vacuo to give the titled compound as a pale yellow solid.

### Example 9: 2-Butoxy-7,8-dihydro-9-[4-(N-methoxycarbonylmethyl-N-methylaminomethyl)benzyl]-8-oxoadenine

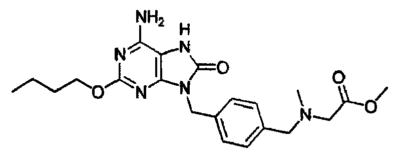

To the compound obtained in Example 8 (200 mg, 0.45 mmol) was added 1N aqueous sodium hydroxide solution (5 ml), and the mixture was heated to stir at 100°C for 6 hours. The mixture was neutralized by concentrated hydrochloric acid, and then evaporated. To the residue were added methanol (10 ml) and concentrated sulfuric acid (200 µl), and the mixture was heated to stir at 80°C for 12 hours. The mixture was neutralized by 28% aqueous ammonia solution, and then thereto was added water. The insoluble solid was filtered, dried and purified by column chromatography, and then dried in vacuo to give the titled compound (80 mg, 34%) as a white solid.
¹H NMR (DMSO-d₆) δ9.95 (1H, brs), 7.25 (4H, s), 6.45 (2H, s), 4.83 (2H, s), 4.13 (2H, t, J = 6.6 Hz), 3.60 (5H, s), 3.27 (2H, s), 2.23 (3H, s), 1.60-1.57 (2H, m), 1.42-4.32 (2H, m), 0.90 (3H, t, J = 7.4 Hz).

### Example 10: 2-Butoxy-7,8-dihydro-9-[4-(N-hydroxycarbonylmethyl-N-methylaminomethyl)benzyl]-8-oxoadenine

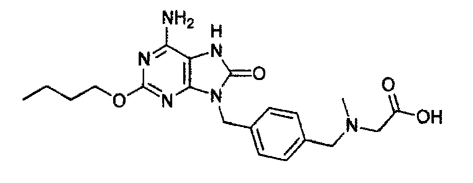

The titled compound was obtained as a white solid in the similar manner to Example 2. Yield 57%.
¹H NMR (DMSO-d₆) δ 10.01 (1H, brs), 7.32-7.26 (4H, m), 6.48 (2H, brs), 4.85 (2H, s), 4.13 (2H, t, J = 6.6 Hz), 3.78 (2H, s), 3.19 (2H, s), 2.34 (3H, s), 1.65-1.58 (2H, m), 1.41-4.32 (2H, m), 0.90 (3H, t, J = 7.4 Hz).

### Example 11: 7,8-Dihydro-9-[4-(N-ethoxycarbonylmethyl-N-methylaminomethyl)benzyl]-2-(2-methoxyethoxy)-8-oxoadenine

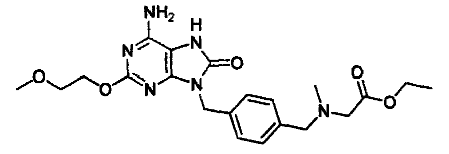

### Step (i): 9-[4-(Hydroxymethyl)benzyl]-2-(2-methoxyethoxy)-8-methoxyadenine

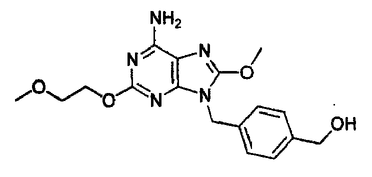

The subtitled compound was obtained as a white solid in the similar manner to Example 8 Step (i). Yield 84%.
¹H NMR (DMSO-d₆) δ 7.26 (2H, d, J = 8.1 Hz), 7.19 (2H, d, J = 8.1 Hz), 6.87 (2H, brs), 5.15 (1H, t, J = 5.7 Hz), 5.01 (2H, s), 4.44 (2H, d, J = 5.7 Hz), 4.29 (2H, dd, J = 4.7, 4.8 Hz), 4.03 (3H, s), 3.60 (2H, d, J = 4.7, 4.8 Hz), 3.16 (3H, s).

### Step (ii): 9-[4-(Chloromethyl)benzyl]-7,8-dihydra-2-(2-methoxyethoxy)-8-oxoadenine

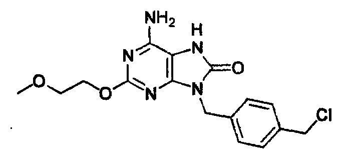

The subtitled compound was obtained as a white solid in the similar manner to Example 8 Step (ii). Yield 83%.
¹H NMR (DMSO-d₆) δ 7.39 (2H, d, J = 8.1 Hz), 7.31 (2H, d, J = 8.1 Hz), 4.88 (2H, s), 4.73 (2H, s), 4.34 (2H, dd, J = 4.7, 4.8 Hz), 3.60 (2H, dd, J = 4.7, 4.8 Hz), 3.27 (3H, s).

### Step (iii): 7,8-Dihydro-9-[4-(N-ethoxycarbonylmethyl-N-methylaminomethyl)benzyl]-2-(2-methoxyethoxy)-8-oxoadenine

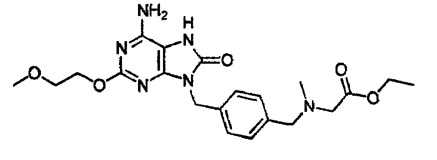

The titled compound was obtained as a white solid in the similar manner to Example 8 Step (iii). Yield 83%.

### Example 12: 7,8-Dihydro-9-[4-(N-methoxycarbonylmethyl-N-methylaminomethyl)benzyl]-2-(2-methoxyethoxy)-8-oxoadenine

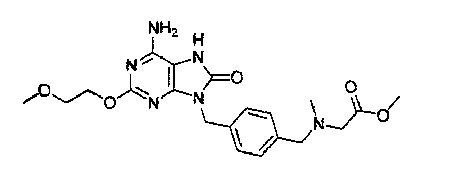

The titled compound was obtained as a white solid in the similar manner to Example 9. Yield 83%.
¹H NMR (DMSO-d₆) δ 10.28 (1H, brs), 7.29-7.24 (4H, m), 6.63 (2H, brs), 4.83 (2H, s), 4.25 (2H, dd, J = 4.7, 4.8 Hz), 3.59 (3H, s), 3.58 (2H, dd, J = 4.7, 4.8 Hz), 3.37 (2H, s), 3.26 (2H, s), 3.26 (3H, s), 2.23 (3H, brs).

### Example 13: 7,8-Dihydro-9-[4-(N-hydroxycarbonylmethyl-N-methylaminomethyl)benzyl]-2-(2-methoxyethoxy)-8-oxoadenine

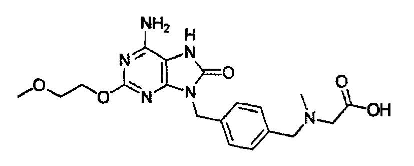

The titled compound was obtained as a white solid in the similar manner to Example 2. Yield 83%.
¹H NMR (DMSO-d₆) δ 10.22 (1H, brs), 7.29-7.24 (4H, m), 6.58 (2H, m), 4.84 (2H, s), 4.25 (2H, dd, J = 4.7, 4.8 Hz), 3.66 (2H, s), 3.58 (2H, dd, J = 4.7, 4.8 Hz), 3.26 (3H, s), 3.13 (2H, s), 2.26 (3H, s).

### Example 14: 7,8-Dihydro-9-[4-(4-methoxycarbonylpiperidin-1-ylmethyl)benzyl]-2-(2-methoxyethoxy)-8-oxoadenine

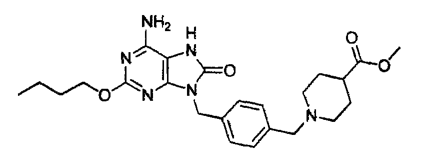

The compound obtained in Example 8 Step (ii) (200 mg, 0.55 mmol) was dissolved in DMF, and thereto was added diisopropylethylamine (285 µl, 1.66 mmol) and then 4-methoxycarbonylpiperidine (222 µl, 1.66 mmol). The mixture was stirred at room temperature for 3 hours, and then evaporated. To the residue was added water, and the mixture was extracted with chloroform-methanol. The organic layer was washed with water and saturated saline, dried over sodium sulfate, and then concentrated. The residue was purified by column chromatography and dried in vacuo to give the titled compound as a white solid. Yield 62%.
¹H NMR (DMSO-d₆) δ9.95 (1H, brs), 7.23 (4H, m), 6.46 (2H, brs), 4.82 (2H, s), 4.13 (2H, t, J = 6.6 Hz), 3.58 (3H, s), 3.38 (2H, s), 2.73-2.66 (2H, m), 2.33-2.24 (1H, m), 1.97-1.70 (2H, m), 1.79-1.72 (2H, m), 1.65-1.57 (2H, m), 1.57-1.46 (2H, m), 1.41-1.31 (2H, m), 0.89 (3H, t, J = 7.4 Hz).

### Example 15: 7,8-Dihydro-9-[4-(4-hydroxycarbonylpiperidin-1-ylmethyl)benzyl]-2-(2-methoxyethoxy)-8-oxoadenine

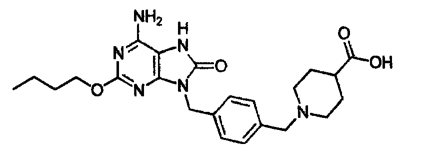

The titled compound was obtained as a white solid in the similar manner to Example 2. Yield 52%.
¹H NMR (DMSO-d₆) δ 12.39 (1H, brs), 10.24 (1H, brs), 7.43 (2H, brs), 7.23-7.31 (2H, m), 6.60 (2H, brs), 4.87 (2H, s), 4.25-4.10 (2H, m), 4.12 (2H, t, J = 6.6 Hz), 3.38 (2H, brs), 3.00-2.71 (2H, m), 2.49-2.30 (1H, m), 2.00-1.85 (2H, m), 1.82-1.62 (2H, m), 1.65-1.55 (2H, m), 1.42-1.32 (2H, m), 0.90 (3H, t, J = 7.4 Hz).

### Example 16: 2-Butoxy-7,8-dihydro-9-[2-methoxy-4-(N-methoxycarbonylmethyl-N-methylaminomethyl)benzyl]-8-oxoadenine

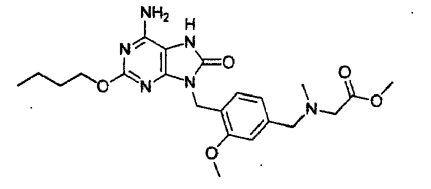

### Step (i): 2-Butoxy-8-methoxy-9-[2-methoxy-4-(methoxycarbonyl)-benzyl] adenine

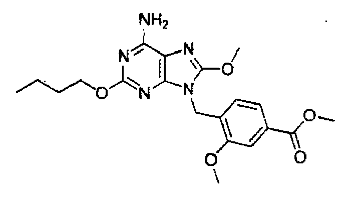

The subtitled compound was obtained as a pale yellow solid in the similar manner to Example 8 Step (i). Yield 55%.
¹H NMR (DMSO-d₆) δ 7.52 (1H, s), 4.49 (1H, d, J = 7.8 Hz), 6.87 (2H, brs), 6.77 (1H, d, J = 7.8 Hz), 5.05 (2H, t, J = 6.6 Hz), 4.09 (2H, t, J = 6.6 Hz), 4.02 (3H, s), 3.92 (3H, s), 3.84 (3H, s), 1.61-1.54 (2H, m), 1.39-1.30 (2H, m), 0.87 (3H, t, J = 7.4 Hz).

### Step (ii): 2-Butoxy-9-(4-hydroxymethyl-2-methoxybenzyl)-8-methoxyadenine

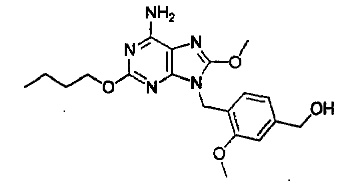

The compound obtained in Step (i) (955 mg, 2.3 mmol) was dissolved in THF (52 ml), and thereto was added portionwise lithium aluminum hydride (131 mg, 3.45 mmol) under ice cooling. The mixture was stirred at room temperature for 2 hours, and then the reaction was quenched by 1N aqueous sodium hydroxide solution, and filtered. The filtrate was concentrated, and then thereto was added water, and the mixture was extracted with chloroform-methanol. The organic layer was washed with water and saturated saline, and then dried over sodium sulfate. The mixture was concentrated, and then dried in vacuo to give the subtitled compound as a yellow oil. Yield 99%.
¹H NMR (DMSO-d₆) δ 6.98 (1H, s), 6.82 (2H, brs), 6.78 (1H, d, J = 7.8 Hz), 6.60 (1H, d, J = 7.8 Hz), 5.17 (1H, t, J = 5.7 Hz), 4.98 (2H, s), 4.45 (2H, d, J = 5.7 Hz), 4.11 (2H, t, J = 6.6 Hz), 4.01 (3H, s), 3.79 (3H, s), 1.62-1.56 (2H, m), 1.39-1.33 (2H, m), 0.89 (3H, t, J = 7.4 Hz).

### Step (iii): 2-Butoxy-7,8-dihydro-9-(4-chloromethyl-2-methoxybenzyl)-8-oxoadenine

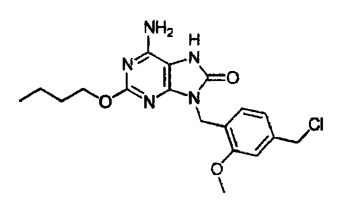

The subtitled compound was obtained as a yellow oil in the similar manner to Example 8 Step (ii). Yield 99%.

### Step (iv): 2-Butoxy-7,8-dihydro-9-[2-methoxy-4-(N-methoxycarbonylmethyl-N-methylaminomethyl)benzyl]-8-oxoadenine

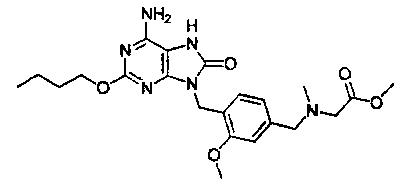

The titled compound was obtained as a yellow oil in the similar manner to Example 8 Step (iii). Yield 63%.
¹H NMR (DMSO-d₆) δ 9.97 (1H, s), 6.96 (1H, s), 6.76 (1H, d, J = 7.8 Hz), 6.66 (1H, d, J = 7.8 Hz), 6.46 (2H, brs), 4.80 (2H, s), 4.08 (2H, t, J = 6.6 Hz), 3.83 (3H, s), 3.60 (2H, s), 3.59 (2H, s), 3.27 (2H, s), 2.25 (3H, s), 1.61-1.54 (2H, m), 1.36-1.30 (2H, m), 0.87 (3H, t, J = 7.4 Hz).

### Example 17: 2-Butoxy-7,8-dihydro-9-[2-methoxy-4-(N-hydroxycarbonylmethyl-N-methylaminomethyl)benzyl]-8-oxoadenine

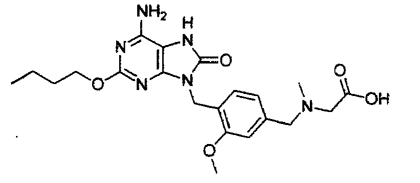

The titled compound was obtained as a white solid in the similar manner to Example 2. Yield 83%.
¹H NMR (DMSO-d₆) δ 10.31 (1H, brs), 7.02 (1H, s), 6.80 (1H, d, J = 7.8 Hz), 6.68 (1H, d, J = 7.8 Hz), 6.65 (2H, brs), 4.80 (2H, s), 4.07 (2H, t, J = 6.6 Hz), 3.84 (3H, s), 3.71 (2H, s), 3.19 (2H, s), 2.31 (3H, s), 1.61-1.54 (2H, m), 1.36-1.30 (2H, m), 0.87 (3H, t, J = 7.4 Hz).

### Example 18: 2-Butoxy-7,8-dihydro-9-{4-[2-(4-methoxycarbonylpiperidin-1-yl)ethyl]benzyl}-8-oxoadenine

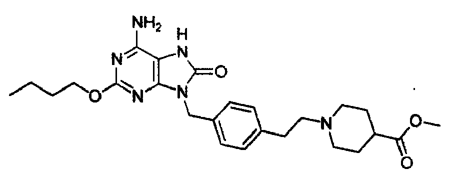

### Step (i): 2-Butoxy-8-methoxy-9-[4-(methoxycarbonylmethyl)benzyl]-adenine

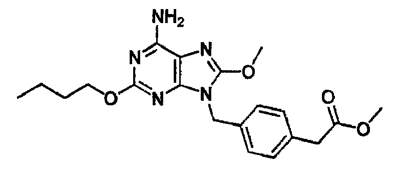

The subtitled compound was obtained as a yellow oil in the similar manner to Example 8 Step (i). Yield 75%.
¹H NMR (DMSO-d₆) δ 7.24-7.17 (4H, m), 6.87 (2H, brs), 5.01 (2H, s), 4.16 (2H, t, J = 6.6 Hz), 4.03 (3H, s), 3.64 (2H, s), 3.58 (3H, s), 1.67-1.49 (2H, m), 1.43-1.34 (2H, m), 0.91 (3H, t, J = 7.4 Hz).

### Step (ii): 2-Butoxy-9-[4-(2-hydroxyethyl)benzyl]-8-methoxyadenine

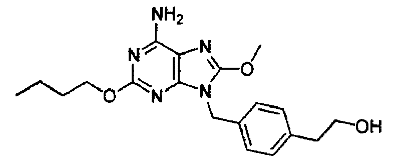

The subtitled compound was obtained as a yellow oil in the similar manner to Example 16 Step (ii). Yield 99%.
¹H NMR (DMSO-d₆) δ 7.18-7.13 (4H, m), 6.85 (2H, brs), 4.99 (2H, s), 4.62 (1H, t, J = 5.2 Hz), 4.16 (2H, t, J = 6.6 Hz), 4.03 (3H, s), 3.54 (2H, dt, J = 5.2, 7.0 Hz), 2.66 (2H, t, J = 7.0 Hz), 1.67-1.50 (2H, m), 1.43-1.35 (2H, m), 0.91 (3H, t, J = 7.1 Hz).

### Step (iii): 2-Butoxy-7,8-dihydro-9-{4-[2-(4-methoxycarbonylpiperidin-1-yl)ethyl]benzyl}-8-oxoadenine

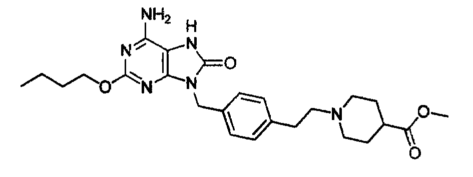

The compound obtained in Step (ii) (1.57 g, 4.23 mmol) was dissolved in THF (20 ml), and thereto were added triethylamine (694 µl, 5.08 mmol) and 4-dimethylaminopyridine (103 mg, 0.85 mmol), and then thereto was added dropwise methanesulfonyl chloride (360 µl, 4.65 mmol) under ice cooling. The mixture was stirred at room temperature for 2 hours, and then evaporated. To the residue was added water, and the mixture was extracted with chloroform-methanol. Then, the organic layer was washed with water and saturated saline, and dried over sodium sulfate. After concentration, the residue was dried in vacuo to give a yellow oil. The residue (200 mg, 0.44 mmol) was dissolved in DMF (5 ml), and thereto were added diisopropylethylamine (400 µl, 2.3 mmol) and 4-ethoxycarbonyl piperidine (260 mg, 2.22 mmol), and the mixture was heated at 60°C for 6 hours and evaporated. Then, thereto was added 1N aqueous sodium hydroxide solution, and the mixture was heated to stir at 90°C for 2 hours. The mixture was neutralized, and then evaporated, and thereto was added methanol (5 ml) and then concentrated sulfuric acid (200 µl). The mixture was heated to stir at 80°C for 3 hours, and then neutralized by ammonia water and evaporated. Then, thereto was added water, and the white solid was filtered and dried to give the titled compound (20 mg, 0.04 mmol) as a white solid. Yield 62%.
¹H NMR (DMSO-d₆) δ9.93 (1H, brs), 7.21-7.14 (4H, mz), 6.44 (2H, brs), 4.80 (2H, s), 4.13 (2H, t, J = 6.6 Hz), 3.59 (3H, s), 2.86-2.80 (2H, m), 2.69-2.63 (2H, m), 2.46-2.40 (2H, m), 2.33-2.25 (1H, m), 2.02-1.93 (2H, m), 1.82-1.74 (2H, m), 1.65-1.58 (2H, m), 1.58-1.45 (2H, m), 1.42-1.32 (2H, m), 0.90 (3H, t, J = 7.4).

### Example 19: 2-Butoxy-7,8-dihydro-9-{4-[2-(4-hydroxycarbonyl-piperidin-1-yl)ethyl]benzyl}-8-oxoadenine

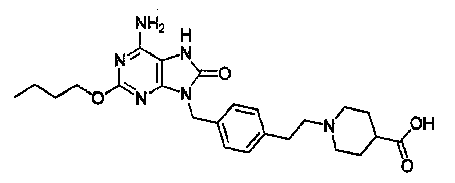

The titled compound was obtained as a white solid in the similar manner to Example 2. Yield 27%.
¹H NMR (DMSO-d₆) δ 11.33 (1H, brs), 9.97 (1H, brs), 7.23-7.16 (4H, m), 6.47 (2H, brs), 4.81 (2H, s), 4.13 (2H, t, J = 6.6 Hz), 2.86-2.80 (2H, m), 2.69-2.63 (2H, m), 2.46-2.40 (2H, m), 2.33-2.25 (1H, m), 2.02-1.93 (2H, m), 1.82-1.74 (2H, m), 1.65-1.58 (2H, m), 1.58-1.45 (2H, m), 1.42-1.32 (2H, m), 0.90 (3H, t, J = 7.4).

### Example 20: 2-Butoxy-7,8-dihydro-9-{4-[2-(N-methoxycarbonylmethyl-N-methylamino)ethyl]benzyl}-8-oxoadenine

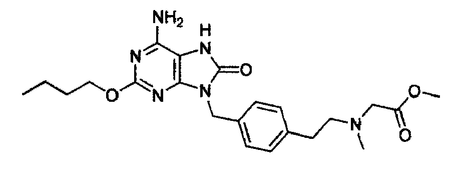

The compound obtained in Example 18 Step (i) was treated in the similar manner to Example 18 Step (ii) to give the titled compound as a white solid. Yield 6%.
¹H NMR (DMSO-d₆) δ 10.46 (1H, brs), 7.21-7.13 (4H, m), 6.61 (2H, brs), 4.80 (2H, s), 4.13 (2H, t, J = 6.6 Hz), 3.58 (3H, s), 3.28 (2H, s), 2.51-2.49 (4H, m), 2.28 (3H, s), 1.65-1.58 (2H, m), 1.42-1.32 (2H, m), 0.90 (3H, t, J = 7.4 Hz).

### Example 21: 2-Butoxy-7,8-dihydro-9-{4-[3-(N-methoxycarbonylmethyl-N-methylamino)propyl]benzyl}-8-oxoadenine

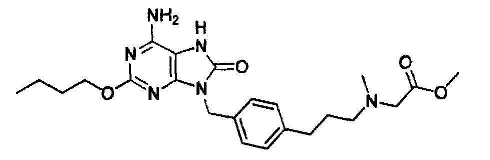

### Step (i): 2-Butoxy-8-methoxy-9-{4-[2-(methoxycarbonyl)ethyl]benzyl}-adenine

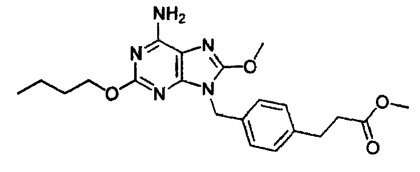

The subtitled compound was obtained as a yellow oil in the similar manner to Example 8 Step (i). Yield 75%.
¹H NMR (DMSO-d₆) δ 7.19-7.13 (4H, m), 6.83 (2H, brs), 4.99 (2H, s), 4.17 (2H, t, J = 6.6 Hz), 4.03 (3H, s), 3.56 (3H, s), 2.80 (2H, t, J = 7.5 Hz), 2.59 (2H, t, J = 7.5 Hz), 1.65-1.60 (2H, m), 1.42-1.36 (2H, m), 0.91 (3H, t, J = 7.4 Hz).

### Step (ii): 2-Butoxy-8-methoxy-9-[4-(3-hyoxypropyl)benzyl]adenine

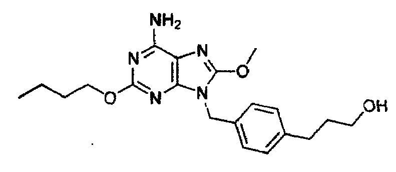

The subtitled compound was obtained as a yellow oil in the similar manner to Example 18 Step (i). Yield 80%.
¹H NMR (DMSO-d₆) δ 7.18 (4H, s), 6.86 (2H, brs), 5.02 (2H, s), 4.47 (1H, t, J = 5.1 Hz), 4.20 (2H, t, J = 6.6 Hz), 4.06 (3H, s), 3.43-3.38 (2H, m), 2.60-2.56 (2H, m), 1.73-1.63 (4H, m), 1.48-1.37 (2H, m), 0.95 (3H, t, J = 7.4 Hz).

### Step (iii): 2-Butoxy-7,8-dihydro-9-{4-[3-(N-methoxycarbonylmethyl-N-methylamino)propyl]benzyl}-8-oxoadenine

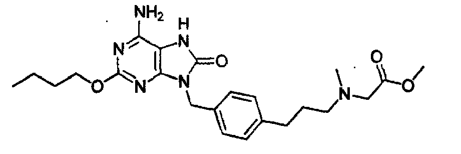

The compound obtained in Step (ii) (660 mg, 1.71 mmol) was dissolved in THF (30 ml), and thereto were added triethylamine (281 µl, 2.05 mmol) and 4-dimethylaminopyridine (42 mg, 0.34 mmol), and then added dropwise methanesulfonyl chloride (146 µl, 1.88 mmol) under ice cooling. The mixture was stirred at room temperature for 1 hour, and then evaporated. To the residue was added water, and the mixture was extracted with chloroform-methanol. Then, the organic layer was washed with water and saturated saline, and then dried over sodium sulfate. After concentration, the residue was dried in vacuo to give a yellow oil. The residue (400 mg, 0.86 mmol) was dissolved in DMF (10 ml), and thereto were added diisopropylethylamine (446 µl, 2.6 mmol) and N-ethoxycarbonylmethyl-N-methylamine (303 mg, 2.59 mmol), and the mixture was heated at 60°C for 7 hours and evaporated. Then, thereto was added 1N aqueous sodium hydroxide solution, and the mixture was heated to stir at 90°C for 2 hours. The mixture was neutralized and then evaporated, and thereto were added methanol (15 ml) and then concentrated sulfuric acid (200 µl). The mixture was heated to stir at 90°C for 6 hours, and then neutralized by ammonia water and evaporated. Then, thereto was added water, and the white solid was filtered, dried and purified by column chromatography to give the titled compound (100 mg, 0.22 mmol) as a white solid. Yield 25%.
¹H NMR (DMSO-d₆) δ9.93 (1H, brs), 7.20 (2H, d, J = 8.1 Hz), 7.13 (2H, d, J = 8.1 Hz), 6.43 (2H, brs), 4.80 (2H, s), 4.14 (2H, t, J = 6.6 Hz), 3.58 (3H, s), 3.23 (2H, s), 2.57-2.47 (2H, m), 2.43-2.39 (2H, m), 2.24 (3H, s), 1.67-1.58 (4H, m), 1.42-1.34 (2H, m), 0.90 (3H, t, J = 7.4 Hz).

### Example 22: 2-Butoxy-7,8-dihydro-9-{4-[3-(N-hydroxycarbonylmethyl-N-methylamino)propyl]benzyl}-8-oxoadenine

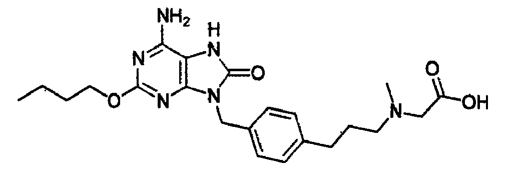

The titled compound was obtained as a white solid in the similar manner to Example 2. Yield 75%.
¹H NMR (DMSO-d₆) δ 10.25 (1H, brs), 7.21 (2H, d, J = 8.1 Hz), 7.15 (2H, d, J = 8.1 Hz), 6.56 (2H, brs), 4.80 (2H, s), 4.13 (2H, t, J = 6.6 Hz), 3.14 (2H, s), 2.70-2.63 (2H, m), 2.57-2.50 (2H, m), 2.37 (3H, s), 1.80-1.70 (2H, m), 1.65-1.58 (2H, m), 1.40-1.34 (2H, m), 0.90 (3H, t, J = 7.4 Hz).

### Example 23: 2-Butoxy-7,8-dihydro-9-{4-[N-(4-dimethylaminobutoxycarbonylmethyl)-N-methylaminomethyl]-2-methoxybenzyl}-8-oxoadenine

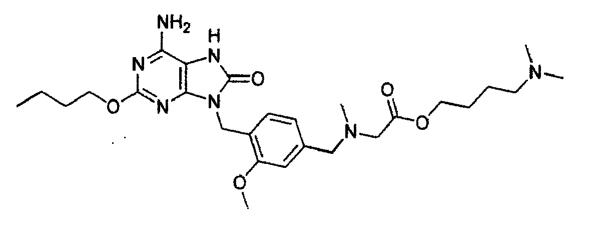

The compound obtained in Example 16 (50 mg, 0.11 mmol) was dissolved in DMF (5 ml), and thereto were added WSC (108 mg, 0.56 mmol), HOBt (76 mg, 0.56 mmol) and dimethylaminobutanol (75 µl, 0.56 mmol), and the mixture was stirred at room temperature for 9 hours and evaporated. Then, thereto was added water, and the mixture was extracted with chloroform-ethanol. The organic layer was washed with water and saturated saline, dried over sodium sulfate, concentrated, and then dried in vacuo to give the titled compound as a white solid. Yield 65%.
¹H NMR (DMSO-d₆) δ 10.14 (1H, brs), 7.95 (1H, brs), 6.96 (1H, s), 6.76 (1H, d, J = 8.1 Hz), 6.67 (2H, d, J = 8.1 Hz), 6.55 (2H, brs), 4.79 (2H, s), 4.09-4.05 (4H, m), 3.83 (3H, s), 3.60 (2H, s), 3.26 (2H, s), 2.81-2.73 (2H, m), 2.53 (6H, s), 2.26 (3H, s), 1.59-1.54 (6H, m), 1.36-1.30 (2H, m), 0.87 (3H, t, J = 7.4 Hz).

### Example 24: Synthesis of 2-butoxy-7,8-dihydro-9-{6-[4-(4-methoxylcarbonylpiperidin-1-yl)butoxy]pyridin-3-ylmethyl}-8-oxoadenine

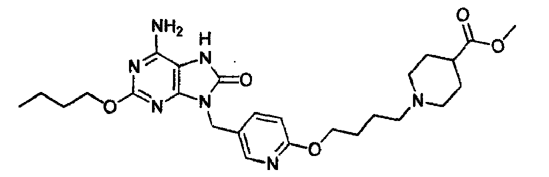

### Step (i): 2-Butoxy-9-(6-chloropyridin-3-ylmethyl)adenine

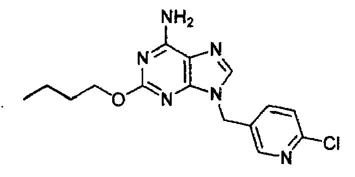

To a solution of 2-butoxyadenine (2.55 g, 12.3 mmol) in DMF (50 ml) were added potassium carbonate (5.11 g, 37.0 mmol) and 2-chloro-5-chloromethylpyridine (2.0 g, 12.3 mmol), and the mixture was stirred at room temperature for 14 hours and evaporated. Then, thereto was added water (80 ml), and the mixture was extracted with 5% methanol-chloroform (100 ml). The organic layer was sequentially washed with water and saturated saline, and then dried over magnesium sulfate, concentrated in vacuo and purified by silica gel column chromatography to give the subtitled compound (2.95 g) as a light brown solid. Yield 72%.
¹H NMR (CDCl₃) δ 8.46 (1H, d, J= 2.3 Hz), 7.69 (1H, s), 7.64 (1H, dd, J= 8.6 Hz, 2.3 Hz), 7.32 (1H, d, J= 8.6 Hz), 6.14 (2H, brs), 5.29 (2H, s), 4.35 (2H, t, J= 6.8 Hz), 1.81-1.74 (2H, m), 1.54-1.45 (2H, m), 0.97 (3H, t, J= 7.4 Hz).

### Step (ii): 2-Butoxy-9-[6-(4-hydroxybutoxy)pyridin-3-yl]methyladenine

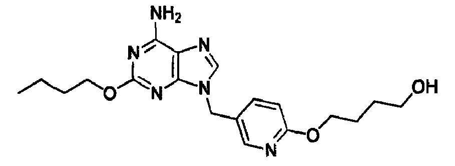

Sodium (350 mg, 15.0 mmol) was dissolved in 1,4-butanediol (10 ml) to prepare alkoxide, and thereto was added the compound obtained in Step (i) (1.0 g, 3.00 mmol), and the mixture was heated to reflux at 130°C for 3 hours. Thereto was added water (50 ml) at 0°C, and added 1N aqueous hydrochloric acid solution to pH9. The mixture was extracted with 30% methanol-chloroform (100 ml). The organic layer was sequentially washed with water and saturated saline, and then dried over magnesium sulfate, concentrated in vacuo and purified by silica gel column chromatography to give the subtitled compound (892 mg) as a white solid. Yield 83%.
¹H NMR (CDCl₃) δ 8.18 (1H, d, J= 2.2 Hz), 7.59 (1H, s), 7.56 (1H, dd, J= 8.6 Hz, 2.2 Hz), 6.69 (1H, d, J= 8.6 Hz), 5.60 (2H, brs), 5.19 (2H, s), 4.34-4.29 (4H, m), 3.72 (2H, q, 6.0 Hz), 1.91-1.70 (6H, m), 1.54-1.49 (2H, m), 0.98 (3H, t, J= 7.4 Hz).

### Step (iii): 2-Butoxy-8-bromo-9-[6-(4-hydroxybutoxy)pyridin-3-yl]-adenine

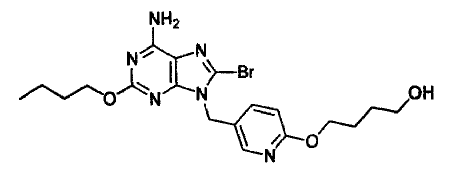

To a solution of the compound obtained in Step (ii) (892 mg, 2.49 mmol) and sodium acetate (613 mg, 7.47 mmol) in chloroform (30 ml) was added bromine (476 mg), and the mixture was stirred for 1 hour. Thereto was added aqueous sodium thiosulfate solution (10 ml), and the mixture was neutralized by aqueous sodium bicarbonate solution, and extracted with chloroform (100 ml). The organic layer was sequentially washed with water and saline, and dried over magnesium sulfate and evaporated. The residue was purified by silica gel column chromatography to give the subtitled compound (919 mg) as a white solid. Yield 84%.
¹H NMR (CDCl₃) δ 8.26 (1H, d, J= 2.3 Hz), 7.66 (1H, dd, J= 8.5 Hz, 2.3 Hz), 6.67 (1H, d, J= 8.5 Hz), 5.66 (2H, brs), 5.22 (2H, s), 4.36-4.28 (4H, m), 3.72 (2H, t, 5.5Hz), 1.87-1.70 (6H, m), 1.54-1.50 (2H, m), 0.98 (3H, t, J= 7.4 Hz).

### Step (iv): 2-Butoxy-9-[6-(4-hydroxybutoxy)pyridin-3-yl]-8-methoxyadenine

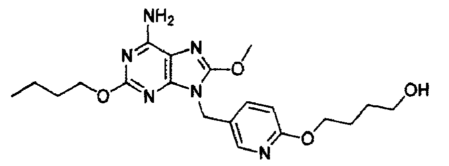

To a solution of the compound obtained in Step (iii) (919 mg, 2.10 mmol) in methanol (20 ml) was added 5N aqueous sodium hydroxide solution (20 ml), and the mixture was heated to reflux at 100°C for 4 hours. The mixture was neutralized by hydrochloric acid, and extracted with 20% methanol-chloroform (100 ml). The organic layer was washed with saline, dried over magnesium sulfate and concentrated in vacuo to give the subtitled compound (815 mg) as a white solid. Yield 99%.
¹H NMR (CDCl₃) δ 8.19 (1H, d, J= 2.4 Hz), 7.64 (1H, dd, J= 8.6 Hz, 2.4 Hz), 6.66 (1H, d, J= 8.6 Hz), 5.21 (2H, brs), 5.01 (2H, s), 4.32-4.29 (4H, m), 4.10 (3H, s), 3.71 (2H, t, 6.3 Hz), 1.87-1.70 (6H, m), 1.54-1.50 (2H, m), 0.98 (3H, t, J= 7.4 Hz).

### Step (v): 2-Butoxy-8-methoxy-9-{6-[4-(4-methoxylcarbonylpiperidin-1-yl)butoxy]pyridin-3-ylmethyl}adenine

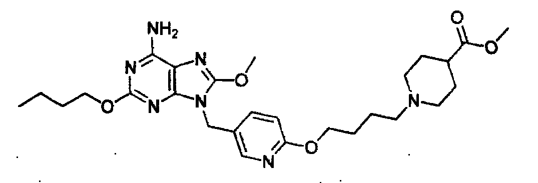

To a solution of the compound obtained in Step (iv) (300 mg, 0.72 mmol) in tetrahydrofuran (20 ml) were added triethylamine (201 µl, 1.44 mmol) and 4-dimethylaminopyridine (18 mg, 0.14 mmol), and the mixture was stirred at room temperature for 10 minutes and then cooled to 0°C. Thereto was added methanesulfonyl chloride (67 µl, 0.86 mmol), and the mixture was stirred at room temperature for 30 minutes. Thereto was added water (20 ml), and the mixture was extracted with chloroform (50 ml). The organic layer was washed with saline, and then dried over magnesium sulfate and concentrated in vacuo. To a solution of the resulting residue in dimethyl formamide (10 ml) were added methyl isonipecotate (515 mg, 3.6 mmol) and diisopropylethylamine (465 mg, 3.6 mmol), and the mixture was heated at 60°C for 24 hours and evaporated in vacuo. Thereto was added water (30 ml), and the mixture was extracted with 30% methanol-chloroform (50 ml). The organic layer was dried over magnesium sulfate, concentrated in vacuo and purified by silica gel column chromatography to give the subtitled compound (294 mg) as a colorless oil. Yield 75%.
¹H NMR (CDCl₃) δ 8.19 (1H, d, J= 2.3 Hz), 7.62 (1H, dd, J= 8.5 Hz, 2.3 Hz), 6.64 (1H, d, J= 8.5 Hz), 5.12 (2H, brs), 5.01 (2H, s), 4.32-4.24 (4H, m), 4.10 (3H, s), 3.67 (3H, s), 2.88 (2H, brs), 2.36 (2H, t, J= 7.6 Hz), 2.28-2.25 (1H, m), 1.98-1.87 (4H, m), 1.80-1.72 (6H, m), 1.65-1.61 (2H, m), 1.54-1.48 (2H, m), 0.97 (3H, t, J= 7.4 Hz).

### Step (vi): 2-Butoxy-7,8-dihydro-9-{6-[4-(4-methoxylcarbonylpiperidin-1-yl)butoxy]pyridin-3-ylmethyl}-8-oxoadenine

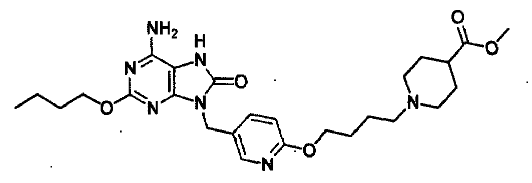

A solution of the compound obtained in Step (v) (293 mg, 0.54 mmol) in 4N hydrochloric acid-methanol (10 ml) was stirred at room temperature for 3 hours, and neutralized by 28% ammonia water and evaporated in vacuo. Thereto was added water (3 ml), and the precipitate was filtered at pH8, and dried to give the titled compound (200 mg) as a white solid. Yield 70%.
¹H NMR (DMSO-d₆) δ 9.97 (1H, s), 8.13 (1H, d, J= 2.2 Hz), 7.64 (1H, dd, J= 8.5 Hz, 2.2 Hz), 6.75 (1H, d, J= 8.5 Hz), 6.46 (2H, brs), 4.80 (2H, s), 4.22 (2H, t, J= 6.5 Hz), 4.16 (2H, t, J= 6.6 Hz), 3.59 (3H, s), 2.76 (2H, brs), 2.28-2.24 (3H, m), 1.90-1.86 (2H, m), 1.78-1.75 (2H, m), 1.68-1.62 (4H, m), 1,52-1.48 (4H, m), 1.41-1.36 (2H, m), 0.92 (3H, t, J= 7.4 Hz).

### Example 25: 2-Butoxy-7,8-dihydro-9-{6-[4-(4-hydroxycarbonylpiperidin-1-yl)) butoxy]pyridin-3-ylmethyl}-8-oxoadenine

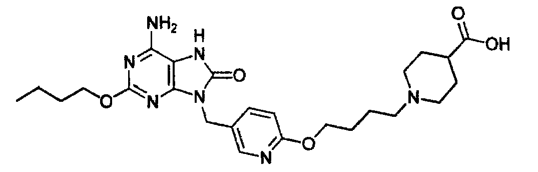

To a solution of the compound obtained in Example 24 (100 mg, 0.17 mmol) in methanol (5 ml) was added 5N aqueous sodium hydroxide solution (2 ml), and the mixture was stirred at room temperature for 3 hours, neutralized by 1N hydrochloric acid and evaporated in vacuo. Thereto was added water (3 ml), and the precipitate was filtered at pH7 and dried to give the titled compound (68 mg) as a white solid. Yield 75%.
¹H NMR (DMSC)-d₆) δ 11.43 (1H, brs), 8.13 (1H, d, J= 2.3 Hz), 7.63 (1H, dd, J= 8.5 Hz, 2.3 Hz), 6.86 (2H, brs), 6.74 (1H, d, J= 8.5 Hz), 4.78 (2H, s), 4.22 (2H, t, J= 6.5 Hz), 4.15 (2H, t, J= 6.6 Hz), 3.59 (3H, s), 2.70 (2H, brs), 2.21 (2H, t, J= 7.4 Hz), 1.91-1.87 (3H, m), 1.67-1.61 (6H, m), 1.48-1.38 (6H, m), 0.92 (3H, t, J= 7.4 Hz).

### Example 26: Synthesis of 2-butoxy-7,8-dihydro-9-(6-{4-[N-methyl-N-methoxylcarbonylmethyl)amino]butoxy}pyridin-3-ylmethyl)-8-oxoadenine

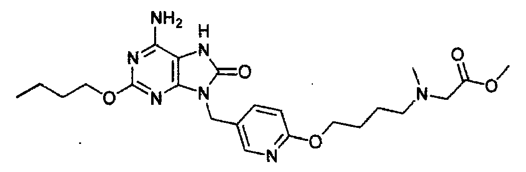

### Step (i): 2-Butoxy-8-methoxy-9-(6-{4-[N-ethoxylcarbonylmethyl-N-methyl)amino]butoxy}pyridin-3-ylmethyl}adenine

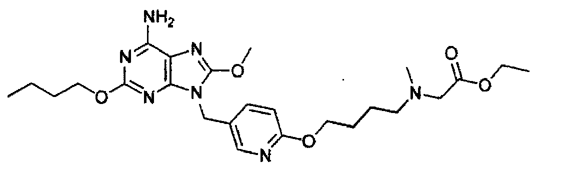

The compound obtained in Example 24 Step (iv) (200 mg, 0.48 mmol) was treated using N-methylglycine ethyl ester (281 mg, 2.4 mmol) in the similar manner to Example 24 Step (v) to give the subtitled compound (168 mg) as a colorless oil. Yield 68%.
¹H NMR (CDCl₃) δ 8.19 (1H, d, J= 2.3 Hz), 7.62 (1H, dd, J= 8.5 Hz, 2.3 Hz), 6.64 (1H, d, J= 8.5 Hz), 5.12 (2H, brs), 5.01 (2H, s), 4.31 (2H, t, J= 6.6 Hz), 4.26 (2H, t, J= 6.5 Hz), 4.17 (2H, q, J= 7.1 Hz), 4.10 (3H, s), 3.23 (2H, s), 2.53 (2H, t, J= 7.5 Hz), 2.36 (3H, s), 1.80-1.75 (4H, m), 1.62-1.59 (2H, m), 1.52-1.49 (2H, m), 1,26 (3H, t, J= 7.1 Hz), 0.98 (3H, t, J= 7.4 Hz).

### Step (ii): 2-Butoxy-7,8-dihydro-9-(6-{4-[N-methyl-N-methoxylcarbonylmethyl)amino]butoxy}pyridin-3-ylmethyl)-8-oxoadenine

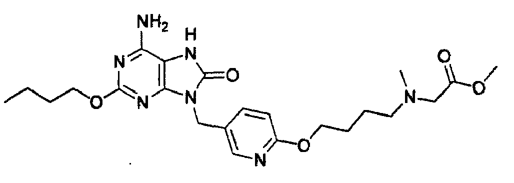

To a solution of the compound obtained in Step (i) (168 mg, 0.33 mmol) in methanol (5 ml) was added 5N aqueous sodium hydroxide solution (2 ml), and the mixture was stirred at room temperature for 3 hours, neutralized by 1N hydrochloric acid and evaporated in vacuo. Thereto was added water (3 ml), and the precipitate was filtered at pH7 and dried. To a solution of the residue in methanol (5 ml) was added concentrated sulfuric acid (0.1 ml), and the mixture was heated to reflux for 48 hours, neutralized by 28% ammonia water and evaporated in vacuo. Thereto was added water (3 ml), and the precipitate was filtered at pH8 and dried to give the titled compound (94 mg) as a white solid. Yield 59%.
¹H NMR (DMSO-d₆) δ 9.94 (1H, brs), 8.13 (1H, d, J= 2.3 Hz), 7.63 (1H, dd, J= 8.6 Hz, 2.3 Hz), 6.75 (1H, d, J= 8.6 Hz), 6.44 (2H, brs), 4.79 (2H, s), 4.20 (2H, t, J= 6.6 Hz), 4.15 (2H, t, J= 6.6 Hz), 3.58 (3H, s), 3.23 (2H, s), 2.44 (2H, t, J= 7.2 Hz), 2.23 (3H, s), 1.68-1.61 (4H, m), 1.50-1.46 (2H, m), 1.41-1.35 (2H, m), 0.98 (3H, t, J= 7.4 Hz).

### Example 27: 2-Butoxy-7,8-dihydro-9-(6-(4-[(N-hydroxylcarbonylmethyl-N-methyl)amino]butoxy)pyridin-3-ylmethyl)-8-oxoadenine

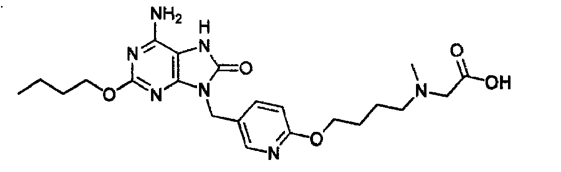

The compound obtained in Example 26 (20 mg, 0.04 mmol) was treated in the similar manner to Example 2 to give the titled compound (14 mg) as a white solid. Yield 70%.
¹H NMR (DMSO-d₆) δ 10.89 (1H, brs), 8.14 (1H, d, J= 2.3 Hz), 7.64 (1H, dd, J= 8.6 Hz, 2.3 Hz), 6.95 (2H, brs), 6.76 (1H, d, J= 8.6 Hz), 4.79 (2H, s), 4.22 (2H, t, J= 6.6 Hz), 4.15 (2H, t, J= 6.6 Hz), 3.16 (2H, s), 2.78 (2H, t, J= 7.2 Hz), 2.45 (3H, s), 1.68-1.59 (6H, m), 1.41-1.36 (2H, m), 0.92 (3H, t, J= 7.4 Hz).

### Example 28: 2-Butoxy-7,8-dihydro-9-[6-(4-{N'-methyl-N'-[4-(N,N-dimethylamino)butoxycarbonylmethyl]}aminobutoxy)pyridin-3-ylmethyl]-8-oxoadenine

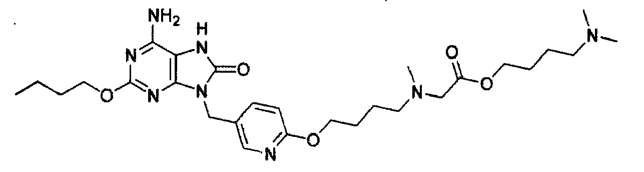

The compound obtained in Example 27 (62 mg, 0.13 mmol) was treated in the similar manner to Example 3 to give the titled compound (25 mg) as a white solid. Yield 33%.
¹H NMR (DMSO-d₆) δ9.96 (1H, brs), 8.14 (1H, d, J= 2.4 Hz), 7.65 (1H, dd, J= 8.6 Hz, 2.4 Hz), 6.75 (1H, d, J= 8.6 Hz), 6.46 (2H, brs), 4.80 (2H, s), 4.21 (2H, t, J= 6.6 Hz), 4.16 (2H, t, J= 6.6 Hz), 4.03 (2H, t, J= 6.6 Hz), 3.23 (2H, s), 3.23 (2H, s), 3.17 (6H, s), 2.24 (3H, t), 2.20 (4H, brs), 1.66-1.38 (12H, m), 0.92 (3H, t, J= 7.4 Hz).

### Example 29: Synthesis of 2-butoxy-7,8-dihydro-9-{6-[3-(4-methoxylcarbonylpiperidin-1-yl)propoxy]pyridin-3-ylmethyl}-8-oxoadenine

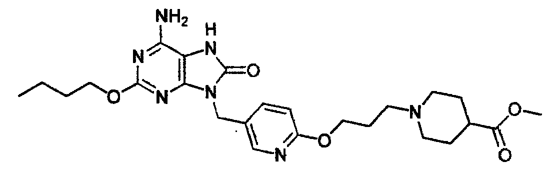

### Step (i): 2-Butoxy-9-[6-(3-hydroxypropoxy)pyridin-3-ylmethyl]adenine

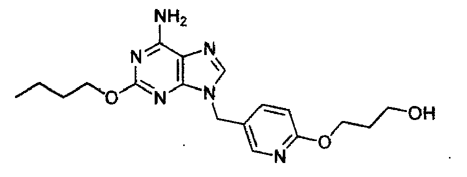

The compound obtained in Example 24 Step (i) (1.5 g, 4.51 mmol) was treated using 1,3-propanediol (10 ml) in the similar manner to Example 24 Step (ii) to give the subtitled compound (1.22 g) as a white solid. Yield 73%.
¹H NMR (CDCl₃) δ 8.17 (1H, d, J= 2.4 Hz), 7.63 (1H, s), 7.58 (1H, dd, J= 8.6 Hz, 2.4 Hz), 6.72 (1H, d, J= 8.6 Hz), 5.95 (2H, brs), 5.20 (2H, s), 4.49 (2H, t, J= 5.9 Hz), 4.33 (2H, t, J= 6.7 Hz), 3.71 (2H, t, 5.9 Hz), 2.92 (1H, brs), 2.01-1.95 (2H, m), 1.82-1.75 (2H, m), 1.54-1.48 (2H, m), 0.98 (3H, t, J= 7.4 Hz).

### Step (ii): 2-Butoxy-8-bromo-9-[6-(3-hydroxypropoxy)pyridin-3-ylmethyl]adenine

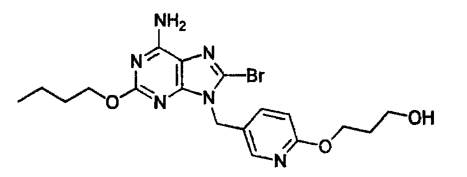

The compound obtained in Step (i) (1.22 g, 2.95 mmol) was treated in the similar manner to Example 24 Step (iii) to give the subtitled compound (1.33 g) as a white solid. Yield 90%.
¹H NMR (CDCl₃) δ 8.24 (1H, d, J= 2.3 Hz), 7.66 (1H, dd, J= 8.6 Hz, 2.3 Hz), 6.71 (1H, d, J= 8.6 Hz), 6.01 (2H, brs), 5.23 (2H, s), 4.49 (2H, t, J= 5.9 Hz), 4.41 (2H, t, J= 6.7 Hz), 3.70 (2H, t, 5.8Hz), 1.99-1.94 (2H, m), 1.83-1.76 (2H, m), 1.54-1.48 (2H, m), 0.98 (3H, t, J= 7.4 Hz).

### Step (iii): 2-Butoxy-9-[6-(3-hydroxypropoxy)pyridin-3-ylmethyl]-8-methoxyadenine

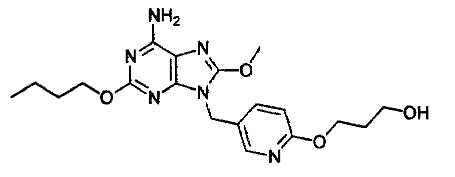

The compound obtained in Step (ii) (1.33 g, 2.95 mmol) was treated in the similar manner to Example 24 Step (iv) to give the subtitled compound (1.18 g) as a white solid. Yield 99%.
¹H NMR (CDCl₃) 88.17 (1H, d, J= 2.2 Hz), 7.67 (1H, dd, J= 8.6 Hz, 2.2 Hz), 6.68 (1H, d, J= 8.6 Hz), 5.14 (2H, brs), 5.01 (2H, s), 4.49 (2H, t, J= 5.8 Hz), 4.31 (2H, t, J= 6.7 Hz), 4.11 (3H, s), 3.68 (2H, q, 5.4 Hz), 1.99-1.93 (2H, m), 1.82-1.75 (2H, m), 1.54-1.48 (2H, m), 0.98 (3H, t, J= 7.4 Hz).

### Step (iv): 2-Butoxy-8-methoxy-9-{6-(3-(4-methoxylcarbonylpiperidin-1-yl)propoxy]pyridin-3-ylmethyl}adenine

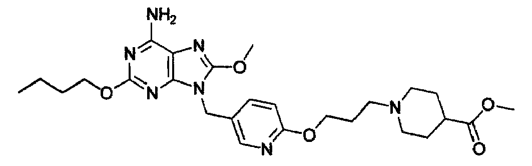

The compound obtained in Step (iii) (400 mg, 0.99 mmol) was treated in the similar manner to Example 24 Step (v) to give the subtitled compound (253 mg) as a colorless oil. Yield 48%.
¹H NMR (CDCl₃) δ 7.43-7.40 (2H, m), 6.50 (1H, d, J= 8.9 Hz), 5.15 (2H, brs), 4.81 (2H, s), 4.29 (2H, t, J= 6.6 Hz), 4.13 (3H, s), 3.95 (2H, t, J= 6.8 Hz), 3.69 (3H, s), 2.81 (2H, brs), 2.31-2.26 (2H, m), 1.95-1.87 (6H, m), 1.80-1.70 (4H, m), 1,53-1.48 (2H, m), 0.97 (3H, t, J= 7.4 Hz).

### Step (v): 2-Butoxy-7,8-dihydro-9-{6-[3-(4-methoxylcarbonylpiperidin-1-yl)propoxy]pyridin-3-ylmethyl}-8-oxoadenine

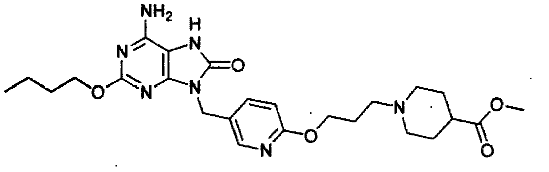

The compound obtained in Step (iv) (253 mg, 0.48 mmol) was treated in the similar manner to Example 24 Step (vi) to give the titled compound (199 mg) as a white solid. Yield 81 %.
¹H NMR (DMSO-d₆) δ9.93 (1H, s), 7.66 (1H, d, J= 2.3 Hz), 7.42 (1H, dd, J= 9.3 Hz, 2.3Hz), 6.45 (2H, brs), 6.34 (1H, d, J= 9.3 Hz), 4.60 (2H, s), 4.16 (2H, t, J= 6.6 Hz), 3.85 (2H, t, J= 6.7 Hz), 3.60 (3H, s), 2.72 (2H, brs), 2.29-2.16 (3H, m), 1.85-1.74 (6H, m), 1.66-1.60 (2H, m), 1.53-1.50 (2H, m), 1.41-1.36 (2H, m), 0.97 (3H, t, J= 7.4 Hz).

### Example 30: 2-Butoxy-7,8-dihydro-9-{6-[3-(4-hydroxycarbonylpiperidin-1-yl)propoxy]pyridin-3-ylmethyl}-8-oxoadenine

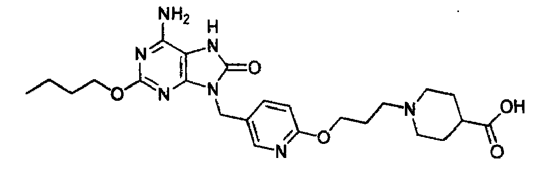

The compound obtained in Example 29 (80 mg, 0.19 mmol) was treated in the similar manner to Example 2 to give the titled compound (13 mg) as a white solid. Yield 18%.
¹H NMR (DMSO-d₆) δ 12.28 (1H, brs), 10.11 (1H, s), 7.70 (1H, s), 7.44 (1H, dd, J= 9.4 Hz, 2.2Hz), 6.55 (2H, brs), 6.37 (1H, d, J= 9.4 Hz), 4.61 (2H, s), 4.16 (2H, t, J= 6.6 Hz), 3.88 (2H, t, J= 6.2 Hz), 2.89 (2H, brs), 2.30 (2H, brs), 1.87 (4H, brs), 1.67-1.61 (4H, m), 1.42-1.36 (2H, m), 0.92 (3H, t, J= 7.4 Hz).

### Example 31: Synthesis of 2-butoxy-7,8-dihydro-9-{6-[3-(methoxycarbonylmethyl)aminopropoxy]pyridin-3-ylmethyl}-8-oxoadenine

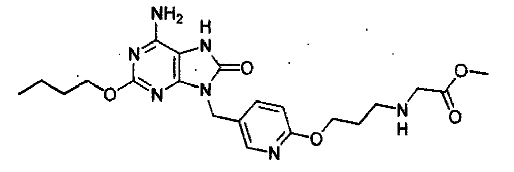

### Step (i): 2-Butoxy-8-methoxy-9-{6-[3-(methoxycarbonylmethyl)-aminopropoxy]pyridin-3-ylmethyl}adenine

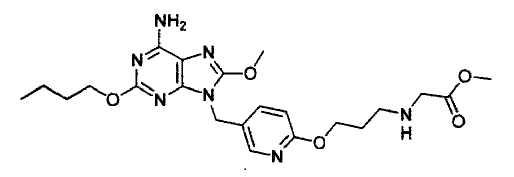

The compound obtained in Example 29 Step (iii) (300 mg, 0.75 mmol) was treated using glycine methyl ester in the similar manner to Example 24 Step (v) to give the subtitled compound (176 mg) as a colorless oil. Yield 50%.
¹H NMR (CDCl₃) δ 7.50-7.47 (2H, m), 6.53 (1H, d, J= 9.4 Hz), 5.15 (2H, brs), 4.84 (2H, s), 4.29 (2H, t, J= 6.6 Hz), 4.13 (3H, s), 4.07 (2H, t, J= 7.1 Hz), 3.77 (3H, s), 2.73 (2H, brs), 2.08 (2H, brs), 1.79-1.76 (2H, m), 1.62-1.56 (2H, m), 1,50-1.46 (2H, m), 0.98 (3H, t, J= 7.4 Hz).

### Step (v): 2-Butoxy-7,8-dihydro-9-{6-[3-(methoxycarbonylmethyl)-aminopropoxy]pyridin-3-ylmethyl}-8-oxoadenine

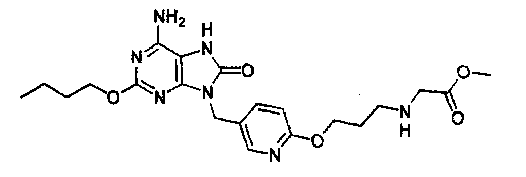

The compound obtained in Step (iv) (176 mg, 0.37 mmol) was treated in the similar manner to Example 24 Step (vi) to give the titled compound (13 mg) as a white solid. Yield 7%.
¹H NMR (DMSO-d₆) δ 10.00 (1H, s), 7.72 (1H, d, J= 2.3 Hz), 7.45 (1H, dd, J=9.4 Hz, 2.3 Hz), 6.50 (2H, brs), 6.39 (1H, d, J= 9.4 Hz), 4.62 (2H, s), 4.16 (2H, t, J= 6.6 Hz), 3.92 (2H, t, J= 7.1 Hz), 3.71 (3H, s), 2.74 (2H, brs), 1.87 (2H, brs), 1.66-1.63 (2H, m), 1.42-1.36 (2H, m), 1,28-1.23 (2H, m), 0.92 (3H, t, J= 7.4 Hz).

### Example 32: Synthesis of 2-butoxy-7,8-dihydro-9-{6-[2-(4-methoxylcarbonylpiperidin-1-yl)ethoxy]pyridin-3-ylmethyl}-8-oxoadenine

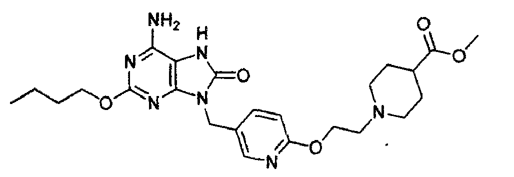

### Step (i): 2-Butoxy-9-[6-(2-hydroxyethoxy)pyridin-3-ylmethyl]adenine

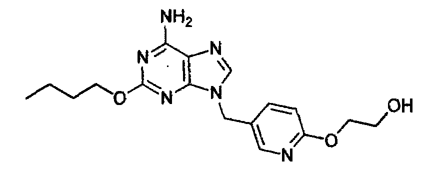

The compound obtained in Example 24 Step (i) (1.0 g, 3.00 mmol) was treated using ethyleneglycol (10 ml) in the similar manner to Example 24 Step (ii) to give the subtitled compound (892 mg) as a white solid. Yield 83%.
¹H NMR (CDCl₃) δ 8.18 (1H, d, J= 2.3 Hz), 7.69 (1H, s), 7.59 (1H, dd, J= 8.6 Hz, 2.3 Hz), 6.79 (1H, d, J= 8.6 Hz), 6.56 (2H, brs), 5.22 (2H, s), 4.46 (2H, t, J= 4.4 Hz), 4.38 (2H, t, J= 6.7 Hz), 3.95 (2H, t, 4.4 Hz), 2.81 (1H, brs), 1.83-1.76 (2H, m), 1.55-1.46 (2H, m), 0.98 (3H, t, J= 7.3 Hz).

### Step (ii): 2-Butoxy-8-bromo-9-[6-(2-hydroxyethoxy)pyridin-3-ylmethyl]adenine

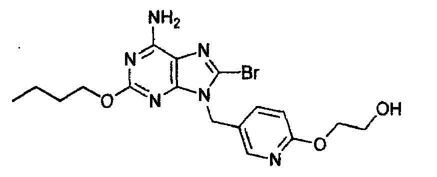

The compound obtained in Step (i) (892 mg, 2.49 mmol) was treated in the similar manner to Example 24 Step (iii) to give the subtitled compound (919 mg) as a white solid. Yield 84%.
¹H NMR (CDCl₃) δ 8.24 (1H, d, J= 2.3 Hz), 7.68 (1H, dd, J= 8.6 Hz, 2.3 Hz), 6.77 (1H, d, J= 8.6 Hz), 5.99 (2H, brs), 5.24 (2H, s), 4.49 (2H, t, J= 4.4 Hz), 4.37 (2H, t, J= 6.6 Hz), 3.94 (2H, t, 4.4 Hz), 1.87-1.77 (2H, m), 1.56-1.47 (2H, m), 0.98 (3H, t, J= 7.4 Hz).

### Step (iii): 2-Butoxy-9-[6-(2-hydroxyethoxy)pyridin-3-ylmethyl]-8-methoxyadenine

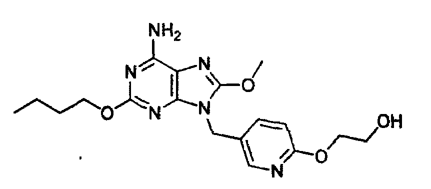

The compound obtained in Step (ii) (919 mg, 2.10 mmol) was treated in the similar manner to Example 24 Step (iv) to give the subtitled compound (815 mg) as a white solid. Yield 99%.
¹H NMR (CDCl₃) δ 8.17 (1H, d, J= 2.3 Hz), 7.66 (1H, dd, J= 8.6 Hz, 2.3 Hz), 6.74 (1H, d, J= 8.6Hz), 5.60 (2H, brs), 5.02 (2H, s), 4.44 (2H, t, J= 4.4 Hz), 4.34 (2H, t, J= 6.6 Hz), 4.12 (3H, s), 3.93 (2H, t, 4.4 Hz), 3.49 (1H, brs), 1.82-1.75 (2H, m), 1.55-1.46 (2H, m), 0.98 (3H, t, J= 7.4 Hz).

### Step (iv): 2-Butoxy-9-[6-(2-chloroethoxy)pyridin-3-ylmethyl]-8-methoxyadenine

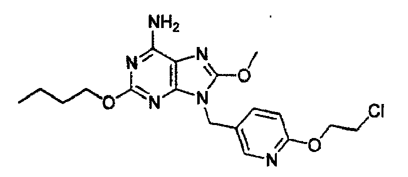

To a solution of the compound obtained in Step (iii) (886 mg, 2.10 mmol) in N,N-dimethylformamide (10 ml) were added triethylamine (350 µl, 2.51 mmol), 4-dimethylaminopyridine (56 mg, 0.46 mmol) and methanesulfonyl chloride (194 µl), and the mixture was stirred at room temperature for 2 hours. The mixture was concentrated in vacuo, extracted with chloroform and dried over anhydrous magnesium sulfate, then concentrated in vacuo and purified by silica gel column chromatography to give the subtitled compound (236 mg) as a white solid. Yield 28%.
¹H NMR (CDCl₃) δ 7.49-7.46 (2H, m), 6.52 (1H, d, J= 9.3 Hz), 5.14 (2H, brs), 4.84 (2H, s), 4.29 (2H, m, J= 6.7 Hz), 4.19 (2H, t, J= 5.4 Hz), 4.13 (3H, s), 3.87 (2H, t, 5.4 Hz), 1.82-1.75 (2H, m), 1.55-1.46 (2H, m), 0.97 (3H, t, J= 7.4 Hz).

### Step (v): 2-Butoxy-8-methoxy-9-{6-[2-(4-methoxylcarbonylpiperidin-1-yl)ethoxy]pyridin-3-ylmethyl}adenine

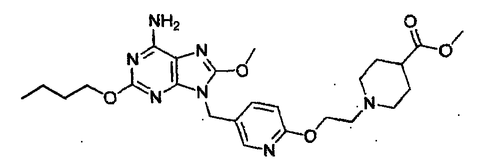

To a solution of the compound obtained in Step (iv) (236 mg, 0.58 mmol) in N,N-dimethylformamide (10 ml) were added potassium carbonate (160 mg, 1.16 mmol) and methyl isonipecotate (249 mg, 1.74 mmol), and the mixture was heated at 50°C for 24 hours and concentrated. Thereto was added water (30 ml), and the mixture was extracted with chloroform, dried over anhydrous magnesium sulfate, and evaporated, and then purified by column chromatography to give the subtitled compound (131 mg) as a colorless oil. Yield 44%.
¹H NMR (CDCl₃) δ 7.44 (1H, dd, J= 9.3 Hz, 2.3 Hz), 7.38 (1H, d, J= 2.3 Hz), 6.49 (1H, d, J= 9.3 Hz), 5.14 (2H, brs), 4.88 (2H, s), 4.29 (2H, t, J= 6.6 Hz), 4.13 (3H, s), 3.97 (2H, t, J= 6.4 Hz), 3.69 (3H, s), 2.82 (2H, brs), 2.62 (2H, t, J= 6.4 Hz), 2.29-2.26 (1H, m), 2.13-2.08 (2H, m), 1.87-1.84 (2H, m), 1.80-1.76 (2H, m), 1.66-1.63 (2H, m), 1.55-1.46 (2H, m), 0.98 (3H, t, J= 7.4 Hz).

### Step (vi): 2-Butoxy-7,8-dihydro-9-{6-[2-(4-methoxylcarbonylpiperidin-1-yl)ethoxy]pyridin-3-ylmethyl}-8-oxoadenine hydrochloride

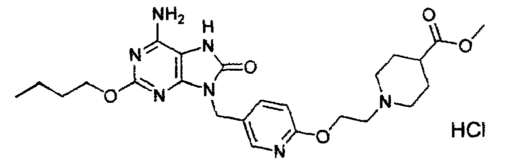

A solution of the compound obtained in Step (v) (131 mg, 0.26 mmol) in 4N hydrochloric acid-methanol was stirred at room temperature for 5 hours, and then evaporated to give the titled compound (128 mg) as a white solid. Yield 93%.
¹H NMR (DMSO-d₆) δ 10.54 (1H, s), 7.78 (1H, d, J= 2.3 Hz), 7.53 (1H, dd, J= 9.4 Hz, 2.3 Hz), 7.10 (2H, brs), 6.46 (1H, d, J= 9.4 Hz), 4.59 (2H, s), 4.29-4.21 (4H, m), 3.61-3.57 (5H, m), 3.35-3.33 (2H, m), 3.01-2.98 (2H, m), 2.09-2.06 (2H, m), 1.97-1.94 (1H, m), 1.81-1.78 (2H, m), 1.68-1.64 (2H, m), 1.42-1.37 (2H, m), 0.98 (3H, t, J= 7.4 Hz).

### Example 33: 2-Butoxy-7,8-dihydro-9-{6-[2-(4-hydroxycarbonylpiperidin-1-yl)ethoxy]pyridin-3-ylmethyl}-8-oxoadenine

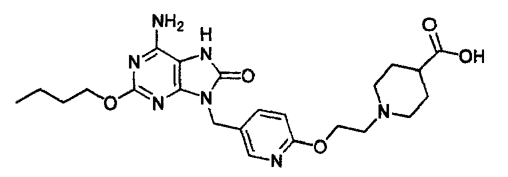

The compound obtained in Example 32 (95 mg, 0.18 mmol) was treated in the similar manner to Example 25 to give the titled compound (49 mg) as a white solid. Yield 57%.
¹H NMR (DMSO-d₆) δ 12.89 (1H, brs), 7.47-7.44 (2H,m), 6.58 (2H, brs), 6.29 (1H, d, J= 9.4 Hz), 4.62 (2H, s), 4.13 (2H, t, J= 6.6 Hz), 3.85 (2H, brs), 3.69 (3H, s), 2.64 (2H, brs), 2.31 (2H, brs), 1.79-1.76 (3H, m), 1.65-1.62 (2H, m), 1.50-1.37 (6H, m), 0.92 (3H, t, J= 7.4 Hz).

### Example 34: Human TLR7 reporter assay

Human TLR7 or rat TLR7 plasmid and reporter plasmid (NF-kB-SEAP) stably-induced HEK293 cells were suspended in DMEM media (10% FBS, 1% NEAA, 10 ug/mL blastocidin S HCl, 100 ug/mL Zeocin), and the suspension was seeded in 90 µl/well on 96 well plate (hTLR7/seap-293: 20000 cells/well, rTLR7/seap-293: 25000 cells/well).
To the cells seeded on 96 well plate was added in 10 µl/well a test compound wherein DMSO stock solution (2 µl) was hundredfold diluted with medium (200 µl) (final concentration; 1 nM-10 µM, common ratio 3). The mixture was stirred tapping in the side of the plate, and then incubated for 20 hours in CO2 incubator. To the cells stimulated by a test compound was added a substrate for reporter assay (substrate for SEAP, pNPP) by 50 µl/well. 10 minutes after the addition of substrate, a quenching solution (4N NaOH) was added by 50 µl/well, and the enzymatic reaction was quenched. Top seal A was attached on the plate, and absorbance was measured by microplate reader (405 nm).
Each human TLR7 binding activity (EC50) of each compound is shown in Table 1.

**Table 1**

| Compound | EC50 (nM) |
|---|---|
| Example 1 | 5.7 |
| Example 2 | 30.0 |
| Example 3 | 7.8 |
| Example 4 | 7.8 |
| Example 5 | 3.6 |
| Example 7 | 5.1 |
| Example 9 | 8.8 |
| Example 12 | 252.1 |
| Example 14 | 1.7 |
| Example 16 | 15.6 |
| Example 18 | 2.2 |
| Example 20 | 18.5 |
| Example 21 | 6.8 |
| Example 23 | 15.7 |
| Example 24 | <1.0 |
| Example 26 | 2.8 |
| Example 28 | 2.0 |

### Example 35: Methyl N-(3-{4-[(6-amino-2-butoxy-8-oxo-7,8-dihydro-9H-purin-9-yl)methyl]phenoxy}propyl)-N-[2-(dimethylamino)ethyl]glycinate

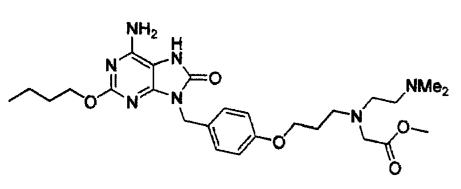

### Step (i): tert-Butyl N-(3-{4-[(6-amino-2-butoxy-8-oxo-7,8-dihydro-9H-purin-9-yl)methyl]phenoxy}propyl)glycinate

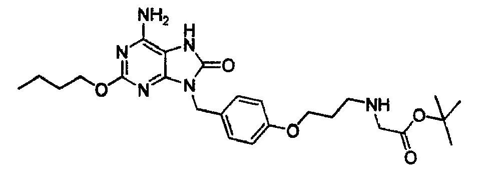

2-Butoxy-9-[4-(4-chloropropyloxy)benzyl]-8-methoxyadenine (0.35 g, 1.6 mmol) was dissolved in DMSO (3 ml), and thereto were added glycine-tert-butyl ester hydrochloride (4.05 g, 24 mmol) and triethylamine (10.2 ml, 75 mmol), and the mixture was stirred at 100°C for 20 hours. The mixture was cooled to room temperature, and then thereto was added saturated sodium bicarbonate water, and the mixture was neutralized and extracted with ethyl acetate. The organic layer was dried over anhydrous sodium sulfate, and then concentrated in vacuo. The residue was purified by silica gel column chromatography to give the subtitled compound (0.39 g) as a white solid.
Yield 49%.
¹H NMR (DMSO-d₆) δ 10.06 (1H, s), 7.21 (2H, d, J = 8.8), 6.84 (2H, d, J = 8.8 Hz), δ 6.47 (2H, s), δ 4.75 (2H, s), 4.13 (2H, t, J = 6.4 Hz), 3.95 (2H, t, J = 6.4 Hz), δ 3.16 (2H, s), 2.59 (2H, t, J = 6.8 Hz), 1.81-1.72 (2H, m), 1.65-1.57 (2H, m), 1.41-1.32 (11H, m), 0.89 (3H, t, J = 6.8 Hz).

### Step (ii): tert-Butyl N-(3-{4-[(6-amino-2-butoxy-8-oxo-7,8-dihydro-9H-purin-9-yl)methyl]phenoxy}propyl)-N-{2-[(tert-butoxycarbonyl)-(methyl)-amino]ethyl}glycinate

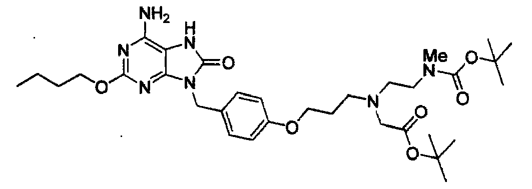

The compound obtained in Step (i) (0.39 g, 0.78 mmol) was dissolved in chloroform (5 ml) and NMP (5 ml), and thereto were added tert-butylmethyl-(2-oxoethyl)-carbamate (0.21 g, 1.17 mmol), acetic acid (0.045 ml, 1.17 mmol) and sodium triacetoxyborohydride (0.248 g, 1.17 mmol), and the mixture was stirred at room temperature for 7 hours. Thereto was added saturated sodium bicarbonate water, and the mixture was neutralized and extracted with ethyl acetate. The organic layer was dried over anhydrous sodium sulfate, and then concentrated in vacuo. The residue was purified by silica gel column chromatography to give the subtitled compound (0.44 g) as a white solid.
Yield 86%.
¹H NMR (DMSO-D₆) δ 9.99 (1H, s), 7.22 (2H, d, J = 8.8), 6.83 (2H, d, J = 8.8 Hz), δ 6.44 (2H, s), δ 4.75 (2H, s), 4.13 (2H, t, J = 6.4 Hz), 3.93 (2H, t, J = 6.4 Hz), δ 3.24 (2H, s), 3.18-3.10 (2H, m), 2.76-2.60 (7H, m), 1.78-1.72 (2H, m), 1.65-1.58 (2H, m), 1.41-1.30 (20H, m), 0.88 (3H, t, J = 6.8 Hz).

### Step (iii): Methyl N-(3-(4-[(6-amino-2-butoxy-8-oxo-7,8-dihydro-9H-purin-9-yl)methyl]phenoxy}propyl)-N-[2-(dimethylamino)ethyl]glycinate

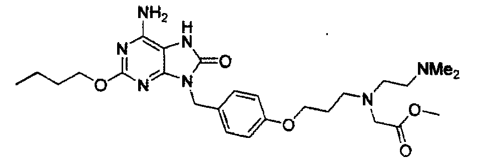

The compound obtained in Step (ii) (0.24 g, 0.49 mmol) was dissolved in THF (5 ml), and thereto was added a solution of 4N hydrochloric acid in dioxane (5 ml), and the mixture was stirred at room temperature for 8 hours. The filtrate was concentrated to give a crude product, and the crude product was dissolved in methanol (5 ml). Thereto were added 30% aqueous formalin solution (0.4 ml) and sodium cyanoborohydride (0.30 g, 4.8 mmol), and the mixture was stirred at room temperature for 3 hours. To the reaction solution was added 2N aqueous sodium hydroxide solution to around pH7, and then the mixture was concentrated in vacuo. To the residue was added water (3 ml), and the mixture was stirred for 30 minutes, and then the precipitate was filtered. The resulting precipitate was dried, and then dissolved in methanol (5 ml). Thereto was added concentrated sulfuric acid (0.5 ml), and the mixture was refluxed for 18 hours. After cooling to room temperature, the reaction solution was added to saturated sodium bicarbonate water, neutralized and extracted with a mixture of chloroform:methanol = 10:1. The organic layer was dried over anhydrous sodium sulfate, and then concentrated in vacuo. The residue was purified by silica gel column chromatography to give the titled compound (0.13 g) as a white solid. Yield 49%.
¹H NMR (DMSO-d₆) δ 9.97 (1H, s), 7.22 (2H, d, J = 8.8), 6.83 (2H, d, J = 8.8 Hz), δ 6.44 (2H, s), δ 4.75 (2H, s), 4.13 (2H, t, J = 6.8 Hz), 3.93 (2H, t, J = 6.4 Hz), δ 3.56 (3H, s), δ 3.37 (2H, s), 2.66 (2H, t, J = 6.8 Hz), 2.62 (2H, t, J = 6.8 Hz), 2.22 (2H, t, J = 6.8 Hz), 2.03 (6H, s), 1.78-1.72 (2H, m), 1.65-1.58 (2H, m), 1.41-1.37 (2H, m), 0.88 (3H, t, J = 6.8 Hz).

### Example 36: 7,8-Dihydro-9-(4-{[N-methyl-N-(N'-methoxycarbonylmethyl-piperidin-4-yl)]amino}methylbenzyl)-2-(2-methoxyethoxy)-8-oxoadenine

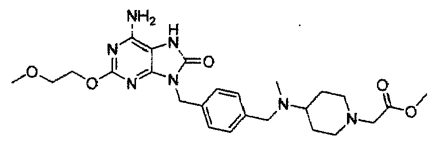

### Step (i): 9-[4-(N-methylamino)methylbenzyl]-2-(2-methoxyethoxy)-8-oxoadenine

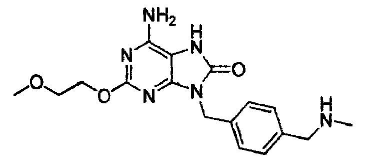

2-(2-Methoxyethoxy)-9-[4-(chloromethyl)benzyl]-8-oxoadenine obtained in Example 11 Step (ii) (0.25 g, 0.69 mmol) was dissolved in DMF (5 ml), and thereto was added 40% aqueous methylamine solution (2 ml), and the mixture was stirred at room temperature for 30 minutes and evaporated. Then, thereto was added 1% ammonia water, and the precipitated solid was filtered to give the subtitled compound (0.18 g) as a white solid. Yield 75%.
¹H NMR (DMSO-d₆) δ 7.24-7.22 (4H, m), 6.47 (2H, brs), 4.82 (2H, s), 4.25 (2H, t, J = 4.7 Hz), 3.60-3.56 (2H, m), 3.57 (2H, s), 3.25 (3H, s), 2.22 (3H, s).

### Step (ii): 7,8-Dihydro-9-(4-{[N-methyl-N-(N'-Boc-piperidin-4-yl)]amino}-methylbenzyl)-2-(2-methoxyethoxy)-8-oxoadenine

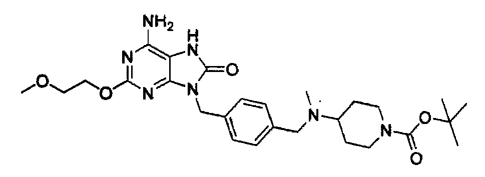

9-[4-(N-methylamino)methylbenzyl]-2-(2-methoxyethoxy)-8-oxoadenine obtained in Step (i) (0.18 g, 0.51 mmol) was dissolved in N-methylpiperidone (2 ml), and thereto was added N-(tert-butoxycarbonyl)-4-piperidone (0.20 g, 1.01 mmol). The mixture was stirred at room temperature for 30 minutes, and then thereto was added sodium triacetoxyborohydride (0.16 g, 0.76 mmol), and the mixture was stirred for 1.5 hours. The mixture was cooled to 0°C, and thereto was added dropwise 1% ammonia water (6 ml), and the precipitated solid was filtered, dried, and then purified by silica gel column (chloroform/methanol = 50/1 to 25/1) to give the subtitled compound as a white solid. 0.19g, Yield 70%.
¹H NMR (DMSO-d₆) δ9.96 (1H, s), 7.24-7.22 (4H, m), 6.47 (2H, brs), 4.82 (2H, s), 4.25 (2H, t, J = 4.7 Hz), 4.24-3.94 (2H, m), 3.58 (2H, t, J = 4.7 Hz), 3.49 (2H, s), 3.26 (3H, s), 2.70-2.60 (2H, m), 2.59-2.48 (1H, m),2.05 (3H, s), 1.72-1.67 (2H, m), 1.38 (9H, s), 1.38-1.26 (2H, m).

### Step (iii): 9-(4-{[N-methyl-N-(N'-methoxycarbonylmethylpiperidin-4-yl)]-amino}methylbenzyl)-2-(2-methoxyethoxy)-8-oxoadenine

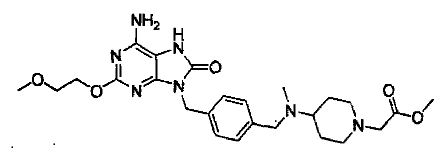

9-(4-{[N-methyl-N-(N'-Boc-piperidin-4-yl)]amino}methylbenzyl)-2-(2-methoxyethoxy)-8-oxoadenine obtained in Step (i) (0.19 g, 0.34 mmol) was suspended in methanol (2 ml), and thereto was added 4M hydrochloric acid/dioxane (2 ml), and the mixture was stirred at room temperature for 30 minutes and evaporated. Then, thereto were sequentially added DMF (2 ml), diisopropylethylamine (0.29 ml, 1.7 mmol) and methyl bromoacetate (0.036 ml, 0.38 mmol), and the mixture was stirred for 1.5 hours. The mixture was cooled to 0°C, and then thereto was added dropwise 1% ammonia water (6 ml), and the precipitated solid was filtered, dried, and then thereto was added acetonitrile (2.5 ml), and the mixture was heated to stir for 30 minutes. The mxiture was cooled to room temperature, and then the solid was filtered to give the titled compound (0.11 g). A white solid. Yield 64%.
¹H NMR (DMSO-d₆) δ 10.08 (1H, s), 7.30-7.22 (4H, m), 6.49 (2H, brs), 4.83 (2H, s), 4.26 (2H, t, J = 4.7 Hz), 3.60 (3H, s), 3.58 (2H, t, J = 4.7 Hz), 3.49 (2H, s), 3.27 (3H, s), 3.18 (2H, s), 2.87-2.83 (2H, m), 2.38-2.29 (1H, m), 2.14-2.08 (2H, m), 2.06 (3H, s), 1.69-1.66 (2H, m), 1.51-1.48 (2H, m).

### Example 37: 7,8-Dihydro-9-{4-[4-(methoxycarbonylmethoxymethyl)-piperidin- 1-ylmethyl]benzyl}-2-(2-methoxyethoxy)-8-oxoadenine

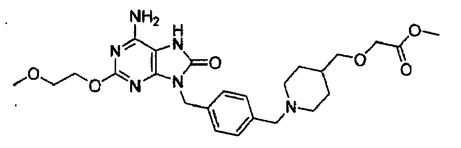

### Step (i): [(1-tert-Butoxycarbonylpiperidin-4-yl)methoxy]acetic acid tert-butyl ester

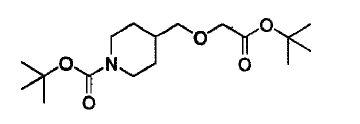

1-Boc-4-piperidinemethanol (1.01 g, 4.69 mmol) was dissolved in toluene (9.5 ml), and thereto were added 50% aqueous sodium hydroxide solution (4.5 ml) and tetrabutylammonium bromide (0.376 g, 1.17 mmol), and the mixture was stirred for 30 minutes. Thereto was added tert-butyl bromoacetate (1.04 ml, 7.04 mmol), and the mixture was stirred for 24 hours. The organic layer was washed with saturated saline twice, and then dried over anhydrous magnesium sulfate. The mixture was concentrated in vacuo, and the residue was purified by silica gel column chromatography (eluent; hexane:ethyl acetate = 8:1 to 6:1) to give the subtitled compound (1.30 g) as a colorless oil. Yield 67%.
¹H NMR (CDCl₃) δ 4.13-4.10 (2H, m), 3.94 (2H, s), 3.35 (2H, d, J = 6.3 Hz), 2.72-2.67 (2H, m), 1.80-1.72 (3H, m), 1.47 (9H, s), 1.45 (9H, s), 1.21-1.12 (2H, m).

### Step (ii): [(Piperidin-4-yl)methoxy]acetic acid methyl ester

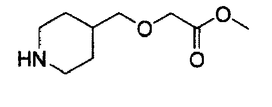

The compound obtained in Step (i) (1.30 g, 3.95 mmol) was dissolved in chloroform (6.5 ml), and thereto was added 4N dioxane hydrochloric acid (6.5 ml), and the mixture was stirred for 20 hours. The mixture was evaporated and then dissolved in methanol (10 ml), and thereto was added concentrated hydrochloric acid (0.1 ml), and the mixture was heated to stir for 3 hours under refluxing. The reaction solution was poured into saturated sodium bicarbonate water, and extracted with chloroform. The organic layer was dried over sodium sulfate, and then concentrated in vacuo to give the subtitled compound (427 mg) as a yellow oil. Yield 58%.
¹H NMR (CDCl₃) 54.07 (2H, s), 3.75 (3H, s), 3.36 (2H, d, J = 6.2 Hz), 3.14-3.11 (2H, m), 2.66-2.60 (2H, m), 1.81-1.76 (3H, m), 1.26-1.19 (2H, m).

### Step (iii): 7,8-Dihydro-9-{4-[4-(methoxycarbonylmethoxymethyl)-piperidin-1-ylmethyl]benzyl}-2-(2-methoxyethoxy)-8-oxoadenine

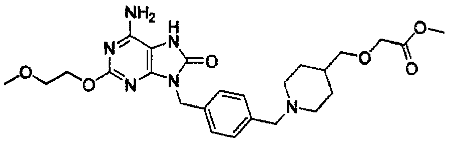

The compound obtained in Step (ii) was treated using compound obtained in Example 11 Step (ii) in the similar manner to Example 8 Step (iii) to give the titled compound. Yield 12%.
¹H NMR (DMSO-d₆) δ9.96 (1H, brs), 7.22 (4H, s), 6.47 (2H, brs), 4.81 (2H, s), 4.24 (2H, t, J = 4.7 Hz), 4.06 (2H, s), 3.62 (3H, s), 3.57 (2H, t, J = 4.7 Hz), 3.36 (2H, s), 3.27 (2H, d, J = 6.4 Hz), 3.25 (3H, s), 2.74-2.72 (2H, m), 1.87-1.82 (2H, m), 1.59-1.56 (2H, m), 1.50-1.49 (1H, m), 1.17-1.11 (2H, m).

### Example 38: 7,8-Dihydro-9-[4-(3-methoxycarbonylmethoxypropyl)-(methyl)aminomethylbenzyl]-2-(2-methoxyethoxy)-8-oxoadenine

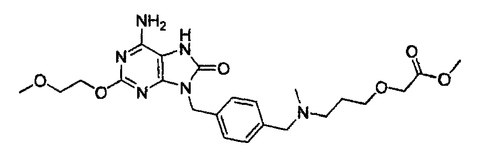

### Step (i): (3-Benzyloxypropoxy)acetic acid tert-butyl ester

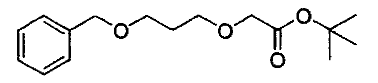

The subtitled compound was obtained as a colorless oil in the similar manner to Example 37 Step (i). Yield 57%.
¹H NMR (CDCl₃) δ 7.34-7.27 (5H, m), 4.51 (2H, s), 3.94 (2H, s), 3.63 (2H, t, J = 5.9 Hz), 3.60 (2H, t, J = 5.9 Hz), 1.93 (2H, qui, J = 5.9 Hz), 1.48 (9H, s).

### Step (ii): (3-Hydroxypropoxy)acetic acid tert-butyl ester

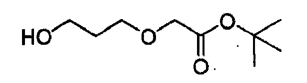

The compound obtained in Step (i) (4.89 g, 17.4 mmol) was dissolved in methanol (25 ml), and thereto was added 10% palladium carbon (1.08 g), and the mixture was stirred for 2 hours and 30 minutes under hydrogen. The reaction mixture was filtered through Celite, and concentrated in vacuo. The residue was purified by silica gel column chromatography (eluent; hexane:ethyl acetate = 2:1) to give the subtitled compound (3.07 g) as a colorless oil. Yield 93%.
¹H NMR (CDCl₃) δ 3.96 (2H, s), 3.81 (2H, q, J = 5.8 Hz), 3.68 (2H, t, J = 5.7 Hz), 2.97 (1H, t, J = 5.8 Hz), 1.83 (2H, qui, J = 5.6 Hz), 1.48 (9H, s).

### Step (iii): (2-Formylethoxy)acetic acid tert-butyl ester

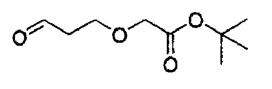

The compound obtained in Step (ii) (423 mg, 2.22 mmol) was dissolved in dimethylsulfoxide (5 ml), and thereto were added triethylamine (2 ml) and sulfur trioxide pyridine complex (2.83 g, 17.8 g), and the mixture was stirred for 1 hour. Thereto was added aqueous saturated ammonium chloride solution, and the mixture was extracted with ethyl acetate. The organic layer was sequentially washed with saturated sodium bicarbonate water, water and saturated saline, and dried over sodium sulfate. The mixture was concentrated in vacuo, and the residue was purified by silica gel column chromatography (eluent; hexane:ethyl acetate = 4:1 to 2:1) to give the subtitled compound (261 mg) as a colorless oil. Yield 63%.
¹H NMR (CDCl₃) δ 9.83 (1H, t, J = 1.7 Hz), 3.98 (2H, s), 3.88 (2H, t, J = 6.1 Hz), 2.74 (2H, dt, J = 1.7 Hz, 6.1 Hz), 1.48 (9H, s).

### Step (iv): 9-[4-(3-tert-Butoxycarbonylmethoxypropyl)-(methyl)-aminomethylbenzyl1-7,8-dihydro-2-(2-methoxyethoxy)-8-oxoadenine

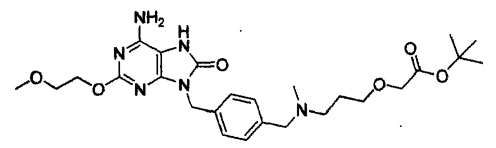

The compound obtained in Step (iii) (183 mg, 0.973 mmol) was dissolved in N-methyl-2-pyrrolidone (2.5 ml), and thereto were added the compound obtained in Example 36 Step (i) (204 mg, 0.569 mmol) and sodium triacetoxyborohydride (300 mg, 1.42 mmol), and the mixture was stirred for 15 hours. Thereto was added 1% ammonia water, and the precipitated solid was filtered, washed with water and purified by silica gel column chromatography (chloroform to chloroform:methanol = 50:1 to 25:1) to give the subtitled compound as a colorless amorphous solid. Yield 44%.
¹H NMR (DMSO-d₆) δ 9.94 (1H, s), 7.24 (4H, s), 6.45 (2H, s), 4.81 (2H, s), 4.24 (2H, t, J = 4.8 Hz), 3.89 (2H, s), 3.57 (2H, t, J = 4.8 Hz), 3.43 (2H, t, J = 6.5 Hz), 3.39 (2H, s), 3.27 (3H, s), 2.34 (2H, t, J = 7.2 Hz), 2.04 (3H, s), 1.66 (2H, qui, J = 6.8 Hz), 1.39 (9H, s).

### Step (v): 7,8-Dihydro-9-[4-(3-methoxycarbonylmethoxypropyl)(methyl)-aminomethylbenzyl]-2-(2-methoxyethoxy)-8-oxoadenine

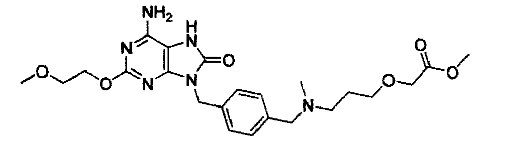

To the compound obtained in Step (iv) (196 mg, 0.369 mmol) were added 4N dioxane hydrochloric acid (5 ml) and chloroform (2 ml), and thereto was added methanol (2 ml), and the solution was stirred for 9 hours and evaporated. The residue was dissolved in methanol (2 ml), and thereto was added concentrated sulfuric acid (0.1 ml), and the mixture was heated to stir for 3 hours under refluxing. Thereto was added 1% ammonia water, and the precipitated solid was washed with water to give the titled compound (139 mg) as a white solid. Yield 73%.
¹H NMR (DMSO-d₆) δ9.95 (1H, s), 7.22 (4H, s), 6.46 (2H, s), 4.82 (2H, s), 4.24 (2H, t, J = 4.8 Hz), 4.03 (2H, s), 3.62 (3H, s), 3.57 (2H, t, J = 4.8 Hz), 3.45 (2H, t, J = 6.4 Hz), 3.38 (2H, s), 3.25 (3H, s), 2.34 (2H, t, J = 7.2 Hz), 2.05 (3H, s), 1.66 (2H, qui, J = 7.0 Hz).

### Example 39: 9-[4-(3-Carboxymethoxypropyl)(methyl)-aminomethylbenzyl]-7,8-dihydro-2-(2-methoxyethoxy)-8-oxoadenine

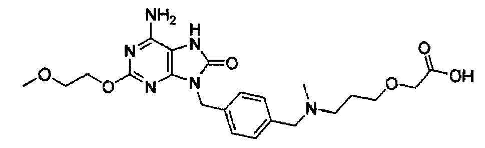

The compound obtained in Example 38 was treated in the similar manner to Example 2 to give the titled compound. Yield 91%.
¹H NMR (DMSO-d₆) δ 10.05 (1H, s), 7.35 (2H, d, J = 8.0 Hz), 7.27 (2H, d, J = 8.0 Hz), 6.50 (2H, s), 4.84 (2H, s), 4.24 (2H, t, J = 4.7 Hz), 3.81 (2H, s), 3.74 (2H, s), 3.57 (2H, t, J = 4.7 Hz), 3.47 (2H, t, J = 5.5 Hz), 3.25 (3H, s), 2.74 (2H, t, J = 6.4 Hz), 2.30 (3H, s), 1.79 (2H, qui, J = 5.8 Hz).

### Example 40: 7,8-Dihydro-9-{4-[(3-methoxycarbonylmethoxypropyl)(1-methylazetidin-3-yl)aminomethyl]benzyl}-2-(2-methoxyethoxy)-8-oxoadenine

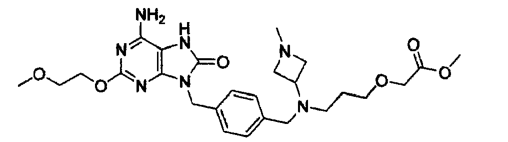

### Step (i): 9-(4-Formylbenzyl)-8-methoxy-2-(2-methoxyethoxy)adenine

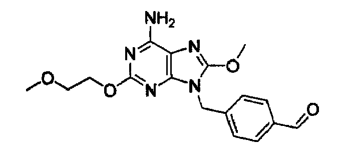

The compound obtained in Example 11 Step (i) (202 mg, 0.562 mmol) was dissolved in chloroform (8 ml), and thereto was added manganese dioxide (488 mg, 5.62 mmol), and the mixture was heated to stir for 2 hours under refluxing. The reaction mixture was filtered through Celite and concentrated in vacuo to give the subtitled compound (191 mg) as a yellow crystal. Yield 95%.
¹H NMR (CDCl₃) δ 9.98 (1H, s), 7.82 (2H, d, J = 8.2 Hz), 7.45(2H, d, J = 8.2 Hz), 5.20 (2H, s), 5.16 (2H, s), 4.45 (2H, t, J = 5.0 Hz), 4.09 (3H, s), 3.74 (2H, t, J = 5.0 Hz), 3.42 (3H, s).

### Step (ii): 9-[4-(1-tert-Butoxycarbonylazetidin-3-yl)aminomethylbenzyl]-8-methoxy-2-(2-methoxyethoxy)adenine

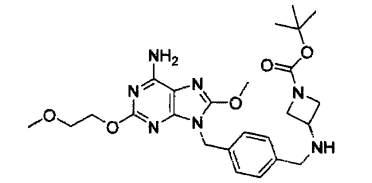

The compound obtained in Step (i) (398 mg, 1.11 mmol) and 1-Boc-3-aminoazetidine were dissolved in chloroform (10 ml), and thereto was added acetic acid (0.064 ml, 1.11 mmol), and the mixture was stirred for 30 minutes. Thereto was added sodium triacetoxyborohydride (704 mg, 3.34 mmol), and the mixture was stirred for 3 hours. Thereto was added saturated sodium bicarbonate water, and the mixture was extracted with chloroform. The organic layer was washed with saturated saline, dried over sodium sulfate, and then concentrated in vacuo. The residue was purified by silica gel column chromatography (chloroform:methanol = 200:1 to 100:1 to 20:1) to give the subtitled compound (570 mg) as a colorless oil. Yield 100%.
¹H NMR (CDCl₃) δ 7.28 (2H, d, J = 8.1 Hz), 7.22 (2H, d, J = 8.1 Hz), 5.22 (2H, s), 5.07 (2H, s), 4.46 (2H, t, J = 5.0 Hz), 4.08 (3H, s), 4.05 (2H, dd, J = 8.6 Hz, 6.4 Hz), 3.75 (2H, t, J = 5.0 Hz), 3.69 (2H, s), 3.64-3.57 (3H, m), 3.43 (3H, s), 1.42 (9H, s).

### Step (iii): 9-[4-(1-tert-Butoxycarbonylazetidin-3-yl)(3-tert-butoxycarbonylmethoxypropyl)aminomethylbenzyl]-8-methoxy-2-(2-methoxyethoxy)adenine

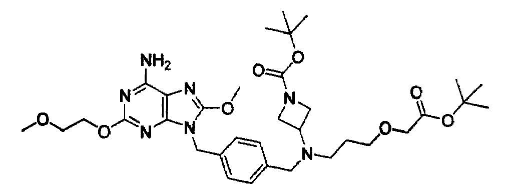

The compound obtained in Step (ii) (394 mg, 0.768 mmol) and the compound obtained in Example 38 Step (iii) (216 mg, 1.15 mmol) were dissolved in chloroform (8 ml), and the mixture was stirred for 20 minutes. Thereto was added sodium triacetoxyborohydride (322 mg, 1.53 mmol), and the mixture was stirred for 13 hours. Thereto was added saturated sodium bicarbonate water, and the mixture was extracted with chloroform. The organic layer was washed with saturated saline, dried over sodium sulfate, and then concentrated in vacuo. The residue was purified by silica gel column chromatography (chloroform to chloroform:methanol = 50:1) to give the subtitled compound (456 mg) as a colorless oil. Yield 87%.
¹H NMR (CDCl₃) δ 7.26 (2H, d, J = 8.0 Hz), 7.19 (2H, d, J = 8.0 Hz), 5.16 (2H, s), 5.06 (2H, s), 4.46 (2H, t, J = 5.0 Hz), 4.09 (3H, s), 3.96-3.89 (4H, m), 3.77-3.74 (4H, m), 3.53 (2H, s), 3.51-3.47 (3H, m), 3.43 (3H, s), 2.50 (2H, t, J = 7.2 Hz), 1.71 (2H, qui, J = 7.2 Hz), 1.46 (9H, s), 1.41 (9H, s).

### Step (iv): 7,8-Dihydro-9-{4-[(3-methoxycarbonylmethoxypropyl)(1-methylazetidin-3-yl)aminomethyl]benzyl}-2-(2-methoxyethoxy)-8-oxoadenine

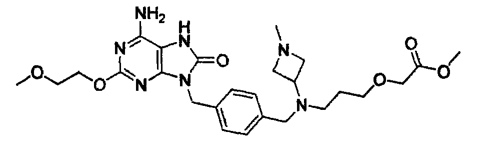

The compound obtained in Step (iii) (76 mg, 0.11 mmol) was dissolved in methanol (1 ml), and thereto was added 4N dioxane hydrochloric acid (1 ml), and the mixture was heated to stir for 1 hour under refluxing. The mixture was concentrated in vacuo and dissolved in methanol (2 ml), and thereto were added 35% aqueous formaldehyde solution (0.1 ml) and sodium cyanoborohydride (14 mg, 0.22 mmol), and the mixture was stirred for 2 hours and 30 minutes. Thereto was added saturated sodium bicarbonate water, and the mixture was extracted with chloroform:methanol (10:1). The organic layer was washed with saturated saline, dried over sodium sulfate, and then concentrated in vacuo. The residue was purified by silica gel column chromatography to give the titled compound (33 mg) as a colorless amorphous solid. Yield 55%.
¹H NMR (DMSO-d₆) δ 9.94 (1H, s), 7.21 (4H, s), 6.45 (2H, s), 4.82 (2H, s), 4.24 (2H, t, J = 4.7 Hz), 4.00 (2H, s), 3.61 (3H, s), 3.57 (2H, t, J = 4.7 Hz), 3.41-3.37 (4H, m), 3.32-3.28 (2H, m), 3.25 (3H, s), 3.09 (1H, qui, J = 7.0 Hz), 2.62 (2H, t, J = 7.0 Hz), 2.29 (2H, t, J = 7.2 Hz), 2.14 (3H, s), 1.55 (2H, qui, J = 6.7 Hz).

### Example 41: 9-{4-[(3-Carboxymethoxypropyl)(1-methylazetizin-3-yl)-aminomethyl]benzyl}-7,8-dihydro-2-(2-methoxyethoxy)-8-oxoadenine 3 hydrochloride

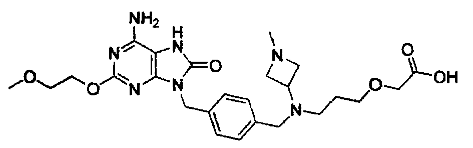

The compound obtained in Example 40 (27 mg, 0.050 mmol) was dissolved in concentrated hydrochloric acid (1 ml), and the mixture was stirred for 5 hours. The reaction solution was concentrated in vacuo, and thereto was added concentrated hydrochloric acid (1 ml), and the mixture was stirred for 1 hour and evaporated to give the titled compound (33 mg) as a white solid. Yield 100%.
¹H NMR (DMSO-d₆) δ 10.35 (1H, s), 7.51 (2H, brs), 7.34 (3H, d, J = 7.8 Hz), 6.72 (2H, brs), 4.88 (2H, s), 4.25 (2H, t, J = 4.7 Hz), 3.98 (2H, s), 3.57 (2H, t, J = 4.7 Hz), 3.48-3.42 (12H, m), 3.07-2.87 (5H, m), 1.91-1.83 (2H, m).

## Claims

1. An adenine compound of the formula (1): 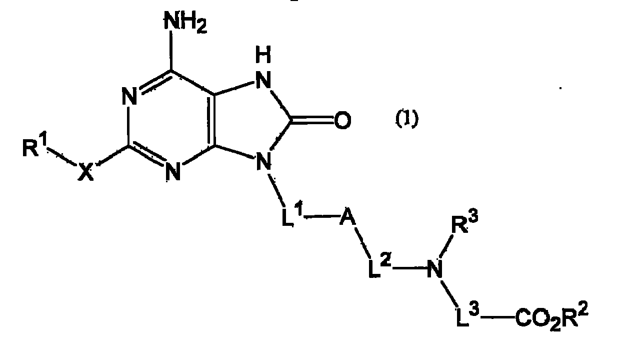 wherein A is substituted or unsubstituted aromatic carbocycle, or substituted or unsubsituted aromatic heterocycle;
L¹ is straight chain or branched chain alkylene, or a single bond;
L² and L³ are independently straight chain or branched chain alkylene, in which any 1 to 3 of methylene group(s) in the alkylene in L¹, L² and L³ may be replaced by oxygen, sulfur, SO, SO₂, carbonyl, NR⁴CO, CONR⁴, NR⁴SO₂, SO₂NR⁴, NR⁴CO₂, OCONR⁴, NR⁵CONR⁴, NR⁶C(=NR⁴)NR⁵, C(=NR⁴)NR⁵ wherein R⁴, R⁵ and R⁶ are independently hydrogen, or substituted or unsubstituted alkyl;
R¹ is halogen, substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl;
R² is hydrogen, or substituted or unsubstituted alkyl;
R³ is substituted or unsubstituted alkyl, substituted or unsubstituted alkenyl, substituted or unsubstituted alkynyl, substituted or unsubstituted cycloalkyl, or substituted or unsubstituted saturated heterocycle, or R³ may be combined together with L² or L³ to form nitrogen-containing saturated heterocycle;
X is oxygen, sulfur, SO, SO₂, NR⁷ wherein R⁷ is hydrogen or alkyl, or a single bond; provided that when R¹ is halogen, then X is a single bond; or a pharmaceutically acceptable salt thereof.

2. The adenine compound according to claim 1, wherein A in the formula (1) is substituted or unsubstituted benzene ring, or substituted or unsubstituted 5- to 6-membered monocyclic nitrogen-containing aromatic heterocycle, in which the substituent on A is selected from halogen, hydroxyl, carboxy, alkyl with 1 to 6 carbon atom(s), alkoxy with 1 to 6 carbon atom(s), alkoxycarbonyl with 2 to 6 carbon atoms, alkylcarbonyl with 2 to 6 carbon atoms, alkylsulfonyl with 1 to 6 carbon atom(s), and alkylsulfinyl with 1 to 6 carbon atom(s), or a pharmaceutically acceptable salt thereof.

3. The adenine compound according to claim 1 or 2, wherein in case that R², R⁴, R⁵ or R⁶ is substituted alkyl, or R³ is substituted alkyl, substituted alkenyl or substituted alkynyl, each group may be substituted by 1 or more substituent(s) selected from the following groups:
halogen, hydroxyl, carboxy, mercapta, alkoxy with 1 to 6 carbon atom(s), haloalkoxy with 1 to 6 carbon atom(s), alkoxycarbonyl with 2 to 6 carbon atoms, alkylsulfonyl with 1 to 6 carbon atom(s), alkylsulfinyl with 1 to 6 carbon atom(s), alkylcarbonyloxy with 2 to 6 carbon atoms, alkylthio with 1 to 6 carbon atom(s), substituted or unsubstituted amino, substituted or unsubstituted carbamoyl, substituted or unsubstituted sulfamoyl, and cycloalkyl in which the cycloalkyl may be substituted by halogen, hydroxyl, carboxy, alkyl with 1 to 4 carbon atom(s) or alkoxy with 1 to 4 carbon atom(s),
the substituted amino, substituted carbamoyl and substituted sulfamoyl may be substituted by 1 or 2 members selected from the following (a') and (b'):
(a') alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, 3- to 8-membered cycloalkoxycarbonyl, 3- to 8-membered cycloalkylsulfonyl, 3-to 8-membered cycloalkylsulfinyl,
wherein each group may further be substituted by halogen, hydroxyl, alkyl with 1 to 6 carbon atom(s), alkoxy with 1 to 6 carbon atom(s), carboxy, or alkoxycarbonyl with 2 to 6 carbon atoms;
(b') 2 substituents are combined together with nitrogen atom to form 4- to 7-membered nitrogen-containing saturated heterocycle with 1 to 2 heteroatom(s) selected from 1 to 2 nitrogen(s), 0 to 1 oxygen and 0 to 1 sulfur,
wherein the nitrogen-containing saturated heterocycle may be substituted on any carbon atoms or nitrogen atoms by halogen, hydroxyl, carboxy, alkyl with 1 to 6 carbon atom(s), alkoxy with 1 to 6 carbon atom(s), alkoxycarbonyl with 2 to 6 carbon atoms or alkylcarbonyl with 2 to 6 carbon atoms, where the substituent may be kept in chemically stable state,
in case that R³ is substituted cycloalkyl or substituted saturated heterocycle, the substituent is 1 or more members selected from halogen, hydroxyl, alkyl with 1 to 6 carbon atom(s), alkoxy with 1 to 6 carbon atom(s) and amino optionally substituted by 1 or 2 alkyl(s) with 1 to 6 carbon atom(s),
the substituted alkyl, substituted alkenyl and substituted alkynyl in R¹ is substituted by 1 or more members independently selected from the following (a) to (c):
(a) halogen, hydroxyl, carboxy, haloalkoxy with 1 to 6 carbon atom(s), mercapto;
(b) alkoxy with 1 to 6. carbon atom(s), alkylthio with 1 to 6 carbon atom(s), alkylcarbonyl with 2 to 6 carbon atoms, alkylcarbonyloxy with 2 to 6 carbon atoms, alkylsulfonyl with 1 to 6 carbon atom(s), alkylsulfinyl with 1 to 6 carbon atom(s), alkoxycarbonyl with 2 to 6 carbon atoms,
wherein each group may further be substituted by 1 or more group(s) independently selected from halogen, hydroxyl, carboxy, alkoxy with 1 to 6 carbon atom(s), alkoxycarbonyl with 2 to 6 carbon atoms, amino optionally substituted by the same or different 1 or 2 alkyl(s) with 1 to 6 carbon atom(s), carbamoyl optionally substituted by the same or different. 1 or 2 alkyl(s) with 1 to 6 carbon atom(s), sulfamoyl optionally substituted by the same or different 1 or 2 alkyl(s) with 1 to 6 carbon atom(s), and alkylsulfonyl with 1 to 6 carbon atom(s);
(c) substituted or unsubstituted amino, substituted or unsubstituted carbamoyl and substituted or unsubstituted sulfamoyl, wherein each group may further be substituted by 1 or 2 substituent(s) selected from the following (j), (k) and (l), substituted or unsubstituted 3- to 8-membered cycloalkyl and substituted or unsubstituted 4- to 8-membered saturated heterocycle, wherein each group may further be substituted by 1 or more substituent(s) selected from the following (d),
(e) and (f), and substituted or unsubstituted 6- to 10-membered aryl, substituted or unsubstituted 5- to 10-membered heteroaryl, substituted or unsubstituted 6- to 10-membered aryloxy and substituted or unsubstituted 5- to 10-membered heteroaryloxy, wherein each group may further be substituted by 1 or more substituent(s) selected from the following (g). (h) and (i);
the substituted cycloalkyl in R¹ is substituted by 1 or more members independently selected from the following (d) to (f):
(d) halogen, hydroxyl, carboxy, mercapto, haloalkyl with 1 to 6 carbon atom(s), haloalkoxy with 1 to 6 carbon atom(s);
(e) alkyl with 1 to 6 carbon atom(s), alkenyl with 2 to 6 carbon atoms, alkynyl with 2 to 6 carbon atoms, alkoxy with 1 to 6 carbon atom(s), alkylcarbonyl with 2 to 6 carbon atoms, alkylcarbonyloxy with 2 to 6 carbon atoms, alkoxycarbonyl with 2 to 6 carbon atoms, alkylthio with 1 to 6 carbon atom(s), alkylsulfonyl with 1 to 6 carbon atom(s), alkylsulfinyl with 1 to 6 carbon atom(s);
wherein each group may further be substituted by 1 or more group(s) independently selected from halogen, hydroxyl, carboxy, alkoxy with 1 to 6 carbon atom(s), alkoxycarbonyl with 2 to 6 carbon atoms, amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), sulfamoyl optionally substituted by the same or different 1 or 2 alkyl(s) with 1 to 6 carbon atom(s), and alkylsulfonyl with 1 to 6 carbon atom(s);
(f) substituted or unsubstituted amino, substituted or unsubstituted carbamoyl and substituted or unsubstituted sulfamoyl, wherein each group may further be substituted by 1 or 2 substituent(s) selected from the following (j), (k) and (l), and substituted or unsubstituted 6- to 10-membered aryl and substituted or unsubstituted 5- to 10-membered heteroaryl, wherein each group may further be substituted by 1 or more substituent(s) selected from the following (g), (h) and (i);
the substituted aryl and substituted heteroaryl in R¹ is substituted by 1 or more members independently selected from the following (g) to (i):
(g) halogen, hydroxyl, mercapto, cyano, nitro, haloalkyl with 1 to 6 carbon atom(s), haloalkoxy with 1 to 6 carbon atom(s);
(h) alkyl with 1 to 6 carbon atom(s), alkenyl with 2 to 6 carbon atoms, alkynyl with 2 to 6 carbon atoms, alkoxy with 1 to 6 carbon atom(s), alkylcarbonyl with 2 to 6 carbon atoms, alkylcarbonyloxy with 2 to 6 carbon atoms, alkylthio with 1 to 6 carbon atom(s), alkylsulfonyl with 1 to 6 carbon atom(s), alkylsulfinyl with 1 to 6 carbon atom(s), 3- to 8-membered cycloalkyl and 4- to 8-membered saturated heterocycle,
wherein each group may further be substituted by 1 or more group(s) independently selected from halogen, hydroxyl, carboxy, alkyl with 1 to 6 carbon atom(s), alkoxy with 1 to 6 carbon atom(s), alkoxycarbonyl with 2 to 6 carbon atoms, amino optionally substituted by the same or different 1 or 2 alkyl(s) with 1 to 6 carbon atom(s), carbamoyl optionally substituted by the same or different 1 or 2 alkyl(s) with 1 to 6 carbon atam(s), sulfamoyl optionally substituted by the same or different 1 or 2 alkyl(s) with 1 to 6 carbon atom(s), and alkylsulfonyl with 1 to 6 carbon atom(s);
(i) substituted or unsubstituted amino, substituted or unsubstituted carbamoyl, substituted or unsubstituted sulfamoyl, wherein each group may further be substituted by 1 or 2 substituent(s) selected from the following (j), (k) and (l);
the substituted amino, substituted carbamoyl and substituted sulfamoyl in the above (a) to (i) are substituted by 1 or 2 members independently selected from the following (j) to (l):
(j) alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, 3- to 8-membered cycloalkoxycarbonyl, 3- to 8-membered cycloalkylsulfonyl, 3-to 8-membered cycloalkylsulfinyl,
wherein each group may further be substituted by 1 or more group(s) independently selected from halogen, hydroxyl, carboxy, alkyl with 1 to 6 carbon atom(s), alkoxy with 1 to 6 carbon atom(s), alkoxycarbonyl with 2 to 6 carbon atoms, amino optionally substituted by the same or different 1 or 2 alkyl(s), carbamoyl optionally substituted by the same or different 1 or 2 alkyl(s), sulfamoyl optionally substituted by the same or different 1 or 2 alkyl(s), or alkylsulfonyl;
(k) 6- to 10-membered aryl, 6- to 10-membered arylcarbonyl, 6- to 10-membered aryloxycarbonyl, 6- to 10-membered arylsulfonyl, 6- to 10-membered arylsulfinyl, 5- to 10-membered heteroaryl, 5- to 10-membered heteroarylcarbonyl, 5- to 10-membered heteroaryloxycarbonyl, 5- to 10-membered heteroarylsulfonyl, 5- to 10-membered heteroarylsulfinyl,
wherein each group may further be substituted by halogen, hydroxyl, carboxy, alkyl with 1 to 6 carbon atom(s), alkoxy with 1 to 6 carbon atom(s), amino optionally substituted by the same or different 1 or 2 alkyl(s) with 1 to 6 carbon atom(s), carbamoyl optionally substituted by the same or different 1 or 2 alkyl(s) with 1 to 6 carbon atom(s), sulfamoyl optionally substituted by the same or different 1 or 2 alkyl(s) with 1 to 6 carbon atom(s), or alkylsulfonyl with 1 to 6 carbon atom(s);
(l) two substituents being combined together with nitrogen atom to form 4- to 7-membered nitrogen-containing saturated heterocycle with 1 to 4 heteroatom(s) selected from 1 to 2 nitrogen(s), 0 to 1 oxygen and 0 to 1 sulfur,

4. The adenine compound according to any one of claims 1 to 3, wherein "-L²-NR³-L³-" in the formula (1) is a group of the formula (10): 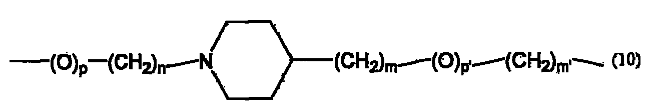 wherein p and p' are independently 0 or 1, m and m' are independently an integer of 0 to 6, n is an integer of 1 to 8 when p is 0 or an integer of 2 to 8 when p is 1,
the formula (11): 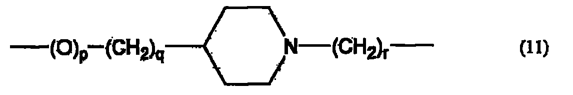 wherein p is 0 or 1, r is an integer of 1 to 6, q is an integer of 1 to 8 when p is 0 or an integer of 0 to 8 when p is 1,
the formula (12): 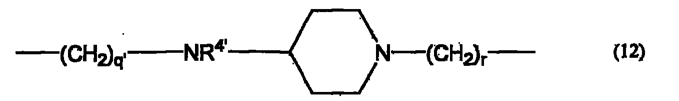 wherein r is the same as defined above, R⁴' is hydrogen or alkyl with 1 to 3 carbon atom(s), q' is an integer of 0 to 4,
the formula (13): 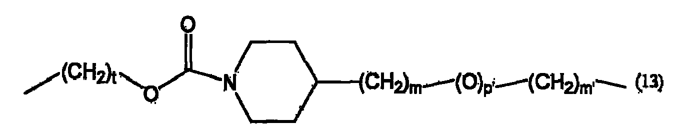 wherein t is an integer of 0 to 6, m, m' and p' are the same as defined above, or
the formula (14):
-(O)ₚ-(CH₂)ₙ-NR^{3'}-(CH₂)ₛ-(O)_{p'}-(CH₂)ₜ-
wherein p, n, t and p' are the same as defined above, s is an integer of 0 to 6 provided that when p' is 1, then s is 2 or more, R³' is hydrogen, alkyl with 1 to 6 carbon atom(s), 3- to 8-membered cycloalkyl or 4- to 8-membered monocyclic saturated heterocycle, in which alkyl, cycloalkyl and saturated heterocycle may be substituted by substituents selected from halogen, hydroxyl, alkyl with 1 to 6 carbon atom(s), alkoxy with 1 to 6 carbon atom(s) and amino optionally substituted by 1 or 2 alkyl(s) with 1 to 6 carbon atom(s);
or a pharmaceutically acceptable salt thereof.

5. The adenine compound according to any one of claims 1 to 4, wherein L¹ is alkylene with 1 to 6 carbon atom(s) in the formula (1), or a pharmaceutically acceptable salt thereof.

6. The adenine compound according to any one of claims 1 to 5, wherein R² is alkyl with 1 to 4 carbon atom(s) in the formula (1), or a pharmaceutically acceptable salt thereof.

7. The adenine compound according to claim 6, wherein R² is methyl in the formula (1), or a pharmaceutically acceptable salt thereof.

8. The adenine compound according to any one of claims 1 to 5, wherein R² is alkyl with 2 to 8 carbon atoms substituted by substituted or unsubstituted amino in the formula (1), or a pharmaceutically acceptable salt thereof.

9. The adenine compound according to claim 1, which is selected from the following compounds:
2-butoxy-7,8-dihydro-9-[4-{4-[4-(methoxycarbonyl)piperidin-1-yl]-butoxy}benzyl]-8-oxoadenine;
2-butoxy-7,8-dihydro-9-[4-{4-[4-(carboxy)pipenam-1-yl]butoxy}benzyl]-8-oxoadenine;
2-butoxy-7,8-dihydro-9-(4-[4-{4-[4-(N,N-dimethylamino)-butoxycarbonyl]-piperidin-1-yl}butoxy]benzyl)-8-oxoadenine;
2-butoxy-7,8-dihydro-9-[4-{3-[(N-methyl-N-methoxycarbonylmethyl)-amino]propoxy}benzyl]-8-oxoadenine;
2-butoxy-7,8-dihydro-9-{4-[1-(methoxycarbonylmethyl)piperidin-4-ylmethyloxy]benzyl}-8-oxoadenine;
2-butoxy-7,8-dihydro-9-{4-[1-(carboxymethyl)piperidin-4-ylmethyloxy]-benzyl}-8-oxoadenine;
2-butoxy-7,8-dihydro-9-[4-{1-[4-(N,N-dimethylamino)-butoxycarbonylmethyl]-piperidin-4-ylmethyloxy}benzyl]-8-oxoadenine;
2-butoxy-7,8-dihydro-9-[4-(N-ethoxycarbonylmothyl-N-methylammomethyl)benzyl]-8-oxoadenine;
2-butoxy-7,8-dihydro-9-[4-(N-methoxycarbonylmethyl-N-methylaminomethyl)benayl]-8-oxoadenine;
2-butoxy-7,8-clihydro-9-[4-(N-hydroxycarbonylmethyl-N-methylaminomethyl)benzyl]-8-oxoadenme;
7,8-dihydro-9-[4-(N-ethoxycarbonyhnethyl-N-niethylaminomethyl)-benzyl]-2-(2-methoxyethoxy)-8-oxoadenine;
7,8-dihydro-9-[4-(N-methoxycarbonylmethyl-N-methylaminomethyl)-benzyl]-2-(2-methoxyethoxy)-8-oxoadenine;
7,8-dihydro-9-[4-(N-hydroxycarbonylmethyl-N-methylaminomethyl)-benzyl]-2-(2-methoxyethoxy)-8-oxoadenine;
7,8-dihydro-9-[4-(4-methoxycarbonylpiperidin-1-ylmethyl)benzyl]-2-(2-methoxyethoxy)-8-oxoadenine;
7,8-dihydro-9-[4-(4-hydroxycarbonylpiperidin-1-ylmethyl)benzyl]-2-(2-methoxyethoxy)-8-oxoadenine;
2-butoxy-7,8-dihydro-9-[2-methoxy-4-(N-methoxycarbonylmethyl-N-methylaminomethyl)benzyl]-8-oxoadenine;
2-butoxy-7,8-dihydro-9-[2-methoxy-4-(N-hydroxycarbonylmethyl-N-methylaminomethyl)benzyl]-8-oxoadenine;
2-butoxy-7,8-dihydro-9-{4-[2-(4-methoxycarbonylpiperidm-1-yl)ethyl]-benzyl}-8-oxoadenine;
2-butoxy-7,8-dihydro-9-{4-[2-(4-hydroxycarbonylpiperidin-1-yl)ethyl]-benzyl}-8-oxoadenine;
2-butoxy-7,8-dihydro-9-{4-[2-(N-methoxycarbonylmethyl-N-methylamino)ethyl]benzyl}-8-oxoadenine;
2-butoxy-7,8-dihydro-9-{4-[3-(N-methoxycarbonylmethyl-N-methylamino)ethyl]propyl}-8-oxoadenine;
2-butoxy-7,8-dihydro-9-{4-[3-(N-hydroxycarbonylmethyl-N-methylamino)ethyl]propyl}-8-oxoadenine;
2-butoxy-7,8-dihydro-9-{4-[N-(4-dimethylaminobutoxycarbonylmethyl)-N-methylaminomethyl]-2-methoxybenzyl}-8-oxoadenine;
2-butoxy-7,8-dihydro-9-{6-[4-(4-methnxycarbonylpiperidin-1-yl)butoxy]-pyridin-3-ylmethyl}-8-oxoadenine;
2-butoxy-7,8-dihydro-9-{6-[4-(4-hydroxycarbonylpiperidin-1-yl)butoxy]-pyridin-3-ylmethyl}-8-oxoadenine;
2-butoxy-7,8-dihydro-9-(6-{4-[(N-methyl-N-methoxycarbonylmethyl)-amino]butoxy}pyridin-3-ylmethyl)-8-oxoadenine;
2-butoxy-7,8-dihydro-9-(6-{4-[(N-hydroxycarbonylmethyl-N-methyl)-amino]butoxy}pyridin-3-ylmethyl}-8-oxoadenine;
2-butoxy-7,8-dihydro-9-[6-(4-{N'-methyl-N'-[4-(N,N-dimethylamino)-butoxycarbonylmethyl]}aminobutoxy)pyridin-3-ylmethyl]-8-oxoadenine;
2-butoxy-7,8-dihydro-9-{6-[3-(4-methoxycarbonylpiperidin-1-yl)-propoxy]pyridin-3-yltnethyl}-8-oxoadenine;
2-butoxy-7,8-dihydro-9-{6-[3-(4-hydroxycarbonylpiperidin-1-yl)-butoxy]-pyridin-3-ylmethyl}-8-oxoadenine;
2-butoxy-7,8-dihydro-9-{6-[3-(methoxycarbonylmethyl)-aminopropoxy]-pyridin-3-ylmethyl}-8-oxoadenine;
2-butoxy-7,8-dihydro-9-{6-[2-(4-methoxycarbonylpiperidin-1-yl)ethoxy]-pyridin-3-ylmethyl}-8-oxoadenine;
2-butoxy-7,8-dihydro-9-{6-[2-(4-hydroxycarbonylpiperidin-1-yl)ethoxy]-pyridin-3-ylmethyl}-8-oxoadenine;
methyl N-(3-{4-[(6-amino-2-butoxy-8-oxo-7,8-dihydro-9H-purin-9-yl)-methyl]phenoxy}propyl)-N-[2-(dimethylamino)ethyl]glycinate;
7,8-dihydro-9-(4-{[N-methyl-N-(N'-methoxycarbonylmethylpiperidin-4-yl)]amino}methylbenzyl)-2-(2-methoxyethoxy)-8-oxoadenine;
7,8-dihydro-9-{4-[4-(methoxycarbonylmethoxymethyl)piperidin-1-ylmethyl]benzyl}-2-(2-methoxyethoxy)-8-oxoadenine;
7,8-dihydro-9-[4-(3-methoxycarbonylmethoxypropyl)(methyl)-aminomethylbenzyl]-2-(2-methoxyethoxy)-8-oxoadenine;
9-[4-(3-carboxymethoxypropyl)(methyl)aminomethylbenzyl]-7,8-dihydro-2-(2-methoxyethoxy)-8-oxoadenine;
7,8-dihydro-9-{4-[(3-methoxycarbonylmethoxypropyl)(1-methylazetidin-3-yl)aminomethyl]benzyl}-2-(2-methoxyethoxy)-8-oxoadenine; or
9-{4-[(3-carboxymethoxypropyl)(1-methylazetidin-3-yl)aminomethyl]-benzyl}-7,8-dihydro-2-(2-methoxyethoxy)-8-oxoadenine;
or a pharmaceutically acceptable salt thereof.

10. A pharmaceutical composition comprising as an active ingredient the adenine compound according to any one of claims 1 to 9, or a pharmaceutically acceptable salt thereof.

11. An agent for increasing TLR7 activity comprising as an active ingredient the adenine compound according to any one of claims 1 to 9, or a pharmaceutically acceptable salt thereof.

12. An immune-regulating agent comprising as an active ingredient the adenine compound according to any one of claims 1 to 9, or a pharmaceutically acceptable salt thereof.

13. An agent for the treatment or prevention of allergic disease, viral disease or cancer, which comprises as an active ingredient the adenine compound according to any one of claims 1 to 9, or a pharmaceutically acceptable salt thereof.

14. An agent for the treatment or prevention of asthma, COPD, allergic rhinitis, allergic conjunctivitis, atopic dermatitis, cancer, hepatitis B, hepatitis C, HIV, HPV, bacterial infectious disease or dermatitis, which comprises as an active ingredient the adenine compound according to any one of claims 1 to 9, or a pharmaceutically acceptable salt thereof.

15. A pharmaceutical composition for local administration, which comprises as an active ingredient the adenine compound according to any one of claims 1 to 9, or a pharmaceutically acceptable salt thereof.
